# EUROPEAN PATENT APPLICATION

(11) **EP 4 382 617 A1**
(43) Date of publication of application: **12.06.2024**
(21) Application number: 22852326.2
(22) Date of filing: 05.08.2022
(51) Int. Cl.: C12Q 1/6886, C12N 15/11

(54) **TUMOR EVALUATION METHOD AND APPLICATION**

(30) Priority: 06.08.2021 CN 202110903691; 06.08.2021 CN 202110903841; 06.08.2021 CN 202110902676; 06.08.2021 CN 202110902703; 29.09.2021 CN 202111155166; 29.09.2021 CN 202111152193; 29.09.2021 CN 202111152207; 29.09.2021 CN 202111155144; 29.09.2021 CN 202111152199; 29.09.2021 CN 202111155147; 29.09.2021 CN 202111152169; 29.09.2021 CN 202111155142; 30.11.2021 CN 202111449060
(71) Applicant: Singlera Genomics (Jiangsu) Ltd., Yangzhou, Jiangsu 225012 (CN); Singlera Genomics (China) Ltd., Yangzhou, Jiangsu 225012 (CN)
(72) Inventor: WANG, Hui, Shanghai 201318 (CN); YANG, Qichang, Shanghai 201318 (CN); SU, Zhixi, Shanghai 201318 (CN); MA, Chengcheng, Shanghai 201318 (CN); LIU, Rui, Shanghai 201318 (CN)
(74) Representative: Weickmann & Weickmann PartmbB
(86) International application number: PCT/CN2022/110445
(87) International publication number: WO 2023/011615

(57) **Abstract**

Provided are a tumor evaluation method and an application. Specifically, provided is a method for confirming the existence of liver tumors, evaluating the formation or formation risk of liver tumors and/or evaluating the progress of liver tumors, and the method comprises: evaluating the existence and/or the content of a modification state of a DNA region or a fragment thereof wherein a group of marker genes is in a sample to be tested. The present invention also relates to nucleic acids, a nucleic acid group, and/or a kit for evaluating modification states of a group of DNA regions, and a preparation method therefor.

## Description

### TECHNICAL FIELD

The present application relates to the field of biomedicine, and specifically to a tumor assessment method and use.

### BACKGROUND

Searching for more effective tumor markers has always been an important direction in tumor-related researches, and the technology for detection of plasma free DNA based on liquid biopsy has a higher demand for the discovery of tumor markers. The basic principle is that free DNA is released into the blood after the death of tumor cells, and by detecting the information related to tumor cells such as DNA mutation, DNA methylation, miRNA and histone modification in the blood, very early tumor signals can be detected. It has been found that the change of DNA methylation may be earlier than the occurrence of DNA mutation, which is a more effective detection marker for early tumor screening.

However, at present, there are very few tumor cells released into the blood in early cancer, and it is urgent to find more effective detection markers for liver tumor in the field. Therefore, it is urgent to develop a method and/or a kit, which can detect accurate, stable and effective biomarkers of liver cancer or combinations thereof, and can efficiently read the epigenetic information from the extremely limited amount of extracellular free DNA in biological samples. More preferably, this method should be easily configured and reliably applied in hospital laboratories.

### SUMMARY OF THE INVENTION

The method provided in the present application can use more efficient methylation markers for identifying or assisting to identify the presence of a disease, assessing the development or development risk of the disease and/or assessing the progress of the disease, and can improve the efficiency of early screening and early diagnosis of tumors, such as liver tumor, and solve the problems of low early diagnosis rate of liver cancer and heavy burden of clinical treatment.

In one aspect, the present application provides a method for identifying the presence of a liver tumor, assessing the development or development risk of the liver tumor, and/or assessing the progress of the liver tumor, comprising determining the presence and/or the content of modification status of a DNA region in which a target gene is located or a fragment thereof in a sample to be tested, wherein the target gene comprises SEPT9 and IKZF1.

In another aspect, the present application also provides a method for assessing the methylation status of a liver tumor-related DNA region, comprising determining the presence and/or the content of modification status of a DNA region in which a target gene is located or a fragment thereof in a sample to be tested, wherein the target gene comprises SEPT9 and IKZF1.

In another aspect, the present application also provides a method for identifying the presence of a disease, assessing the development or development risk of the disease and/or assessing the progress of the disease, comprising determining the presence and/or the content of modification status of a target DNA region, or a complementary region thereof, or a fragment thereabove in a sample to be tested, wherein the target DNA region comprises regions defined from human chr17:75368651-75370720 and from human chr7:50343720-50344547.

In another aspect, the present application also provides a method for determining methylation status of a DNA region, comprising determining the presence and/or the content of modification status of a target DNA region, or a complementary region thereof, or a fragment thereabove in a sample to be tested, wherein the target DNA region comprises regions defined from human chr17:75368651-75370720 and from human chr7:50343720-50344547.

In another aspect, the present application also provides a nucleic acid comprising a sequence capable of binding to a DNA region in which a target gene is located, or a complementary region thereof, or a transformed region thereabove, or a fragment thereabove in the method of the present application; or comprising a sequence capable of binding to a target DNA region, or a complementary region thereof, or a transformed region thereabove, or a fragment thereabove in the method of the present application.

In another aspect, the present application also provides a method for preparing a nucleic acid, comprising, based on modification status of a DNA region in which a target gene is located, or a complementary region thereof, or a transformed region thereabove, or a fragment thereabove in the method of the present application, designing a nucleic acid capable of binding to the DNA region in which a target gene is located, or a complementary region thereof, or a transformed region thereabove, or a fragment thereabove; or, based on a modification status of a target DNA region, or a complementary region thereof, or a transformed region thereabove, or a fragment thereabove in the method of the present application, designing a nucleic acid capable of binding to the target DNA region, or a complementary region thereof, or a transformed region thereabove, or a fragment thereabove.

In another aspect, the present application also provides a nucleic acid set, comprising a sequence capable of binding to a DNA region in which a target gene is located, or a complementary region thereof, or a transformed region thereabove, or a fragment thereabove in the method of the present application; or comprising a sequence capable of binding to a target DNA region, or a complementary region thereof, or a transformed region thereabove, or a fragment thereabove in the method of the present application.

In another aspect, the present application also provides a method for preparing a nucleic acid set, comprising, based on modification status of a DNA region in which a target gene is located, or a complementary region thereof, or a transformed region thereabove, or a fragment thereabove in the method of the present application, designing a nucleic acid set capable of binding to the DNA region in which a target gene is located, or a complementary region thereof, or a transformed region thereabove, or a fragment thereabove; or, based on a modification status of a target DNA region, or a complementary region thereof, or a transformed region thereabove, or a fragment thereabove in the method of the present application, designing a nucleic acid set capable of binding to the target DNA region, or a complementary region thereof, or a transformed region thereabove, or a fragment thereabove.

In another aspect, the present application also provides a kit comprising the nucleic acid of the present application and/or the nucleic acid set of the present application.

In another aspect, the present application also provides use of the nucleic acid of the present application, the nucleic acid set of the present application and/or the kit of the present application in the preparation of a substance for determining modification status of a DNA region or a fragment thereof.

In another aspect, the present application also provides use of the nucleic acid of the present application, the nucleic acid set of the present application and/or the kit of the present application in the preparation of a disease detection product.

In another aspect, the present application also provides use of the nucleic acid of the present application, the nucleic acid set of the present application and/or the kit of the present application in the preparation of a substance for identifying the presence of a disease, assessing the development or development risk of the disease and/or assessing the progress of the disease.

In another aspect, the present application also provides use of a nucleic acid, a nucleic acid set and/or a kit for determining modification status of a DNA region in the preparation of a substance for identifying the presence of a liver tumor, assessing the development or development risk of the liver tumor and/or assessing the progress of the liver tumor, wherein the DNA region for determination comprises a DNA region in which a target gene is located, or a fragment thereof in the method of the present application.

In another aspect, the present application also provides use of a nucleic acid, a nucleic acid set and/or a kit for determining modification status of a DNA region in the preparation of a substance for identifying the presence of a disease, assessing the development or development risk of the disease and/or assessing the progress of the disease, wherein the DNA region for determination comprises a target DNA region, or a complementary region thereof, or a fragment thereabove in the method of the present application.

In another aspect, the present application also provides use of a nucleic acid of a DNA region in which a target gene is located, or a transformed region thereof or a fragment thereabove, or a combination thereof in the method of the present application, in the preparation of a substance for identifying the presence of a liver tumor, assessing the development or development risk of the liver tumor, and/or assessing the progress of the liver tumor.

In another aspect, the present application also provides use of a nucleic acid of a target DNA region in the method of the present application, or a complementary region thereof or a transformed region thereabove or a fragment thereabove, or a combination thereof, in the preparation of a substance for identifying the presence of a disease, assessing the development or development risk of the disease, and/or assessing the progress of the disease.

In another aspect, the present application also provides a storage medium recording a program capable of executing the method of the present application.

In another aspect, the present application also provides a device comprising the storage medium of the present application.

Those skilled in the art will readily appreciate other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the descriptions in the specification of the present application are illustrative only and not limited.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
Figs. 1, 3, 5, and 7 show the comparison of DNA methylation signals of paracancerous tissues, cancerous tissues and leukocytes at the detection site.
Figures 2, 4, 6 and 8 show the comparison of DNA methylation signals in control plasma and liver cancer plasma at the detection site.

### DETAILED DESCRIPTION OF THE INVENTION

The embodiments of the invention of the present application will be described below with specific examples. Those skilled in the art can easily understand other advantages and effects of the invention of the present application from the disclosure of the specification.

### Definition of Terms

In the present application, the term "BCAT1" generally refers to a gene or an expression product thereof. For example, the UniProt accession number of BCAT1 (Branched-chain-aminoacid aminotransferase) protein may be P54687. In the present application, BCAT1 may encompass its unprocessed form, any processed form, its variant or a substance comprising its functional active fragment.

In the present application, the term "VASH2" generally refers to a gene or an expression product thereof. For example, the UniProt accession number of VASH2 (Tubulinyl-Tyr carboxypeptidase 2) protein may be Q86V25. In the present application, VASH2 may encompass its unprocessed form, any processed form, its variant or a substance comprising its functional active fragment.

In the present application, the term "GPAM" generally refers to a gene or an expression product thereof. For example, the UniProt accession number of GPAM (Glycerol-3-phosphate acyltransferase 1, mitochondrial) protein may be Q9HCL2. In the present application, GPAM may encompass its unprocessed form, any processed form, its variant or a substance comprising its functional active fragment.

In the present application, the term "VAV3" generally refers to a gene or an expression product thereof. For example, the UniProt accession number of VAV3 (Guanine nucleotide exchange factor VAV3) protein may be Q9UKW4. In the present application, VAV3 may encompass its unprocessed form, any processed form, its variant or a substance comprising its functional active fragment.

In the present application, the term "SEPT9" generally refers to a gene or an expression product thereof. For example, the UniProt accession number of SEPT9 (SEPTIN9) protein may be Q9UHD8. In the present application, SEPT9 may encompass its unprocessed form, any processed form, its variant or a substance comprising its functional active fragment. For example, SEPT9 in the present application may also indicate a detection site, such as methylation detection for a specific nucleic acid sequence of the gene. For example, a detection site indicated by SEPT9 may be a nucleic acid sequence of one or more regions under the gene.

In the present application, the term "IKZF1" generally refers to a gene or an expression product thereof. For example, the UniProt accession number of IKZF1 (DNA-binding protein Ikaros) may be Q13422. In the present application, IKZF1 may encompass its unprocessed form, any processed form, its variant or a substance comprising its functional active fragment. For example, IKZF1 in the present application may also indicate a detection site, such as methylation detection for a specific nucleic acid sequence of the gene. For example, a detection site indicated by IKZF1 may be a nucleic acid sequence of one or more regions under the gene.

In the present application, the term "BEST4" generally refers to a gene or an expression product thereof. For example, the UniProt accession number of BEST4 (Bestrophin-4) protein may be Q8NFU0. In the present application, BEST4 may encompass its unprocessed form, any processed form, its variant or a substance comprising its functional active fragment. For example, BEST4 in the present application may also indicate a detection site, such as methylation detection for a specific nucleic acid sequence of the gene. For example, a detection site indicated by BEST4 may be a nucleic acid sequence of one or more regions under the gene.

In the present application, the term "B4GALNT1" generally refers to a gene or an expression product thereof. For example, the UniProt accession number of B4GALNT1 (Beta-1,4 N-acetylgalactosaminyltransferase 1) protein may be Q00973. In the present application, B4GALNT1 may encompass its unprocessed form, any processed form, its variant or a substance comprising its functional active fragment. For example, B4GALNT1 in the present application may also indicate a detection site, such as methylation detection for a specific nucleic acid sequence of the gene. For example, a detection site indicated by B4GALNT1 may be a nucleic acid sequence of one or more regions under the gene.

In the present application, the term "GRASP" generally refers to a gene or an expression product thereof. For example, the UniProt accession number of GRASP (GRP1 (General receptor for phosphoinositides 1)-associated scaffold protein) protein may be K7CHN5. In the present application, GRASP may encompass its unprocessed form, any processed form, its variant or a substance comprising its functional active fragment. For example, GRASP in the present application may also indicate a detection site, such as methylation detection for a specific nucleic acid sequence of the gene. For example, a detection site indicated by GRASP may be a nucleic acid sequence of one or more regions under the gene.

In the present application, the term "IRF4" generally refers to a gene or an expression product thereof. For example, the UniProt accession number of IRF4 (Interferon regulatory factor 4) protein may be Q15306. In the present application, IRF4 may encompass its unprocessed form, any processed form, its variant or a substance comprising its functional active fragment. For example, IRF4 in the present application may also indicate a detection site, such as methylation detection for a specific nucleic acid sequence of the gene. For example, a detection site indicated by IRF4 may be a nucleic acid sequence of one or more regions under the gene.

In the present application, the term "BEND4" generally refers to a gene or an expression product thereof. For example, the UniProt accession number of BEND4 (BEN domain-containing protein 4) protein may be Q6ZU67. In the present application, BEND4 may encompass its unprocessed form, any processed form, its variant or a substance comprising its functional active fragment. For example, BEND4 in the present application may also indicate a detection site, such as methylation detection for a specific nucleic acid sequence of the gene. For example, a detection site indicated by BEND4 may be a nucleic acid sequence of one or more regions under the gene.

In the present application, the term "sample to be tested" generally refers to a sample that needs to be tested. For example, it can be detected whether one or more gene regions on the sample to be tested are modified.

In the present application, the term "cell-free free nucleic acid" or "cfDNA" generally refers to DNA in a sample that, when collected, is not contained within a cell. For example, cell-free nucleic acid may not refer to DNA that is rendered non-intracellular by in vitro disruption of cells or tissues. For example, cfDNA may include DNA derived from both normal cells and cancer cells. For example, cfDNA may be obtained from blood or plasma ("circulatory system"). For example, cfDNA may be released into the circulatory system through secretion or cell death processes such as necrosis or apoptosis.

In the present application, the term "complementary nucleic acid" generally refers to nucleotide sequences that are complementary to a reference nucleotide sequence. For example, complementary nucleic acids can be nucleic acid molecules that optionally have opposite orientations. For example, the complementarity may refer to having the following complementary associations: guanine and cytosine; adenine and thymine; adenine and uracil.

In the present application, the term "DNA region" generally refers to the sequence of two or more covalently bound naturally occurring or modified deoxyribonucleotides. For example, the DNA region of a gene may refer to the position of a specific deoxyribonucleotide sequence where the gene is located, for example, the deoxyribonucleotide sequence encodes the gene. For example, the DNA region of the present application includes the full length of the DNA region, complementary regions thereof, or fragments thereof. For example, a sequence of at least about 20 kb upstream and downstream of the detection region provided in the present application may be used as a detection site. For example, a sequence of at least about 20 kb, at least about 15 kb, at least about 10 kb, at least about 5 kb, at least about 3 kb, at least about 2 kb, at least about 1 kb, or at least about 0.5 kb upstream and downstream of the region provided in the present application may be used as a detection site. For example, suitable primers and probes can be designed according to the microcomputer for methylation detection of the sample.

In the present application, the term "modification status" generally refers to a modification status that a gene fragment, a nucleotide, or a base thereof has in the present application. For example, the modification status in the present application may refer to the modification status of cytosine. For example, a gene fragment with a modification status in the present application may have altered gene expression activity. For example, the modification status in the present application may refer to the methylation modification of a base. For example, the modification status in the present application may refer to the covalent binding of a methyl group at the 5' carbon position of cytosine in the CpG region of genomic DNA, which may become 5-methylcytosine (5mC), for example. For example, the modification status may refer to the presence or absence of 5-methylcytosine ("5-mCyt") within the DNA sequence.

In the present application, the term "methylation" generally refers to the methylation status of a gene fragment, a nucleotide, or a base thereof in the present application. For example, the DNA fragment in which the gene is located in the present application may have methylation on one or more strands. For example, the DNA fragment in which the gene is located in the present application may have methylation on one or more sites.

In the present application, the term "transformation" generally refers to the transformation of one or more structures into another structure. For example, the transformation in the present application may be specific. For example, cytosine without methylation modification can be transformed into other structures (such as uracil), and cytosine with methylation modification can remain substantially unchanged after transformation. For example, cytosine without methylation modification can be cleaved after transformation, and cytosine with methylation modification can remain substantially unchanged after transformation.

In the present application, the term "deamination reagent" generally refers to a substance that has the ability to remove amino groups. For example, deamination reagents can remove amino groups from unmodified cytosine.

In the present application, the term "bisulfite" generally refers to a reagent that can differentiate a DNA region that has modification status from one that does not have modification status. For example, bisulfite may include bisulfite, or analogs thereof, or a combination thereof. For example, bisulfite can deaminate the amino group of unmodified cytosine to differentiate it from modified cytosine. In the present application, the term "analog" generally refers to a substance having a similar structure and/or function. For example, an analog of bisulfite may have a similar structure to bisulfite. For example, an analog of bisulfite may refer to a reagent that can also distinguish DNA regions with and without a modification status.

In the present application, the term "methylation-sensitive restriction enzyme" generally refers to an enzyme that selectively digests nucleic acids according to the methylation status of its recognition site. For example, for a restriction enzyme that specifically cleaves when the recognition site is unmethylated, cleavage may not occur or occur with significantly reduced efficiency when the recognition site is methylated. For a restriction enzyme that specifically cleaves when the recognition site is methylated, cleavage may not occur or occur with significantly reduced efficiency when the recognition site is unmethylated. For example, methylation-specific restriction enzymes can recognize sequences containing CG dinucleotides (e.g., cgcg or cccggg).

In the present application, the term "tumor" generally refers to cells and/or tissues that exhibit at least partial loss of control during normal growth and/or development. For example, common tumors or cancer cells may often have lost contact inhibition and may be invasive and/or have the ability to metastasize. For example, the tumor of the present application may be benign or malignant.

In the present application, the term "progress" generally refers to a change in the disease from a less severe condition to a more severe condition. For example, tumor progress may include an increase in the number or severity of tumors, the extent of cancer cell metastasis, and the rate at which the cancer grows or spreads. For example, tumor progress may include the progress of the cancer from a less severe state to a more severe state, such as from Stage I to Stage II, and from Stage II to Stage III.

In the present application, the term "development" generally refers to the occurrence of a lesion in an individual. For example, when a tumor is developed, the individual can be diagnosed as a tumor patient.

In the present application, the term "fluorescence PCR" generally refers to a quantitative or semi-quantitative PCR technique. For example, it can be a PCR technique of real-time quantitative polymerase chain reaction, quantitative polymerase chain reaction or kinetic polymerase chain reaction. For example, the initial amount of a target nucleic acid can be quantitatively detected by using PCR amplification with the aid of an intercalating fluorescent dye or a sequence-specific probe, and the sequence-specific probe can contain a fluorescent reporter molecule that is detectable only if it hybridizes to the target nucleic acid.

In the present application, the term "PCR amplification" generally refers to a polymerase chain reaction. For example, PCR amplification in the present application may comprise any polymerase chain amplification reaction currently known for use in DNA amplification.

In the present application, the term" fluorescence Ct value" generally refers to a measurement value for the quantitative or semi-quantitative assessment of a target nucleic acid. For example, it may refer to the number of amplification reaction cycles experienced when the fluorescence signal reaches a set threshold value.

### Detailed Description of the Invention

According to the present application, a variety of highly effective methylation markers related to liver tumor can be found, the efficiency of early screening and early diagnosis of liver tumor can be improved, and the problems of low early diagnosis rate of liver cancer and heavy burden of clinical treatment can be solved. At the same time, the present application provides multiple groups of efficient liver tumor methylation markers, and lists efficient marker combinations.

### Methylation marker combination

In one aspect, the present application provides a method for identifying the presence of a liver tumor, assessing the development or development risk of the liver tumor, and/or assessing the progress of the liver tumor, which may comprise determining the presence and/or the content of modification status of a DNA region in which a target gene is located, or a fragment thereof in a sample to be tested, wherein the target gene may comprise SEPT9 and IKZF1.

For example, the method of the present application may comprise identifying the presence or absence of a liver tumor based on the determination result of the presence and/or the content of modification status of a DNA region in which a target gene is located or a fragment thereof in a sample to be tested. For example, the method of the present application may comprise assessing whether the development of a liver tumor is diagnosed based on the determination result of the presence and/or the content of modification status of a DNA region in which a target gene is located or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which a target gene is located or a fragment thereof in a sample to be tested. For example, the method of the present application may comprise assessing the progress of a liver tumor based on the determination result of the presence and/or the content of modification status of a DNA region in which a target gene is located or a fragment thereof in a sample to be tested.

In another aspect, the present application provides a method for assessing the methylation status of a liver tumor-related DNA region, which may comprise determining the presence and/or the content of modification status of a DNA region in which a target gene is located or a fragment thereof in a sample to be tested, wherein the target gene may comprise SEPT9 and IKZF1.

For example, the methylation status of a liver tumor-related DNA region is assessed based on the determination of the presence and/or the content of modification status of a DNA region in which a target gene is located or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg 19 coordinates.

For example, in the method of the present application, the DNA region of SEPT9 may be derived from human chr17:75276651-75496678.

For example, in the method of the present application, the DNA region of IKZF1 may be derived from human chr7:50343720-50472799.

For example, in the method of the present application, the target gene may further comprise a gene selected from the group consisting of BEST4, B4GALNT1, GRASP, IRF4 and BEND4.

For example, in the method of the present application, the target gene may comprise SEPT9, IKZF1, BEST4 and B4GALNT1. For example, in the method of the present application, the target gene may comprise SEPT9, IKZF1, BEST4, GRASP and B4GALNT1. For example, in the method of the present application, the target gene may comprise SEPT9, IKZF1, BEST4, IRF4, B4GALNT1 and BEND4.

For example, in the method of the present application, the target gene may comprise at least 2 genes.

For example, in the method of the present application, the target gene may comprise 2 to 7 genes. For example, the target genes of the present application may comprise 2, 3, 4, 5, 6, or 7 target genes provided by the present application. For example, for the same target gene, the present application can select one or more DNA regions in which the target gene is located. For example, in the method of the present application, the DNA region of BEST4 may be derived from human chr1:45249257-45253377. For example, in the method of the present application, the DNA region of B4GALNT1 may be derived from human chr12:58017193-58027138. For example, in the method of the present application, the DNA region of GRASP may be derived from human chr12:52400724-52409673. For example, in the method of the present application, the DNA region of IRF4 may be derived from human chr6:391739-411447. For example, in the method of the present application, the DNA region of BEND4 may be derived from human chr4:42112955-42154895.

For example, in the method of the present application, the target gene may comprise 2 genes selected from the group consisting of SEPT9, IKZF1, BEST4, B4GALNT1, GRASP, IRF4 and BEND4. For example, in the method of the present application, the target gene may comprise SEPT9 and IKZF1. For example, in the method of the present application, the target gene may comprise SEPT9 and BEST4. For example, in the method of the present application, the target gene may comprise SEPT9 and B4GALNT1. For example, in the method of the present application, the target gene may comprise SEPT9 and GRASP. For example, in the method of the present application, the target gene may comprise SEPT9 and IRF4. For example, in the method of the present application, the target gene may comprise SEPT9 and BEND4. For example, in the method of the present application, the target gene may comprise IKZF1 and BEST4. For example, in the method of the present application, the target gene may comprise IKZF1 and B4GALNT1. For example, in the method of the present application, the target gene may comprise IKZF1 and GRASP. For example, in the method of the present application, the target gene may comprise IKZF1 and IRF4. For example, in the method of the present application, the target gene may comprise IKZF1 and BEND4. For example, in the method of the present application, the target gene may comprise BEST4 and B4GALNT1. For example, in the method of the present application, the target gene may comprise BEST4 and GRASP. For example, in the method of the present application, the target gene may comprise BEST4 and IRF4. For example, in the method of the present application, the target gene may comprise BEST4 and BEND4. For example, in the method of the present application, the target gene may comprise B4GALNT1 and GRASP. For example, in the method of the present application, the target gene may comprise B4GALNT1 and IRF4. For example, in the method of the present application, the target gene may comprise B4GALNT1 and BEND4. For example, in the method of the present application, the target gene may comprise GRASP and IRF4. For example, in the method of the present application, the target gene may comprise GRASP and BEND4. For example, in the method of the present application, the target gene may comprise IRF4 and BEND4.

For example, in the method of the present application, the target gene may comprise 3 genes selected from the group consisting of SEPT9, IKZF1, BEST4, B4GALNT1, GRASP, IRF4 and BEND4. For example, in the method of the present application, the target gene may comprise SEPT9, IKZF1 and BEST4. For example, in the method of the present application, the target gene may comprise SEPT9, IKZF1 and B4GALNT1. For example, in the method of the present application, the target gene may comprise SEPT9, IKZF1 and GRASP. For example, in the method of the present application, the target gene may comprise SEPT9, IKZF1 and IRF4. For example, in the method of the present application, the target gene may comprise SEPT9, IKZF1 and BEND4. For example, in the method of the present application, the target gene may comprise SEPT9, BEST4 and B4GALNT1. For example, in the method of the present application, the target gene may comprise SEPT9, BEST4 and GRASP. For example, in the method of the present application, the target gene may comprise SEPT9, BEST4 and IRF4. For example, in the method of the present application, the target gene may comprise SEPT9, BEST4 and BEND4. For example, in the method of the present application, the target gene may comprise SEPT9, B4GALNT1 and GRASP. For example, in the method of the present application, the target gene may comprise SEPT9, B4GALNT1 and IRF4. For example, in the method of the present application, the target gene may comprise SEPT9, B4GALNT1 and BEND4. For example, in the method of the present application, the target gene may comprise SEPT9, GRASP and IRF4. For example, in the method of the present application, the target gene may comprise SEPT9, GRASP and BEND4. For example, in the method of the present application, the target gene may comprise SEPT9, IRF4 and BEND4. For example, in the method of the present application, the target gene may comprise IKZF1, BEST4 and B4GALNT1. For example, in the method of the present application, the target gene may comprise IKZF1, BEST4 and GRASP. For example, in the method of the present application, the target gene may comprise IKZF1, BEST4 and IRF4. For example, in the method of the present application, the target gene may comprise IKZF1, BEST4 and BEND4. For example, in the method of the present application, the target gene may comprise IKZF1, B4GALNT1 and GRASP. For example, in the method of the present application, the target gene may comprise IKZF1, B4GALNT1 and IRF4. For example, in the method of the present application, the target gene may comprise IKZF1, B4GALNT1 and BEND4. For example, in the method of the present application, the target gene may comprise IKZF1, GRASP and IRF4. For example, in the method of the present application, the target gene may comprise IKZF1, GRASP and BEND4. For example, in the method of the present application, the target gene may comprise IKZF1, IRF4 and BEND4. For example, in the method of the present application, the target gene may comprise BEST4, B4GALNT1 and GRASP. For example, in the method of the present application, the target gene may comprise BEST4, B4GALNT1 and IRF4. For example, in the method of the present application, the target gene may comprise BEST4, B4GALNT1 and BEND4. For example, in the method of the present application, the target gene may comprise BEST4, GRASP and IRF4. For example, in the method of the present application, the target gene may comprise BEST4, GRASP and BEND4. For example, in the method of the present application, the target gene may comprise BEST4, IRF4 and BEND4. For example, in the method of the present application, the target gene may comprise B4GALNT1, GRASP and IRF4. For example, in the method of the present application, the target gene may comprise B4GALNT1, GRASP and BEND4. For example, in the method of the present application, the target gene may comprise B4GALNT1, IRF4 and BEND4. For example, in the method of the present application, the target gene may comprise GRASP, IRF4 and BEND4.

For example, in the method of the present application, the target gene may comprise 4 genes selected from the group consisting of SEPT9, IKZF1, BEST4, B4GALNT1, GRASP, IRF4 and BEND4. For example, in the method of the present application, the target gene may comprise SEPT9, IKZF1, BEST4 and B4GALNT1. For example, in the method of the present application, the target gene may comprise SEPT9, IKZF1, BEST4 and GRASP. For example, in the method of the present application, the target gene may comprise SEPT9, IKZF1, BEST4 and IRF4. For example, in the method of the present application, the target gene may comprise SEPT9, IKZF1, BEST4 and BEND4. For example, in the method of the present application, the target gene may comprise SEPT9, IKZF1, B4GALNT1 and GRASP. For example, in the method of the present application, the target gene may comprise SEPT9, IKZF1, B4GALNT1 and IRF4. For example, in the method of the present application, the target gene may comprise SEPT9, IKZF1, B4GALNT1 and BEND4. For example, in the method of the present application, the target gene may comprise SEPT9, IKZF1, GRASP and IRF4. For example, in the method of the present application, the target gene may comprise SEPT9, IKZF1, GRASP and BEND4. For example, in the method of the present application, the target gene may comprise SEPT9, IKZF1, IRF4 and BEND4. For example, in the method of the present application, the target gene may comprise SEPT9, BEST4, B4GALNT1 and GRASP. For example, in the method of the present application, the target gene may comprise SEPT9, BEST4, B4GALNT1 and IRF4. For example, in the method of the present application, the target gene may comprise SEPT9, BEST4, B4GALNT1 and BEND4. For example, in the method of the present application, the target gene may comprise SEPT9, BEST4, GRASP and IRF4. For example, in the method of the present application, the target gene may comprise SEPT9, BEST4, GRASP and BEND4. For example, in the method of the present application, the target gene may comprise SEPT9, BEST4, IRF4 and BEND4. For example, in the method of the present application, the target gene may comprise SEPT9, B4GALNT1, GRASP and IRF4. For example, in the method of the present application, the target gene may comprise SEPT9, B4GALNT1, GRASP and BEND4. For example, in the method of the present application, the target gene may comprise SEPT9, B4GALNT1, IRF4 and BEND4. For example, in the method of the present application, the target gene may comprise SEPT9, GRASP, IRF4 and BEND4. For example, in the method of the present application, the target gene may comprise IKZF1, BEST4, B4GALNT1 and GRASP. For example, in the method of the present application, the target gene may comprise IKZF1, BEST4, B4GALNT1 and IRF4. For example, in the method of the present application, the target gene may comprise IKZF1, BEST4, B4GALNT1 and BEND4. For example, in the method of the present application, the target gene may comprise IKZF1, BEST4, GRASP and IRF4. For example, in the method of the present application, the target gene may comprise IKZF1, BEST4, GRASP and BEND4. For example, in the method of the present application, the target gene may comprise IKZF1, BEST4, IRF4 and BEND4. For example, in the method of the present application, the target gene may comprise IKZF1, B4GALNT1, GRASP and IRF4. For example, in the method of the present application, the target gene may comprise IKZF1, B4GALNT1, GRASP and BEND4. For example, in the method of the present application, the target gene may comprise IKZF1, B4GALNT1, IRF4 and BEND4. For example, in the method of the present application, the target gene may comprise IKZF1, GRASP, IRF4 and BEND4. For example, in the method of the present application, the target gene may comprise BEST4, B4GALNT1, GRASP and IRF4. For example, in the method of the present application, the target gene may comprise BEST4, B4GALNT1, GRASP and BEND4. For example, in the method of the present application, the target gene may comprise BEST4, B4GALNT1, IRF4 and BEND4. For example, in the method of the present application, the target gene may comprise B4GALNT1, GRASP, IRF4 and BEND4.

For example, in the method of the present application, the target gene may comprise 5 genes selected from the group consisting of SEPT9, IKZF1, BEST4, B4GALNT1, GRASP, IRF4 and BEND4. For example, in the method of the present application, the target gene may comprise SEPT9, IKZF1, BEST4, B4GALNT1 and GRASP. For example, in the method of the present application, the target gene may comprise SEPT9, IKZF1, BEST4, B4GALNT1 and IRF4. For example, in the method of the present application, the target gene may comprise SEPT9, IKZF1, BEST4, B4GALNT1 and BEND4. For example, in the method of the present application, the target gene may comprise SEPT9, IKZF1, BEST4, GRASP and IRF4. For example, in the method of the present application, the target gene may comprise SEPT9, IKZF1, BEST4, GRASP and BEND4. For example, in the method of the present application, the target gene may comprise SEPT9, IKZF1, BEST4, IRF4 and BEND4. For example, in the method of the present application, the target gene may comprise SEPT9, BEST4, B4GALNT1, GRASP and IRF4. For example, in the method of the present application, the target gene may comprise SEPT9, BEST4, B4GALNT1, GRASP and BEND4. For example, in the method of the present application, the target gene may comprise SEPT9, BEST4, B4GALNT1, IRF4 and BEND4. For example, in the method of the present application, the target gene may comprise SEPT9, B4GALNT1, GRASP, IRF4 and BEND4. For example, in the method of the present application, the target gene may comprise IKZF1, BEST4, B4GALNT1, GRASP and IRF4. For example, in the method of the present application, the target gene may comprise IKZF1, BEST4, B4GALNT1, GRASP and BEND4. For example, in the method of the present application, the target gene may comprise IKZF1, BEST4, B4GALNT1, IRF4 and BEND4. For example, in the method of the present application, the target gene may comprise IKZF1, B4GALNT1, GRASP, IRF4 and BEND4. For example, in the method of the present application, the target gene may comprise BEST4, B4GALNT1, GRASP, IRF4 and BEND4.

For example, in the method of the present application, the target gene may comprise 6 genes selected from the group consisting of SEPT9, IKZF1, BEST4, B4GALNT1, GRASP, IRF4 and BEND4. For example, in the method of the present application, the target gene may comprise SEPT9, IKZF1, BEST4, B4GALNT1, GRASP and IRF4. For example, in the method of the present application, the target gene may comprise SEPT9, IKZF1, BEST4, B4GALNT1, GRASP and BEND4. For example, in the method of the present application, the target gene may comprise SEPT9, IKZF1, BEST4, B4GALNT1, IRF4 and BEND4. For example, in the method of the present application, the target gene may comprise SEPT9, IKZF1, BEST4, GRASP, IRF4 and BEND4. For example, in the method of the present application, the target gene may comprise SEPT9, IKZF1, B4GALNT1, GRASP, IRF4 and BEND4. For example, in the method of the present application, the target gene may comprise SEPT9, BEST4, B4GALNT1, GRASP, IRF4 and BEND4. For example, in the method of the present application, the target gene may comprise IKZF1, BEST4, B4GALNT1, GRASP, IRF4 and BEND4.

For example, in the method of the present application, the target gene may comprise 7 genes selected from the group consisting of SEPT9, IKZF1, BEST4, B4GALNT1, GRASP, IRF4 and BEND4. For example, in the method of the present application, the target gene may comprise SEPT9, IKZF1, BEST4, B4GALNT1, GRASP, IRF4 and BEND4.

For example, a target DNA region located in the DNA region in which SEPT9 gene is located in the method of the present application may comprise a region derived from the region defined by human chr17:75368651-75370720. For example, it is derived from the region defined by human chr17:75369558-75369622. For example, the target DNA region may be indicated by SEPT (1).

For example, a target DNA region located in the DNA region in which IKZF1 gene is located in the method of the present application may comprise a region derived from the region defined by human chr7:50343720-50344547. For example, it is derived from the region defined by human chr7:50343793-50343896. For example, the target DNA region may be indicated by IKZF1 (1).

For example, a target DNA region located in the DNA region in which BEST4 gene is located in the method of the present application may comprise a region derived from the region defined by human chr1:45251728-45252477. For example, it is derived from the region defined by human chr1:45252095-45252176. For example, the target DNA region may be indicated by BEST4 (1).

For example, a target DNA region located in the DNA region in which B4GALNT1 gene is located in the method of the present application may comprise a region derived from the region defined by human chr12:58020498-58022962. For example, it is derived from the region defined by human chr12:58021586-58021670. For example, the target DNA region may be indicated by B4GALNT1 (1).

For example, a target DNA region located in the DNA region in which GRASP gene is located in the method of the present application may comprise a region derived from the region defined by human chr12:52400724-52401698. For example, it is derived from the region defined by human chr12:52401083-52401169. For example, the target DNA region may be indicated by GRASP (1).

For example, a target DNA region located in the DNA region in which IRF4 gene is located in the method of the present application may comprise a region derived from the region defined by human chr6:391739-394056. For example, it is derived from the region defined by human chr6:392282-392377. For example, the target DNA region may be indicated by IRF4 (1).

For example, a target DNA region located in the DNA region in which BEND4 gene is located in the method of the present application may comprise a region derived from the region defined by human chr4:42152705-42154895. For example, it is derived from the region defined by human chr4:42153816-42153921. For example, the target DNA region may be indicated by BEND4 (1).

For example, a target DNA region located in the DNA region in which SEPT9 gene is located in the method of the present application may comprise a region derived from the region defined by human chr17:75368651-75370720. For example, it is derived from the region defined by human chr17:75369603-75369693. For example, the target DNA region may be indicated by SEPT9 (1a).

In another aspect, the present application provides a method for identifying the presence of a disease, assessing the development or development risk of the disease, and/or assessing the progress of the disease, which may comprise determining the presence and/or the content of modification status of a specific subregion of a DNA region in which a target gene is located (such as a target DNA region), or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a target DNA region, or a complementary region thereof, or a fragment thereabove in a sample to be tested, wherein the target DNA region may comprise regions defined from human chr17:75368651-75370720 and from human chr7:50343720-50344547. For example, the method of the present application may comprise identifying the presence or absence of a disease based on the determination result of the presence and/or the content of modification status of the target DNA region, or a complementary region thereof or a fragment thereabove in a sample to be tested. For example, the method of the present application may comprise assessing whether a disease development is diagnosed based on the determination result of the presence and/or the content of modification status of the target DNA region, or a complementary region thereof or a fragment thereabove in a sample to be tested. For example, the method of the present application may comprise assessing whether a disease risk is diagnosed and/or the level of the risk based on the determination result of the presence and/or the content of modification status of the target DNA region, or a complementary region thereof or a fragment thereabove in a sample to be tested. For example, the method of the present application may comprise assessing the progress of a disease based on the determination result of the presence and/or the content of modification status of the target DNA region, or a complementary region thereof or a fragment thereabove in a sample to be tested.

In another aspect, the present application provides a method for determining a methylation status of a DNA region, which may comprise determining the presence and/or the content of modification status of a target DNA region, or a complementary region thereof, or a fragment thereabove in a sample to be tested, wherein the target DNA region may comprises regions defined from human chr17:75368651-75370720 and from human chr7:50343720-50344547.

For example, the identified presence or increased amount of the methylation of the target DNA region, relative to a reference level, may be correlated with the occurrence of a disease. For example, the target DNA region of the present application may refer to one or more specific segments of a target genomic DNA. For example, the target DNA region of the present application may be specified by a gene name or a set of chromosomal coordinates. For example, the target DNA region indicated by SEPT9 can be one or more specific segments of a DNA region in which SEPT9 gene is located. For example, the target DNA region indicated by SEPT9 can be one or more specific segments of a target DNA region in the SEPT9 gene provided by the present application. For example, one or more different specific segments of SEPT9 can be indicated respectively by distinguishable forms such as SEPT9 (1) and SEPT9 (la), etc.

For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region comprising SEQ ID NO: 1, or a complementary region thereof, or a transformed region thereabove, or a fragment thereabove.

For example, in the method of the present application, the target region may comprise a region defined from human chr17:75369558-75369622. For example, the target DNA region may be indicated by SEPT (1).

For example, the method of the present application may comprise providing a nucleic acid as set forth in SEQ ID NO: 2, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 3 and 4, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr17:75369558-75369622.

For example, the method of the present application may comprise providing a nucleic acid capable of binding to a DNA region comprising SEQ ID NO: 5, or a complementary region thereof, or a transformed region thereabove, or a fragment thereabove.

For example, in the method of the present application, the target region may comprise a region defined from human chr7:50343793-50343896. For example, the target DNA region may be indicated by IKZF1 (1).

For example, the method of the present application may comprise providing a nucleic acid as set forth in SEQ ID NO: 6, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 7 and 8, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr7:50343793-50343896.

For example, in the method of the present application, the target DNA region may further comprise a region selected from the group consisting of the regions defined from human chr1:45251728-45252477, from human chr12:58020498-58022962, from human chr12:52400724-52401698, from human chr6:391739-394056, and from human chr4:42152705-42154895.

For example, in the method of the present application, the target DNA region may comprise at least 2 regions.

For example, in the method of the present application, the target DNA region may comprise 2 to 8 regions. For example, in the method of the present application, the target DNA region may comprise 2, 3, 4, 5, 6, 7, or 8 regions.

For example, in the method of the present application, the target DNA region may comprise a region defined from human chr17: 75368651-75370720, from human chr7: 50343720-50344547, from human chr1: 45251728-45252477, and from human chr12: 58020498-58022962.

For example, in the method of the present application, the target DNA region may comprise a region defined from human chr17: 75368651-75370720, from human chr7: 50343720-50344547, from human chr1: 45251728-45252477, from human chr12:52400724-52401698, and from human chr12: 58020498-58022962.

For example, in the method of the present application, the target DNA region may comprise a region defined from human chr6:391739-394056, from human chr7:50343720-50344547, from human chr1:45251728-45252477, from human chr12:58020498-58022962, from human chr4:42152705-42154895, and from human chr17:75368651-75370720.

For example, the method of the present application may comprise providing a nucleic acid capable of binding to a DNA region comprising SEQ ID NO: 9, or a complementary region thereof, or a transformed region thereabove, or a fragment thereabove.

For example, in the method of the present application, the target region may comprise a region defined from human chr1:45252095-45252176. For example, the target DNA region may be indicated by BEST4 (1).

For example, the method of the present application may comprise providing a nucleic acid as set forth in SEQ ID NO: 10, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 11 and 12, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr1:45252095-45252176.

For example, the method of the present application may comprise providing a nucleic acid capable of binding to a DNA region comprising SEQ ID NO: 13, or a complementary region thereof, or a transformed region thereabove, or a fragment thereabove.

For example, in the method of the present application, the target region may comprise a region defined from human chr12:58021586-58021670. For example, the target DNA region may be indicated by B4GALNT1 (1).

For example, the method of the present application may comprise providing a nucleic acid as set forth in SEQ ID NO: 14, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 15 and 16, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr12:58021586-58021670.

For example, the method of the present application may comprise providing a nucleic acid capable of binding to a DNA region comprising SEQ ID NO: 17, or a complementary region thereof, or a transformed region thereabove, or a fragment thereabove.

For example, in the method of the present application, the target region may comprise a region defined from human chr12:52401083-52401169. For example, the target DNA region may be indicated by GRASP (1).

For example, the method of the present application may comprise providing a nucleic acid as set forth in SEQ ID NO: 18, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 19 and 20, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr12:52401083-52401169.

For example, the method of the present application may comprise providing a nucleic acid capable of binding to a DNA region comprising SEQ ID NO: 21, or a complementary region thereof, or a transformed region thereabove, or a fragment thereabove.

For example, in the method of the present application, the target region may comprise a region defined from human chr6:392282-392377. For example, the target DNA region may be indicated by IRF4 (1).

For example, the method of the present application may comprise providing a nucleic acid as set forth in SEQ ID NO: 22, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 23 and 24, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr6:392282-392377.

For example, the method of the present application may comprise providing a nucleic acid capable of binding to a DNA region comprising SEQ ID NO: 25, or a complementary region thereof, or a transformed region thereabove, or a fragment thereabove.

For example, in the method of the present application, the target region may comprise a region defined from human chr4:42153816-42153921. For example, the target DNA region may be indicated by BEND4 (1).

For example, the method of the present application may comprise providing a nucleic acid as set forth in SEQ ID NO: 26, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 27 and 28, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr4:42153816-42153921.

For example, the method of the present application may comprise providing a nucleic acid capable of binding to a DNA region comprising SEQ ID NO: 1, or a complementary region thereof, or a transformed region thereabove, or a fragment thereabove.

For example, in the method of the present application, the target region may comprise a region defined from human chr17:75369603-75369693. For example, the target DNA region may be indicated by SEPT9 (1a).

For example, the method of the present application may comprise providing a nucleic acid as set forth in SEQ ID NO: 29, or a complementary nucleic acid thereof, or a fragment thereabove. For example, the nucleic acid may be used to detect a target region. For example, the nucleic acid may be used as a probe.

For example, the method of the present application may comprise providing a nucleic acid set as set forth in SEQ ID NO: 30 and 31, or a complementary nucleic acid set thereof, or a fragment thereabove. For example, the nucleic acid set may be used to amplify a target region. For example, the nucleic acid set may serve as a primer set.

For example, the above probe and/or primer set may detect and/or amplify a DNA region derived from a region defined by human chr17:75369603-75369693.

For example, one or more of the above target regions may serve as amplification regions and/or detection regions.

### Methylation marker

In one aspect, the present application provides a method for identifying the presence of a liver tumor, assessing the development or development risk of the liver tumor, and/or assessing the progress of the liver tumor, which may comprise determining the presence and/or the content of modification status of a DNA region in which BCAT1 gene is located or a fragment thereof in a sample to be tested. For example, the method of the present application may comprise identifying the presence or absence of a liver tumor based on the determination result of the presence and/or the content of modification status of a DNA region in which BCAT1 gene is located or a fragment thereof in a sample to be tested. For example, the method of the present application may comprise assessing whether the development of a liver tumor is diagnosed based on the determination result of the presence and/or the content of modification status of a DNA region in which BCAT1 gene is located or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which BCAT1 gene is located or a fragment thereof in a sample to be tested. For example, the method of the present application may comprise assessing the progress of a liver tumor based on the determination result of the presence and/or the content of modification status of a DNA region in which BCAT1 gene is located or a fragment thereof in a sample to be tested.

In another aspect, the present application provides a method for assessing the methylation status of a liver tumor-related DNA region, which may comprise determining the presence and/or the content of modification status of a DNA region in which BCAT1 gene is located or a fragment thereof in a sample to be tested. For example, the methylation status of a liver tumor-related DNA region is assessed based on the determination of the presence and/or the content of modification status of a DNA region in which BCAT1 gene is located or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr12:24964295-25102393. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific subregion of a DNA region in which BCAT1 gene is located, or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from the following group, or a complementary region thereof, or a fragment thereabove in a sample to be tested: derived from human chr12:25101630-25102393, derived from human chr:12:25078661-25079410, and derived from human chr12:25054781-25056311. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr12:25101630-25102393, derived from human chr:12:25078661-25079410, and derived from human chr12:25054781-25056311. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining a methylation status of a DNA region, which may comprise determining the presence and/or the content of modification status of a DNA region derived from human chr12:25101630-25102393, or a complementary region thereof, or a fragment thereabove in a sample to be tested.

In another aspect, the present application provides a method for determining a methylation status of a DNA region, which may comprise determining the presence and/or the content of modification status of a DNA region derived from human chr:12:25078661-25079410, or a complementary region thereof, or a fragment thereabove in a sample to be tested.

In another aspect, the present application provides a method for determining a methylation status of a DNA region, which may comprise determining the presence and/or the content of modification status of a DNA region derived from human chr12:25054781-25056311, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region which may comprise a region selected from the group consisting of SEQ ID NO: 32, SEQ ID NO: 39 and SEQ ID NO: 43, or a complementary region thereof, or a transformed region thereabove, or a fragment thereabove.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr12:25102016-25102110, derived from human chr12:25101992-25102093, derived from human chr12:25079051-25079133, and derived from human chr12:25056027-25056134.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr12:25102016-25102110, derived from human chr12:25101992-25102093, derived from human chr12:25079051-25079133, and derived from human chr12:25056027-25056134.

For example, the method of the present application may comprise providing a nucleic acid capable of binding to a DNA region which may comprise a region selected from the following group, or a complementary region thereof, or a transformed region thereabove, or a fragment thereabove: derived from human chr12:25102016-25102110, derived from human chr12:25101992-25102093, derived from human chr12:25079051-25079133, and derived from human chr12:25056027-25056134.

For example, one or more of the above regions may serve as amplification regions and/or detection regions.

For example, the method of the present application may comprise providing a nucleic acid selectable from the group consisting of SEQ ID NO: 33, SEQ ID NO: 36, SEQ ID NO: 40 and SEQ ID NO: 44, or a complementary nucleic acid thereof, or a fragment thereabove. For example, the nucleic acid may be used to detect a target region. For example, the nucleic acid may be used as a probe.

For example, the method of the present application may comprise providing a nucleic acid as set forth in SEQ ID NO: 33, or a complementary nucleic acid thereof, or a fragment thereabove. For example, the nucleic acid as a probe may be used to detect the DNA region derived from human chr12:25101630-25102393. For example, the nucleic acid as a probe may be used to detect the DNA region derived from human chr12:25102016-25102110.

For example, the method of the present application may comprise providing a nucleic acid as set forth in SEQ ID NO: 36, or a complementary nucleic acid thereof, or a fragment thereabove. For example, the nucleic acid as a probe may be used to detect the DNA region derived from human chr12:25101630-25102393. For example, the nucleic acid as a probe may be used to detect the DNA region derived from human chr12:25101992-25102093.

For example, the method of the present application may comprise providing a nucleic acid as set forth in SEQ ID NO: 40, or a complementary nucleic acid thereof, or a fragment thereabove. For example, the nucleic acid as a probe may be used to detect the DNA region derived from human chr:12:25078661-25079410. For example, the nucleic acid as a probe may be used to detect the DNA region derived from human chr12:25079051-25079133.

For example, the method of the present application may comprise providing a nucleic acid as set forth in SEQ ID NO: 44, or a complementary nucleic acid thereof, or a fragment thereabove. For example, the nucleic acid as a probe may be used to detect the DNA region derived from human chr12:25054781-25056311. For example, the nucleic acid as a probe may be used to detect the DNA region derived from human chr12:25056027-25056134.

For example, the method of the present application may comprise providing a nucleic acid set selectable from the following group, or a complementary nucleic acid set thereof, or a fragment thereabove: SEQ ID NO: 34 and 35, SEQ ID NO: 37 and 38, SEQ ID NO: 41 and 42, and SEQ ID NO: 45 and 46. For example, the nucleic acid set may be used to amplify a target region. For example, the nucleic acid set may serve as a primer set.

For example, the method of the present application may comprise providing a nucleic acid set as set forth in SEQ ID NO: 34 and 35, or a complementary nucleic acid set thereof, or a fragment thereabove. For example, the nucleic acid set as a primer set may be used to amplify the DNA region derived from human chr12:25101630-25102393. For example, the nucleic acid set as a primer set may be used to amplify the DNA region derived from human chr12:25102016-25102110.

For example, the method of the present application may comprise providing a nucleic acid set as set forth in SEQ ID NO: 37 and 38, or a complementary nucleic acid set thereof, or a fragment thereabove. For example, the nucleic acid set as a primer set may be used to amplify the DNA region derived from human chr12:25101630-25102393. For example, the nucleic acid set as a primer set may be used to amplify the DNA region derived from human chr12:25101992-25102093.

For example, the method of the present application may comprise providing a nucleic acid set as set forth in SEQ ID NO: 41 and 42, or a complementary nucleic acid set thereof, or a fragment thereabove. For example, the nucleic acid set as a primer set may be used to amplify the DNA region derived from human chr:12:25078661-25079410. For example, the nucleic acid set as a primer set may be used to amplify the DNA region derived from human chr12:25079051-25079133.

For example, the method of the present application may comprise providing a nucleic acid set as set forth in SEQ ID NO: 45 and 46, or a complementary nucleic acid set thereof, or a fragment thereabove. For example, the nucleic acid set as a primer set may be used to amplify the DNA region derived from human chr12:25054781-25056311. For example, the nucleic acid set as a primer set may be used to amplify the DNA region derived from human chr12:25056027-25056134.

In one aspect, the present application provides a method for identifying the presence of a liver tumor, assessing the development or development risk of the liver tumor, and/or assessing the progress of the liver tumor, which may comprise determining the presence and/or the content of modification status of a DNA region in which IKZF1 gene is located or a fragment thereof in a sample to be tested. For example, the method of the present application may comprise identifying the presence or absence of a liver tumor based on the determination result of the presence and/or the content of modification status of a DNA region in which IKZF1 gene is located or a fragment thereof in a sample to be tested. For example, the method of the present application may comprise assessing whether the development of a liver tumor is diagnosed based on the determination result of the presence and/or the content of modification status of a DNA region in which IKZF1 gene is located or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which IKZF 1 gene is located or a fragment thereof in a sample to be tested. For example, the method of the present application may comprise assessing the progress of a liver tumor based on the determination result of the presence and/or the content of modification status of a DNA region in which IKZF1 gene is located or a fragment thereof in a sample to be tested.

In another aspect, the present application provides a method for assessing the methylation status of a liver tumor-related DNA region, which may comprise determining the presence and/or the content of modification status of a DNA region in which IKZF 1 gene is located or a fragment thereof in a sample to be tested. For example, the methylation status of a liver tumor-related DNA region is assessed based on the determination of the presence and/or the content of modification status of a DNA region in which IKZF1 gene is located or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr7:50343720:50472799. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific subregion of a DNA region in which IKZF 1 gene is located, or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from the following group, or a complementary region thereof, or a fragment thereabove in a sample to be tested: derived from human chr7:50343720-50344547, derived from human chr7:50450118-50450531, and derived from human chr7:50467368-50469092. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr7:50343720-50344547, derived from human chr7:50450118-50450531, and derived from human chr7:50467368-50469092. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining a methylation status of a DNA region, which may comprise determining the presence and/or the content of modification status of a DNA region derived from human chr7:50343720-50344547, or a complementary region thereof, or a fragment thereabove in a sample to be tested.

In another aspect, the present application provides a method for determining a methylation status of a DNA region, which may comprise determining the presence and/or the content of modification status of a DNA region derived from human chr7:50450118-50450531, or a complementary region thereof, or a fragment thereabove in a sample to be tested.

In another aspect, the present application provides a method for determining a methylation status of a DNA region, which may comprise determining the presence and/or the content of modification status of a DNA region derived from human chr7:50467368-50469092, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region which may comprise a region selected from the group consisting of SEQ ID NO: 5, SEQ ID NO: 50 and SEQ ID NO: 54, or a complementary region thereof, or a transformed region thereabove, or a fragment thereabove.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr7:50343793-50343896, derived from human chr7:50343867-50343961, derived from human chr7:50450311-50450411, and derived from human chr7:50467865-50467980.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr7:50343793-50343896, derived from human chr7:50343867-50343961, derived from human chr7:50450311-50450411, and derived from human chr7:50467865-50467980.

For example, the method of the present application may comprise providing a nucleic acid capable of binding to a DNA region which may comprise a region selected from the following group, or a complementary region thereof, or a transformed region thereabove, or a fragment thereabove: derived from human chr7:50343793-50343896, derived from human chr7:50343867-50343961, derived from human chr7:50450311-50450411, and derived from human chr7:50467865-50467980.

For example, one or more of the above regions may serve as amplification regions and/or detection regions.

For example, the method of the present application may comprise providing a nucleic acid selectable from the group consisting of SEQ ID NO: 6, SEQ ID NO: 47, SEQ ID NO: 51 and SEQ ID NO: 55, or a complementary nucleic acid thereof, or a fragment thereabove. For example, the nucleic acid may be used to detect a target region. For example, the nucleic acid may be used as a probe.

For example, the method of the present application may comprise providing a nucleic acid as set forth in SEQ ID NO: 6, or a complementary nucleic acid thereof, or a fragment thereabove. For example, the nucleic acid as a probe may be used to detect the DNA region derived from human chr7:50343720-50344547. For example, the nucleic acid as a probe may be used to detect the DNA region derived from human chr7:50343793-50343896.

For example, the method of the present application may comprise providing a nucleic acid as set forth in SEQ ID NO: 47, or a complementary nucleic acid thereof, or a fragment thereabove. For example, the nucleic acid as a probe may be used to detect the DNA region derived from human chr7:50343720-50344547. For example, the nucleic acid as a probe may be used to detect the DNA region derived from human chr7:50343867-50343961.

For example, the method of the present application may comprise providing a nucleic acid as set forth in SEQ ID NO: 51, or a complementary nucleic acid thereof, or a fragment thereabove. For example, the nucleic acid as a probe may be used to detect the DNA region derived from human chr7:50450118-50450531. For example, the nucleic acid as a probe may be used to detect the DNA region derived from human chr7:50450311-50450411.

For example, the method of the present application may comprise providing a nucleic acid as set forth in SEQ ID NO: 55, or a complementary nucleic acid thereof, or a fragment thereabove. For example, the nucleic acid as a probe may be used to detect the DNA region derived from human chr7:50467368-50469092. For example, the nucleic acid as a probe may be used to detect the DNA region derived from human chr7:50467865-50467980.

For example, the method of the present application may comprise providing a nucleic acid set selectable from the following group, or a complementary nucleic acid set thereof, or a fragment thereabove: SEQ ID NO: 7 and 8, SEQ ID NO: 48 and 49, SEQ ID NO: 52 and 53, and SEQ ID NO: 56 and 57. For example, the nucleic acid set may be used to amplify a target region. For example, the nucleic acid set may serve as a primer set.

For example, the method of the present application may comprise providing a nucleic acid set as set forth in SEQ ID NO: 7 and 8, or a complementary nucleic acid set thereof, or a fragment thereabove. For example, the nucleic acid set as a primer set may be used to amplify the DNA region derived from human chr7:50343720-50344547. For example, the nucleic acid set as a primer set may be used to amplify the DNA region derived from human chr7:50343793-50343896.

For example, the method of the present application may comprise providing a nucleic acid set as set forth in SEQ ID NO: 48 and 49, or a complementary nucleic acid set thereof, or a fragment thereabove. For example, the nucleic acid set as a primer set may be used to amplify the DNA region derived from human chr7:50343720-50344547. For example, the nucleic acid set as a primer set may be used to amplify the DNA region derived from human chr7:50343867-50343961.

For example, the method of the present application may comprise providing a nucleic acid set as set forth in SEQ ID NO: 52 and 53, or a complementary nucleic acid set thereof, or a fragment thereabove. For example, the nucleic acid set as a primer set may be used to amplify the DNA region derived from human chr7:50450118-50450531. For example, the nucleic acid set as a primer set may be used to amplify the DNA region derived from human chr7:50450311-50450411.

For example, the method of the present application may comprise providing a nucleic acid set as set forth in SEQ ID NO: 56 and 57, or a complementary nucleic acid set thereof, or a fragment thereabove. For example, the nucleic acid set as a primer set may be used to amplify the DNA region derived from human chr7:50467368-50469092. For example, the nucleic acid set as a primer set may be used to amplify the DNA region derived from human chr7:50467865-50467980.

In one aspect, the present application provides a method for identifying the presence of a liver tumor, assessing the development or development risk of the liver tumor, and/or assessing the progress of the liver tumor, which may comprise determining the presence and/or the content of modification status of a DNA region in which VAV3 gene is located or a fragment thereof in a sample to be tested. For example, the method of the present application may comprise identifying the presence or absence of a liver tumor based on the determination result of the presence and/or the content of modification status of a DNA region in which VAV3 gene is located or a fragment thereof in a sample to be tested. For example, the method of the present application may comprise assessing whether the development of a liver tumor is diagnosed based on the determination result of the presence and/or the content of modification status of a DNA region in which VAV3 gene is located or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which VAV3 gene is located or a fragment thereof in a sample to be tested. For example, the method of the present application may comprise assessing the progress of a liver tumor based on the determination result of the presence and/or the content of modification status of a DNA region in which VAV3 gene is located or a fragment thereof in a sample to be tested.

In another aspect, the present application provides a method for assessing the methylation status of a liver tumor-related DNA region, which may comprise determining the presence and/or the content of modification status of a DNA region in which VAV3 gene is located or a fragment thereof in a sample to be tested. For example, the methylation status of a liver tumor-related DNA region is assessed based on the determination of the presence and/or the content of modification status of a DNA region in which VAV3 gene is located or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr1:108113782-108507766. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific subregion of a DNA region in which VAV3 gene is located, or a complementary region thereof, or a fragment thereabove in a sample to be tested.

In another aspect, the present application provides a method for identifying the presence of a disease, assessing the development or risk of development of a disease, and/or assessing the progress of a disease, which may comprise determining the presence and/or the content of modification status of a DNA region selected from DNA regions derived from human chr1:108507215-108507766, or a complementary region thereof, or a fragment thereabove in a sample to be tested. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining the methylation status of a DNA region, which may comprise: determining the presence and/or the content of modification status of a DNA region selected from DNA regions derived from human chr1:108507215-108507766, or a complementary region thereof, or a fragment thereabove in a sample to be tested. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr1:108507215-108507766, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise: providing a nucleic acid capable of binding to a DNA region selected from SEQ ID NO: 58, or a complementary region thereof, or a transformed region thereabove, or a fragment thereabove.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr1:108507591-108507674 and derived from human chr1:108507624-108507725.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr1:108507591-108507674 and derived from human chr1:108507624-108507725.

For example, the method of the present application may comprise providing a nucleic acid capable of binding to a DNA region which may comprise a region selected from the following group, or a complementary region thereof, or a transformed region thereabove, or a fragment thereabove: derived from human chr1:108507591-108507674 and derived from human chr1:108507624-108507725.

For example, one or more of the above regions may serve as amplification regions and/or detection regions.

For example, the method of the present application may comprise: providing a nucleic acid selectable from the group consisting of SEQ ID NO: 59 and SEQ ID NO: 62, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 59, or a complementary nucleic acid thereof, or a fragment thereabove. For example, the nucleic acid as a probe may be used to detect the DNA region derived from human chr1:108507215-108507766. For example, the nucleic acid as a probe may be used to detect the DNA region derived from human chr1:108507591-108507674.

For example, the method of the present application may comprise: providing a nucleic acid as set forth in SEQ ID NO: 62, or a complementary nucleic acid thereof, or a fragment thereabove. For example, the nucleic acid as a probe may be used to detect the DNA region derived from human chr1:108507215-108507766. For example, the nucleic acid as a probe may be used to detect the DNA region derived from human chr1:108507624-108507725.

For example,the method of the present application may comprise providing a nucleic acid set selectable from the following group, or a complementary nucleic acid set thereof, or a fragment thereabove: SEQ ID NOs: 60 and 61, and SEQ ID NOs: 63 and 64. For example, the nucleic acid set may be used to amplify a target region. For example, the nucleic acid set may serve as a primer set.

For example, the method of the present application may comprise: providing a nucleic acid set as set forth in SEQ ID NOs: 60 and 61, or a complementary nucleic acid set thereof, or a fragment thereabove. For example, the nucleic acid set as a primer set may be used to amplify the DNA region derived from human chr1:108507215-108507766. For example, the nucleic acid set as a primer set may be used to amplify the DNA region derived from human chr1:108507591-108507674.

For example, the method of the present application may comprise: providing a nucleic acid set as set forth in SEQ ID NOs: 63 and 64, or a complementary nucleic acid set thereof, or a fragment thereabove. For example, the nucleic acid set as a primer set may be used to amplify the DNA region derived from human chr1:108507215-108507766. For example, the nucleic acid set as a primer set may be used to amplify the DNA region derived from human chr1:108507624-108507725.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region in which IRF4 gene is located or a fragment thereof in a sample to be tested. For example, the method of the present application may comprise identifying the presence or absence of a liver tumor based on the determination result of the presence and/or the content of modification status of a DNA region in which IRF4 gene is located, or a fragment thereof in a sample to be tested. For example, the method of the present application may comprise assessing whether the development of a liver tumor is diagnosed based on the determination result of the presence and/or the content of modification status of a DNA region in which IRF4 gene is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which IRF4 gene is located, or a fragment thereof in a sample to be tested. For example, the method of the present application may comprise assessing the progress of a liver tumor based on the determination result of the presence and/or the content of modification status of a DNA region in which IRF4 gene is located, or a fragment thereof in a sample to be tested.

In another aspect, the present application provides a method for assessing the methylation status of a liver tumor-related DNA region, which may comprise determining the presence and/or the content of modification status of a DNA region in which IRF4 gene is located, or a fragment thereof in a sample to be tested. For example, it comprises assessing the methylation status of a liver tumor-related DNA region based on the determination result of the presence and/or the content of modification status of a DNA region in which IRF4 gene is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr6:391739-411447. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or risk of development of a disease, and/or assessing the progress of a disease, which may comprise determining the presence and/or the content of modification status of a specific sub-region of a DNA region in which IRF4 gene is located, or a complementary region thereof, or a fragment thereabove in a sample to be tested.

In another aspect, the present application provides a method for identifying the presence of a disease, assessing the development or risk of development of a disease, and/or assessing the progress of a disease, which may comprise determining the presence and/or the content of modification status of a DNA region selected from the following group, or a complementary region thereof, or a fragment thereabove in a sample to be tested: derived from human chr6:391739-394056 and derived from human chr6:401233-401801. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr6:391739-394056 and derived from human chr6:401233-401801. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr6:391739-394056, or a complementary region thereof, or a fragment thereabove in a sample to be tested.

In another aspect, the present application provides a method for determining the methylation status of a DNA region, which may comprise: determining the presence and/or the content of modification status of a DNA region derived from human chr6:401233-401801, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region which may comprise a region selected from the following group, or a complementary region thereof, or a transformed region thereabove, or a fragment thereabove: SEQ ID NO: 21 and SEQ ID NO: 71.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr6:392282-392377, derived from human chr6:392036-392145, derived from human chr6:392405-392500 and derived from human chr6:401641-401752.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr6:392282-392377, derived from human chr6:392036-392145, derived from human chr6:392405-392500 and derived from human chr6:401641-401752.

For example, the method of the present application may comprise providing a nucleic acid capable of binding to a DNA region which may comprise a region selected from the following group, or a complementary region thereof, or a transformed region thereabove, or a fragment thereabove: derived from human chr6:392282-392377, derived from human chr6:392036-392145, derived from human chr6:392405-392500 and derived from human chr6:401641-401752.

For example, one or more of the above regions may serve as amplification regions and/or detection regions.

For example, the method of the present application may comprise: providing a nucleic acid selectable from the group consisting of SEQ ID NO: 22, SEQ ID NO: 65, SEQ ID NO: 68 and SEQ ID NO: 72, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 22, or a complementary nucleic acid thereof, or a fragment thereabove. For example, the nucleic acid as a probe may be used to detect the DNA region derived from human chr6:391739-394056. For example, the nucleic acid as a probe may be used to detect the DNA region derived from human chr6:392282-392377.

For example, the method of the present application may comprise: providing a nucleic acid as set forth in SEQ ID NO: 65, or a complementary nucleic acid thereof, or a fragment thereabove. For example, the nucleic acid as a probe may be used to detect the DNA region derived from human chr6:391739-394056. For example, the nucleic acid as a probe may be used to detect the DNA region derived from human chr6:392036-392145.

For example, the method of the present application may comprise: providing a nucleic acid as set forth in SEQ ID NO: 68, or a complementary nucleic acid thereof, or a fragment thereabove. For example, the nucleic acid as a probe may be used to detect the DNA region derived from human chr6:391739-394056. For example, the nucleic acid as a probe may be used to detect the DNA region derived from human chr6:392405-392500.

For example, the method of the present application may comprise: providing a nucleic acid as set forth in SEQ ID NO: 72, or a complementary nucleic acid thereof, or a fragment thereabove. For example, the nucleic acid as a probe may be used to detect the DNA region derived from human chr6:401233-401801. For example, the nucleic acid as a probe may be used to detect the DNA region derived from human chr6:401641-401752.

For example, the method of the present application may comprise providing a nucleic acid set selectable from the following group, or a complementary nucleic acid set thereof, or a fragment thereabove: SEQ ID NOs: 23 and 24, SEQ ID NOs: 66 and 67, SEQ ID NOs: 69 and 70 and SEQ ID NOs: 73 and 74. For example, the nucleic acid set may be used to amplify a target region. For example, the nucleic acid set may serve as a primer set.

For example, the method of the present application may comprise: providing a nucleic acid set as set forth in SEQ ID NOs: 23 and 24, or a complementary nucleic acid set thereof, or a fragment thereabove. For example, the nucleic acid set as a primer set may be used to amplify the DNA region derived from human chr6:391739-394056. For example, the nucleic acid set as a primer set may be used to amplify the DNA region derived from human chr6:392282-392377.

For example, the method of the present application may comprise: providing a nucleic acid set as set forth in SEQ ID NOs: 66 and 67, or a complementary nucleic acid set thereof, or a fragment thereabove. For example, the nucleic acid set as a primer set may be used to amplify the DNA region derived from human chr6:391739-394056. For example, the nucleic acid set as a primer set may be used to amplify the DNA region derived from human chr6:392036-392145.

For example, the method of the present application may comprise: providing a nucleic acid set as set forth in SEQ ID NOs: 69 and 70, or a complementary nucleic acid set thereof, or a fragment thereabove. For example, the nucleic acid set as a primer set may be used to amplify the DNA region derived from human chr6:391739-394056. For example, the nucleic acid set as a primer set may be used to amplify the DNA region derived from human chr6:392405-392500.

For example, the method of the present application may comprise: providing a nucleic acid set as set forth in SEQ ID NOs: 73 and 74, or a complementary nucleic acid set thereof, or a fragment thereabove. For example, the nucleic acid set as a primer set may be used to amplify the DNA region derived from human chr6:401233-401801. For example, the nucleic acid set as a primer set may be used to amplify the DNA region derived from human chr6:401641-401752.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region in which gene B4GALNT1 is located, or a fragment thereof in a sample to be tested. For example, the method of the present application may comprise identifying the presence or absence of a liver tumor based on the determination result of the presence and/or the content of modification status of a DNA region in which gene B4GALNT1 is located, or a fragment thereof in a sample to be tested. For example, the method of the present application may comprise assessing whether the development of a liver tumor is diagnosed based on the determination result of the presence and/or the content of modification status of a DNA region in which gene B4GALNT1 is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which gene B4GALNT1 is located, or a fragment thereof in a sample to be tested. For example, the method of the present application may comprise assessing the progress of a liver tumor based on the determination result of the presence and/or the content of modification status of a DNA region in which gene B4GALNT1 is located, or a fragment thereof in a sample to be tested.

In another aspect, the present application provides a method for assessing the methylation status of a liver tumor-related DNA region, which may comprise determining the presence and/or the content of modification status of a DNA region in which gene B4GALNT 1 is located, or a fragment thereof in a sample to be tested. For example, the methylation status of a liver tumor-related DNA region is assessed based on the determination result of the presence and/or the content of modification status of a DNA region in which gene B4GALNT1 is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr12: 58017193-58027138. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region in which gene B4GALNT 1 is located, or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from the following group, or a complementary region thereof, or a fragment thereabove in a sample to be tested: derived from human chr12:58020498-58022962 and derived from human chr12:58025539-58027138. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr12:58020498-58022962 and derived from human chr12:58025539-58027138. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr12:58020498-58022962, or a complementary region thereof, or a fragment thereabove in a sample to be tested.

In another aspect, the present application provides a method for determining the methylation status of a DNA region, which may comprise: determining the presence and/or the content of modification status of a DNA region derived from human chr12:58025539-58027138, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, tthe method of the present application may comprise providing a nucleic acid capable of binding to a DNA region which may comprise a region selected from the following group, or a complementary region thereof, or a transformed region thereabove, or a fragment thereabove: SEQ ID NO: 13 and SEQ ID NO: 78.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr12:58021586-58021670, derived from human chr12:58021907-58021987 and derived from human chr12:58026383-58026475.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr12:58021586-58021670, derived from human chr12:58021907-58021987, and derived from human chr12:58026383-58026475.

For example, the method of the present application may comprise providing a nucleic acid capable of binding to a DNA region which may comprise a region selected from the following group, or a complementary region thereof, or a transformed region thereabove, or a fragment thereabove: derived from human chr12:58021586-58021670, derived from human chr12:58021907-58021987 and derived from human chr12:58026383-58026475.

For example, one or more of the above regions may serve as amplification regions and/or detection regions.

For example, the method of the present application may comprise: providing a nucleic acid selectable from the group consisting of SEQ ID NO: 14, SEQ ID NO: 75 and SEQ ID NO: 79, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 14, or a complementary nucleic acid thereof, or a fragment thereabove. For example, the nucleic acid as a probe may be used to detect the DNA region derived from human chr12:58020498-58022962. For example, the nucleic acid as a probe may be used to detect the DNA region derived from human chr12:58021586-58021670.

For example, the method of the present application may comprise: providing a nucleic acid as set forth in SEQ ID NO: 75, or a complementary nucleic acid thereof, or a fragment thereabove. For example, the nucleic acid as a probe may be used to detect the DNA region derived from human chr12:58020498-58022962. For example, the nucleic acid as a probe may be used to detect the DNA region derived from human chr12:58021907-58021987.

For example, the method of the present application may comprise: providing a nucleic acid as set forth in SEQ ID NO: 79, or a complementary nucleic acid thereof, or a fragment thereabove. For example, the nucleic acid as a probe may be used to detect the DNA region derived from human chr12:58025539-58027138. For example, the nucleic acid as a probe may be used to detect the DNA region derived from human chr12:58026383-58026475.

For example, the method of the present application may comprise providing a nucleic acid set selectable from the following group, or a complementary nucleic acid set thereof, or a fragment thereabove: SEQ ID NOs: 15 and 16, SEQ ID NOs: 76 and 77, and SEQ ID NOs: 80 and 81. For example, the nucleic acid set may be used to amplify a target region. For example, the nucleic acid set may serve as a primer set.

For example, the method of the present application may comprise: providing a nucleic acid set as set forth in SEQ ID NOs: 15 and 16, or a complementary nucleic acid set thereof, or a fragment thereabove. For example, the nucleic acid set as a primer set may be used to amplify the DNA region derived from human chr12:58020498-58022962. For example, the nucleic acid set as a primer set may be used to amplify the DNA region derived from human chr12:58021586-58021670.

For example, the method of the present application may comprise: providing a nucleic acid set as set forth in SEQ ID NOs: 76 and 77, or a complementary nucleic acid set thereof, or a fragment thereabove. For example, the nucleic acid set as a primer set may be used to amplify the DNA region derived from human chr12:58020498-58022962. For example, the nucleic acid set as a primer set may be used to amplify the DNA region derived from human chr12:58021907-58021987.

For example, the method of the present application may comprise: providing a nucleic acid set as set forth in SEQ ID NOs: 80 and 81, or a complementary nucleic acid set thereof, or a fragment thereabove. For example, the nucleic acid setas a primer set may be used to amplify the DNA region derived from human chr12:58025539-58027138. For example, the nucleic acid set as a primer set may be used to amplify the DNA region derived from human chr12:58026383-58026475.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region in which gene GRASP is located, or a fragment thereof in a sample to be tested. For example, the method of the present application may comprise identifying the presence or absence of a liver tumor based on the determination result of the presence and/or the content of modification status of a DNA region in which gene GRASP is located, or a fragment thereof in a sample to be tested. For example, the method of the present application may comprise assessing whether the development of a liver tumor is diagnosed based on the determination result of the presence and/or the content of modification status of a DNA region in which gene GRASP is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which gene GRASP is located , or a fragment thereof in a sample to be tested. For example, the method of the present application may comprise assessing the progress of a liver tumor based on the determination result of the presence and/or the content of modification status of a DNA region in which gene GRASP is located, or a fragment thereof in a sample to be tested.

In another aspect, the present application provides a method for assessing the methylation status of a liver tumor-related DNA region, which may comprise determining the presence and/or the content of modification status of a DNA region in which gene GRASP is located , or a fragment thereof in a sample to be tested. For example, it comprises assessing the methylation status of a liver tumor-related DNA region based on the determination result of the presence and/or the content of modification status of a DNA region in which gene GRASP is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr12: 52400724-52409673. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region in which gene GRASP is located, or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from the following group, or a complementary region thereof, or a fragment thereabove in a sample to be tested: derived from human chr12:52400724-52401698 and derived from human chr12:52406880-52409127. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr12:52400724-52401698 and derived from human chr12:52406880-52409127. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr12:52400724-52401698, or a complementary region thereof, or a fragment thereabove in a sample to be tested.

In another aspect, the present application provides a method for determining the methylation status of a DNA region, which may comprise: determining the presence and/or the content of modification status of a DNA region derived from human chr12:52406880-52409127, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region which may comprise a region selected from the following group, or a complementary region thereof, or a transformed region thereabove, or a fragment thereabove: SEQ ID NO: 17 and SEQ ID NO: 85.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr12:52401083-52401169, derived from human chr12:52400965-52401055 and derived from human chr12:52408471-52408566.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr12:52401083-52401169, derived from human chr12:52400965-52401055 and derived from human chr12:52408471-52408566.

For example, the method of the present application may comprise providing a nucleic acid capable of binding to a DNA region which may comprise a region selected from the following group, or a complementary region thereof, or a transformed region thereabove, or a fragment thereabove: derived from human chr12:52401083-52401169, derived from human chr12:52400965-52401055 and derived from human chr12:52408471-52408566.

For example, one or more of the above regions may serve as amplification regions and/or detection regions.

For example, the method of the present application may comprise: providing a nucleic acid selectable from the group consisting of SEQ ID NO: 18, SEQ ID NO: 82 and SEQ ID NO: 86, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 18, or a complementary nucleic acid thereof, or a fragment thereabove. For example, the nucleic acid as a probe may be used to detect the DNA region derived from human chr12:52400724-52401698. For example, the nucleic acid as a probe may be used to detect the DNA region derived from human chr12:52401083-52401169.

For example, the method of the present application may comprise: providing a nucleic acid as set forth in SEQ ID NO: 82, or a complementary nucleic acid thereof, or a fragment thereabove. For example, the nucleic acid as a probe may be used to detect the DNA region derived from human chr12:52400724-52401698. For example, the nucleic acid as a probe may be used to detect the DNA region derived from human chr12:52400965-52401055.

For example, the method of the present application may comprise: providing a nucleic acid as set forth in SEQ ID NO: 86, or a complementary nucleic acid thereof, or a fragment thereabove. For example, the nucleic acid as a probe may be used to detect the DNA region derived from human chr12:52406880-52409127. For example, the nucleic acid as a probe may be used to detect the DNA region derived from human chr12:52408471-52408566.

For example, the method of the present application may comprise providing a nucleic acid set selectable from the following group, or a complementary nucleic acid set thereof, or a fragment thereabove: SEQ ID NOs: 19 and 20, SEQ ID NOs: 83 and 84, and SEQ ID NOs: 87 and 88. For example, the nucleic acid set may be used to amplify a target region. For example, the nucleic acid set may serve as a primer set.

For example, the method of the present application may comprise: providing a nucleic acid set as set forth in SEQ ID NOs: 19 and 20, or a complementary nucleic acid set thereof, or a fragment thereabove. For example, the nucleic acid as a primer set may be used to amplify the DNA region derived from human chr12:52400724-52401698. For example, the nucleic acid set as a primer set may be used to amplify the DNA region derived from human chr12:52401083-52401169.

For example, the method of the present application may comprise: providing a nucleic acid set as set forth in SEQ ID NOs: 83 and 84, or a complementary nucleic acid set thereof, or a fragment thereabove. For example, the nucleic acid as a primer set may be used to amplify the DNA region derived from human chr12:52400724-52401698. For example, the nucleic acid set as a primer set may be used to amplify the DNA region derived from human chr12:52400965-52401055.

For example, the method of the present application may comprise: providing a nucleic acid set as set forth in SEQ ID NOs: 87 and 88, or a complementary nucleic acid set thereof, or a fragment thereabove. For example, the nucleic acid set as a primer set may be used to amplify the DNA region derived from human chr12:52406880-52409127. For example, the nucleic acid set as a primer set may be used to amplify the DNA region derived from human chr12:52408471-52408566.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region in which gene BEST4 is located, or a fragment thereof in a sample to be tested. For example, the method of the present application may comprise identifying the presence or absence of a liver tumor based on the determination result of the presence and/or the content of modification status of a DNA region in which gene BEST4 is located, or a fragment thereof in a sample to be tested. For example, the method of the present application may comprise assessing whether the development of a liver tumor is diagnosed based on the determination result of the presence and/or the content of modification status of a DNA region in which gene BEST4 is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which gene BEST4 is located, or a fragment thereof in a sample to be tested. For example, the method of the present application may comprise assessing the progress of a liver tumor based on the determination result of the presence and/or the content of modification status of a DNA region in which gene BEST4 is located, or a fragment thereof in a sample to be tested.

In another aspect, the present application provides a method for assessing the methylation status of a liver tumor-related DNA region, which may comprise determining the presence and/or the content of modification status of a DNA region in which gene BEST4 is located, or a fragment thereof in a sample to be tested. For example, the methylation status of a liver tumor-related DNA region is assessed based on the determination result of the presence and/or the content of modification status of a DNA region in which gene BEST4 is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr1: 45249257-45253377. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region in which gene BEST4 is located, or a fragment thereof, or a fragment thereabove in a sample to be tested.

In another aspect, the present application provides a method for identifying the presence of a disease, assessing the development or development risk of the disease, and/or assessing the progress of the disease, which may comprise: which may comprise determining the presence and/or the content of modification status of a DNA region selected from the following group, or a complementary region thereof, or a fragment thereabove in a sample to be tested: derived from human chr1:45251728-45252477 and derived from human chr1:45249853-45250527. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr1:45251728-45252477 and derived from human chr1:45249853-45250527. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr1:45251728-45252477, or a complementary region thereof, or a fragment thereabove in a sample to be tested.

In another aspect, the present application provides a method for determining the methylation status of a DNA region, which may comprise: determining the presence and/or the content of modification status of a DNA region derived from human chr1:45249853-45250527, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region which may comprise a region selected from the following group, or a complementary region thereof, or a transformed region thereabove, or a fragment thereabove: SEQ ID NO: 9 and SEQ ID NO: 92.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr1:45252095-45252176, derived from human chr1:45252187-45252275 and derived from human chr1:45249894-45249981.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chrl :45252095-45252176, derived from human chr1:45252187-45252275 and derived from human chr1:45249894-45249981.

For example, the method of the present application may comprise providing a nucleic acid capable of binding to a DNA region which may comprise a region selected from the following group, or a complementary region thereof, or a transformed region thereabove, or a fragment thereabove: derived from human chr1:45252095-45252176, derived from human chr1:45252187-45252275 and derived from human chr1:45249894-45249981.

For example, one or more of the above regions may serve as amplification regions and/or detection regions.

For example, the method of the present application may comprise: providing a nucleic acid selectable from the group consisting of SEQ ID NO: 10, SEQ ID NO: 89 and SEQ ID NO: 93, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 10, or a complementary nucleic acid thereof, or a fragment thereabove. For example, the nucleic acid as a probe may be used to detect the DNA region derived from human chr1:45251728-45252477. For example, the nucleic acid as a probe may be used to detect the DNA region derived from human chr1:45252095-45252176.

For example, the method of the present application may comprise: providing a nucleic acid as set forth in SEQ ID NO: 89, or a complementary nucleic acid thereof, or a fragment thereabove. For example, the nucleic acid as a probe may be used to detect the DNA region derived from human chr1:45251728-45252477. For example, the nucleic acid as a probe may be used to detect the DNA region derived from human chr1:45252187-45252275.

For example, the method of the present application may comprise: providing a nucleic acid as set forth in SEQ ID NO: 93, or a complementary nucleic acid thereof, or a fragment thereabove. For example, the nucleic acid as a probe may be used to detect the DNA region derived from human chr1:45249853-45250527. For example, the nucleic acid as a probe may be used to detect the DNA region derived from human chr1:45249894-45249981.

For example, the method of the present application may comprise providing a nucleic acid set selectable from the following group, or a complementary nucleic acid set thereof, or a fragment thereabove: SEQ ID NOs: 11 and 12, SEQ ID NOs: 90 and 91, and SEQ ID NOs: 94 and 95. For example, the nucleic acid set may be used to amplify a target region. For example, the nucleic acid set may serve as a primer set.

For example, the method of the present application may comprise: providing a nucleic acid set as set forth in SEQ ID NOs: 11 and 12, or a complementary nucleic acid set thereof, or a fragment thereabove. For example, the nucleic acid set as a primer set may be used to amplify the DNA region derived from human chr1:45251728-45252477. For example, the nucleic acid set may be used as a primer set to amplify the DNA region derived from human chrl :45252095-45252176.

For example, the method of the present application may comprise: providing a nucleic acid set as set forth in SEQ ID NOs: 90 and 91, or a complementary nucleic acid set thereof, or a fragment thereabove. For example, the nucleic acid set as a primer set may be used to amplify the DNA region derived from human chr1:45251728-45252477. For example, the nucleic acid set may be used as a primer set to amplify the DNA region derived from human chrl :45252187-45252275.

For example, the method of the present application may comprise: providing a nucleic acid set as set forth in SEQ ID NOs: 94 and 95, or a complementary nucleic acid set thereof, or a fragment thereabove. For example, the nucleic acid set as a primer set may be used to amplify the DNA region derived from human chr1:45249853-45250527. For example, the nucleic acid set as a primer set may be used to amplify the DNA region derived from human chrl :45249894-45249981.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region with gene BEND4, or a fragment thereof in a sample to be tested. For example, the method of the present application may comprise identifying the presence or absence of a liver tumor based on the determination result of the presence and/or the content of modification status of a DNA region in which gene BEND4 is located, or a fragment thereof in a sample to be tested. For example, the method of the present application may comprise assessing whether the development of a liver tumor is diagnosed based on the determination result of the presence and/or the content of modification status of a DNA region in which gene BEND4 is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which gene BEND4 is located, or a fragment thereof in a sample to be tested. For example, the method of the present application may comprise assessing the progress of a liver tumor based on the determination result of the presence and/or the content of modification status of a DNA region in which gene BEND4 is located, or a fragment thereof in a sample to be tested.

In another aspect, the present application provides a method for assessing the methylation status of a liver tumor-related DNA region, which may comprise determining the presence and/or the content of modification status of a DNA region in which gene BEND4 is located, or a fragment thereof in a sample to be tested. For example, the methylation status of a liver tumor-related DNA region is assessed based on the determination result of the presence and/or the content of modification status of a DNA region in which gene BEND4 is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr4: 42112955-42154895. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region in which gene BEND4 is located, or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from DNA regions derived from human chr4:42152705-42154895, or a complementary region thereof, or a fragment thereabove in a sample to be tested. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining the methylation status of a DNA region, which may comprise: determining the presence and/or the content of modification status of a DNA region selected from DNA regions derived from human chr4:42152705-42154895, or a complementary region thereof, or a fragment thereabove in a sample to be tested. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr4:42152705-42154895, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise: providing a nucleic acid capable of binding to a DNA region selected from SEQ ID NO: 25, or a complementary region thereof, or a transformed region thereabove, or a fragment thereabove.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr4:42153816-42153921 and derived from human chr4:42153513-42153601.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr4:42153816-42153921 and derived from human chr4:42153513-42153601.

For example, tthe method of the present application may comprise providing a nucleic acid capable of binding to a DNA region which may comprise a region selected from the following group, or a complementary region thereof, or a transformed region thereabove, or a fragment thereabove: derived from human chr4:42153816-42153921 and derived from human chr4:42153513-42153601.

For example, one or more of the above regions may serve as amplification regions and/or detection regions.

For example, the method of the present application may comprise: providing a nucleic acid selectable from the group consisting of SEQ ID NO: 26 and SEQ ID NO: 96, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 26, or a complementary nucleic acid thereof, or a fragment thereabove. For example, the nucleic acid as a probe may be used to detect the DNA region derived from human chr4:42152705-42154895. For example, the nucleic acid as a probe may be used to detect the DNA region derived from human chr4:42153816-42153921.

For example, the method of the present application may comprise: providing a nucleic acid as set forth in SEQ ID NO: 96, or a complementary nucleic acid thereof, or a fragment thereabove. For example, the nucleic acid as a probe may be used to detect the DNA region derived from human chr4:42152705-42154895. For example, the nucleic acid as a probe may be used to detect the DNA region derived from human chr4:42153513-42153601.

For example, the method of the present application may comprise providing a nucleic acid set selectable from the following group, or a complementary nucleic acid set thereof, or a fragment thereabove: SEQ ID NOs: 27 and 28, and SEQ ID NOs: 97 and 98. For example, the nucleic acid set may be used to amplify a target region. For example, the nucleic acid set may serve as a primer set.

For example, the method of the present application may comprise: providing a nucleic acid set as set forth in SEQ ID NOs: 27 and 28, or a complementary nucleic acid set thereof, or a fragment thereabove. For example, the nucleic acid set as a primer set may be used to amplify the DNA region derived from human chr4:42152705-42154895. For example, the nucleic acid set as a primer set may be used to amplify the DNA region derived from human chr4:42153816-42153921.

For example, the method of the present application may comprise: providing a nucleic acid set as set forth in SEQ ID NOs: 97 and 98, or a complementary nucleic acid set thereof, or a fragment thereabove. For example, the nucleic acid set as a primer set may be used to amplify the DNA region derived from human chr4:42152705-42154895. For example, the nucleic acid set as a primer set may be used to amplify the DNA region derived from human chr4:42153513-42153601.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region derived from human chr1:145384249:145413094 or a fragment thereof in a sample to be tested. For example, the method of the present application may comprise identifying the presence or absence of a liver tumor based on the determination result of the presence and/or the content of modification status of a DNA region derived from human chr1:145384249:145413094 or a fragment thereof in a sample to be tested. For example, the method of the present application may comprise assessing whether the development of a liver tumor is diagnosed based on the determination result of the presence and/or the content of modification status of a DNA region derived from human chr1:145384249:145413094 or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region derived from human chr1:145384249:145413094 or a fragment thereabove in a sample to be tested. For example, the method of the present application may comprise assessing the progress of a liver tumor based on the determination result of the presence and/or the content of modification status of a DNA region derived from human chr1:145384249:145413094 or a fragment thereabove in a sample to be tested.

In another aspect, the present application provides a method for assessing the methylation status of a liver tumor-related DNA region, which may comprise determining the presence and/or the content of modification status of a DNA region derived from human chr1:145384249:145413094 or a fragment thereabove in a sample to be tested. For example, the methylation status of a liver tumor-related DNA region is assessed based on the determination result of the presence and/or the content of modification status of a DNA region derived from human chr1:145384249:145413094 or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg 19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region derived from human chr1:145384249:145413094, or a complementary region thereof, or a fragment thereabove in a sample to be tested.

In another aspect, tthe present application provides a method for identifying the presence of a disease, assessing the development or development risk of the disease, and/or assessing the progress of the disease, which may comprise determining the presence and/or the content of modification status of a DNA region selected from the following group, or a complementary region thereof, or a fragment thereabove in a sample to be tested: derived from human chr1:145389746-145401075, derived from human chr1:145384910-145385929 and derived from human chr1:145406714-145408013. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr1:145389746-145401075, derived from human chr1:145384910-145385929 and derived from human chr1:145406714-145408013. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr1:145389746-145401075, or a complementary region thereof, or a fragment thereabove in a sample to be tested.

In another aspect, the present application provides a method for determining the methylation status of a DNA region, which may comprise: determining the presence and/or the content of modification status of a DNA region derived from human chr1:145384910-145385929, or a complementary region thereof, or a fragment thereabove in a sample to be tested.

In another aspect, the present application provides a method for determining the methylation status of a DNA region, which may comprise: determining the presence and/or the content of modification status of a DNA region derived from human chr1:145406714-145408013, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise providing a nucleic acid capable of binding to a DNA region which may comprise a region selected from the following group, or a complementary region thereof, or a transformed region thereabove, or a fragment thereabove: SEQ ID NO: 99, SEQ ID NO: 106 and SEQ ID NO: 110.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr1:145399249:145399476, derived from human chr1:145396880-145396983, derived from human chr1:145385298-145385376 and derived from human chr1:145407431-145407518.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr1:145399249:145399476, derived from human chr1:145396880-145396983, derived from human chr1:145385298-145385376 and derived from human chr1:145407431-145407518.

For example, the method of the present application may comprise providing a nucleic acid capable of binding to a DNA region which may comprise a region selected from the following group, or a complementary region thereof, or a transformed region thereabove, or a fragment thereabove: derived from human chr1:145399249:145399476, derived from human chr1:145396880-145396983, derived from human chr1:145385298-145385376 and derived from human chr1:145407431-145407518.

For example, one or more of the above regions may serve as amplification regions and/or detection regions.

For example, the method of the present application may comprise: providing a nucleic acid selectable from the group consisting of SEQ ID NO: 100, SEQ ID NO: 103, SEQ ID NO: 107 and SEQ ID NO: 111, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 100, or a complementary nucleic acid thereof, or a fragment thereabove. For example, the nucleic acid as a probe may be used to detect the DNA region derived from human chr1:145389746-145401075. For example, the nucleic acid as a probe may be used to detect the DNA region derived from human chr1:145399249:145399476.

For example, the method of the present application may comprise: providing a nucleic acid as set forth in SEQ ID NO: 103, or a complementary nucleic acid thereof, or a fragment thereabove. For example, the nucleic acid as a probe may be used to detect the DNA region derived from human chr1:145389746-145401075. For example, the nucleic acid as a probe may be used to detect the DNA region derived from human chr1:145396880-145396983.

For example, the method of the present application may comprise: providing a nucleic acid as set forth in SEQ ID NO: 107, or a complementary nucleic acid thereof, or a fragment thereabove. For example, the nucleic acid as a probe may be used to detect the DNA region derived from human chr1:145384910-145385929. For example, the nucleic acid as a probe may be used to detect the DNA region derived from human chr1:145385298-145385376.

For example, the method of the present application may comprise: providing a nucleic acid as set forth in SEQ ID NO: 111, or a complementary nucleic acid thereof, or a fragment thereabove. For example, the nucleic acid as a probe may be used to detect the DNA region derived from human chr1:145406714-145408013. For example, the nucleic acid as a probe may be used to detect the DNA region derived from human chr1:145407431-145407518.

For example, the method of the present application may comprise providing a nucleic acid set selectable from the following group, or a complementary nucleic acid set thereof, or a fragment thereabove: SEQ ID NOs: 101 and 102, SEQ ID NOs: 104 and 105, SEQ ID NOs: 108 and 109, and SEQ ID NOs: 112 and 113. For example, the nucleic acid set may be used to amplify a target region. For example, the nucleic acid set may serve as a primer set.

For example, the method of the present application may comprise: providing a nucleic acid set as set forth in SEQ ID NOs: 101 and 102, or a complementary nucleic acid set thereof, or a fragment thereabove. For example, the nucleic acid set as a primer set may be used to amplify the DNA region derived from human chr1:145389746-145401075. For example, the nucleic acid set as a primer set may be used to amplify the DNA region derived from human chr1:145399249:145399476.

For example, the method of the present application may comprise: providing a nucleic acid set as set forth in SEQ ID NOs: 104 and 105, or a complementary nucleic acid set thereof, or a fragment thereabove. For example, the nucleic acid set as a primer set may be used to amplify the DNA region derived from human chr1:145389746-145401075. For example, the nucleic acid set as a primer set may be used to amplify the DNA region derived from human chr1:145396880-145396983.

For example, the method of the present application may comprise: providing a nucleic acid set as set forth in SEQ ID NOs: 108 and 109, or a complementary nucleic acid set thereof, or a fragment thereabove. For example, the nucleic acid set as a primer set may be used to amplify the DNA region derived from human chr1:145384910-145385929. For example, the nucleic acid set as a primer set may be used to amplify the DNA region derived from human chr1:145385298-145385376.

For example, the method of the present application may comprise: providing a nucleic acid set as set forth in SEQ ID NOs: 112 and 113, or a complementary nucleic acid set thereof, or a fragment thereabove. For example, the nucleic acid set as a primer set may be used to amplify the DNA region derived from human chr1:145406714-145408013. For example, the nucleic acid set as a primer set may be used to amplify the DNA region derived from human chr1:145407431-145407518.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region derived from human chr7:26415938:26416740 or a fragment thereof in a sample to be tested. For example, the method of the present application may comprise identifying the presence or absence of a liver tumor based on the determination result of the presence and/or the content of modification status of a DNA region derived from human chr7:26415938:26416740 or a fragment thereof in a sample to be tested. For example, the method of the present application may comprise assessing whether the development of a liver tumor is diagnosed based on the determination result of the presence and/or the content of modification status of a DNA region derived from human chr7:26415938:26416740 or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region derived from human chr7:26415938:26416740 or a fragment thereof in a sample to be tested. For example, the method of the present application may comprise assessing the progress of a liver tumor based on the determination result of the presence and/or the content of modification status of a DNA region derived from human chr7:26415938:26416740 or a fragment thereof in a sample to be tested.

In another aspect, the present application provides a method for assessing the methylation status of a liver tumor-related DNA region, which may comprise determining the presence and/or the content of modification status of a DNA region derived from human chr7:26415938:26416740 or a fragment thereof in a sample to be tested. For example, the methylation status of a liver tumor-related DNA region is assessed based on the determination result of the presence and/or the content of modification status of a DNA region derived from human chr7:26415938:26416740 or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg 19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region derived from human chr7:26415938:26416740, or a complementary region thereof, or a fragment thereabove in a sample to be tested.

For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr7:26416257-26416363 and derived from human chr7:26416026-26416126.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr7:26416257-26416363 and derived from human chr7:26416026-26416126.

For example, the method of the present application may comprise providing a nucleic acid capable of binding to a DNA region which may comprise a region selected from the following group, or a complementary region thereof, or a transformed region thereabove, or a fragment thereabove: derived from human chr7:26416257-26416363 and derived from human chr7:26416026-26416126.

For example, one or more of the above regions may serve as amplification regions and/or detection regions.

For example, the method of the present application may comprise: providing a nucleic acid selectable from the group consisting of SEQ ID NO: 114 and SEQ ID NO: 117, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 114, or a complementary nucleic acid thereof, or a fragment thereabove. For example, the nucleic acid as a probe may be used to detect the DNA region derived from human chr7:26416257-26416363.

For example, the method of the present application may comprise: providing a nucleic acid as set forth in SEQ ID NO: 117, or a complementary nucleic acid thereof, or a fragment thereabove. For example, the nucleic acid as a probe may be used to detect the DNA region derived from human chr7:26416026-26416126.

For example, the method of the present application may comprise providing a nucleic acid set selectable from the following group, or a complementary nucleic acid set thereof, or a fragment thereabove: SEQ ID NOs: 115 and 116, and SEQ ID NOs: 118 and 119. For example, the nucleic acid set may be used to amplify a target region. For example, the nucleic acid set may serve as a primer set.

For example, the method of the present application may comprise: providing a nucleic acid set as set forth in SEQ ID NOs: 115 and 116, or a complementary nucleic acid set thereof, or a fragment thereabove. For example, the nucleic acid set as a primer set may be used to amplify the DNA region derived from human chr7:26416257-26416363.

For example, the method of the present application may comprise: providing a nucleic acid set as set forth in SEQ ID NOs: 118 and 119, or a complementary nucleic acid set thereof, or a fragment thereabove. For example, the nucleic acid set as a primer set may be used to amplify the DNA region derived from human chr7:26416026-26416126.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region in which gene GPAM is located, or a fragment thereof in a sample to be tested. For example, the method of the present application may comprise identifying the presence or absence of a liver tumor based on the determination result of the presence and/or the content of modification status of a DNA region in which gene GPAM is located, or a fragment thereof in a sample to be tested. For example, the method of the present application may comprise assessing whether the development of a liver tumor is diagnosed based on the determination result of the presence and/or the content of modification status of a DNA region in which gene GPAM is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which gene GPAM is located, or a fragment thereof in a sample to be tested. For example, the method of the present application may comprise assessing the progress of a liver tumor based on the determination result of the presence and/or the content of modification status of a DNA region in which gene GPAM is located, or a fragment thereof in a sample to be tested.

In another aspect, the present application provides a method for assessing the methylation status of a liver tumor-related DNA region, which may comprise determining the presence and/or the content of modification status of a DNA region in which gene GPAM is located, or a fragment thereof in a sample to be tested. For example, the methylation status of a liver tumor-related DNA region is assessed based on the determination result of the presence and/or the content of modification status of a DNA region in which gene GPAM is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr10: 113909624-113975135. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region in which gene GPAM is located, or a fragment thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from DNA regions derived from human chr10:113942657-113943906, or a complementary region thereof, or a fragment thereabove in a sample to be tested. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining the methylation status of a DNA region, which may comprise: determining the presence and/or the content of modification status of a DNA region selected from DNA regions derived from human chr10:113942657-113943906, or a complementary region thereof, or a fragment thereabove in a sample to be tested. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr10:113942657-113943906, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise: providing a nucleic acid capable of binding to a DNA region selected from SEQ ID NO: 120, or a complementary region thereof, or a transformed region thereabove, or a fragment thereabove.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr10:113943540:113943739 and derived from human chr10:113943511-113943582.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr10:113943540:113943739 and derived from human chr10:113943511-113943582.

For example, the method of the present application may comprise providing a nucleic acid capable of binding to a DNA region which may comprise a region selected from the following group, or a complementary region thereof, or a transformed region thereabove, or a fragment thereabove: derived from human chr10:113943540:113943739 and derived from human chr10:113943511-113943582.

For example, one or more of the above regions may serve as amplification regions and/or detection regions.

For example, the method of the present application may comprise: providing a nucleic acid selectable from the group consisting of SEQ ID NO: 121 and SEQ ID NO: 124, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 121, or a complementary nucleic acid thereof, or a fragment thereabove. For example, the nucleic acid as a probe may be used to detect the DNA region derived from human chr10:113942657-113943906. For example, the nucleic acid as a probe may be used to detect the DNA region derived from human chr10:113943540:113943739.

For example, the method of the present application may comprise: providing a nucleic acid as set forth in SEQ ID NO: 124, or a complementary nucleic acid thereof, or a fragment thereabove. For example, the nucleic acid as a probe may be used to detect the DNA region derived from human chr10:113942657-113943906. For example, the nucleic acid as a probe may be used to detect the DNA region derived from human chr10:113943511-113943582.

For example, the method of the present application may comprise providing a nucleic acid set selectable from the following group, or a complementary nucleic acid set thereof, or a fragment thereabove: SEQ ID NOs: 122 and 123, and SEQ ID NOs: 125 and 126. For example, the nucleic acid set may be used to amplify a target region. For example, the nucleic acid set may serve as a primer set.

For example, the method of the present application may comprise: providing a nucleic acid set as set forth in SEQ ID NOs: 122 and 123, or a complementary nucleic acid set thereof, or a fragment thereabove. For example, the nucleic acid as a primer set may be used to amplify the DNA region derived from human chr10:113942657-113943906. For example, the nucleic acid set as a primer set may be used to amplify the DNA region derived from human chr10:113943540:113943739.

For example, the method of the present application may comprise: providing a nucleic acid set as set forth in SEQ ID NOs: 125 and 126, or a complementary nucleic acid set thereof, or a fragment thereabove. For example, the nucleic acid as a primer set may be used to amplify the DNA region derived from human chr10:113942657-113943906. For example, the nucleic acid set as a primer set may be used to amplify the DNA region derived from human chr10:113943511-113943582.

In one aspect, the present application provides a method for identifying the presence of a liver tumor, assessing the development or risk of development of a liver tumor, and/or assessing the progress of a liver tumor, which may comprise determining the presence and/or the content of modification status of a DNA region in which gene VASH2 is located, or a fragment thereof in a sample to be tested. For example, the method of the present application may comprise identifying the presence or absence of a liver tumor based on the determination result of the presence and/or the content of modification status of a DNA region in which gene VASH2 is located, or a fragment thereof in a sample to be tested. For example, the method of the present application may comprise assessing whether the development of a liver tumor is diagnosed based on the determination result of the presence and/or the content of modification status of a DNA region in which gene VASH2 is located, or a fragment thereof in a sample to be tested. For example, the method of the present application may comprise assessing whether there is a risk of being diagnosed with the development of a liver tumor and/or the level of risk based on the determination result of the presence and/or the content of modification status of a DNA region in which gene VASH2 is located, or a fragment thereof in a sample to be tested. For example, the method of the present application may comprise assessing the progress of a liver tumor based on the determination result of the presence and/or the content of modification status of a DNA region in which gene VASH2 is located, or a fragment thereof in a sample to be tested.

In another aspect, the present application provides a method for assessing the methylation status of a liver tumor-related DNA region, which may comprise determining the presence and/or the content of modification status of a DNA region in which gene VASH2 is located, or a fragment thereof in a sample to be tested. For example, the methylation status of a liver tumor-related DNA region is assessed based on the determination result of the presence and/or the content of modification status of a DNA region in which gene VASH2 is located, or a fragment thereof in a sample to be tested. For example, the methylation status of a liver tumor-related DNA region may refer to the identified presence or increased amount of the methylation of the DNA region, relative to a reference level, which may be correlated with the occurrence of a liver tumor.

For example, the DNA region of the present application may be derived from human chr1: 213123862-213165379. For example, the genes of the present application can be described by their names and their chromosomal coordinates. For example, chromosomal coordinates can be consistent with the Hg19 version of the human genome database (or "Hg19 coordinates"), published in February 2009. For example, a DNA region of the present application may be derived from a region defined by Hg19 coordinates.

In another aspect, the present application provides a method for identifying the presence of a disease, assessing the development or development risk of the disease, and/or assessing the progress of the disease, which may comprise determining the presence and/or the content of modification status of a specific sub-region of a DNA region in which gene VASH2 is located, or a complementary region thereof, or a fragment thereabove in a sample to be tested.

In another aspect, the present application provides a method for identifying the presence of a disease, assessing the development or development risk of the disease, and/or assessing the progress of the disease, which may comprise determining the presence and/or the content of modification status of a DNA region selected from DNA regions derived from human chr1:213123862-213125211, or a complementary region thereof, or a fragment thereabove in a sample to be tested. For example, the method of the present application may comprise identifying the presence or absence of a disease based on the determination result of the presence and/or the content of modification status of the DNA region, or a complementary region thereof or a fragment thereabove in a sample to be tested. For example, the method of the present application may comprise assessing whether a disease development is diagnosed based on the determination result of the presence and/or the content of modification status of the DNA region, or a complementary region thereof or a fragment thereabove in a sample to be tested. For example, the method of the present application may comprise assessing whether a disease risk is diagnosed and/or the level of risk based on the determination result of the presence and/or the content of modification status of the DNA region, or a complementary region thereof, or a fragment thereabove in a sample to be tested. For example, the method of the present application may comprise assessing the progress of a disease based on the determination result of the presence and/or the content of modification status of the DNA region, or a complementary region thereof or a fragment thereabove in a sample to be tested.

In another aspect, the present application provides a method for determining the methylation status of a DNA region, which may comprise: determining the presence and/or the content of modification status of a DNA region selected from DNA regions derived from human chr1:213123862-213125211, or a complementary region thereof, or a fragment thereabove in a sample to be tested. For example, the identified presence or increased amount of the methylation of the DNA region, relative to a reference level, may be correlated with the occurrence of a disease. For example, the DNA region in the present application may refer to a specific segment of genomic DNA. For example, the DNA region of the present application may be designated by a gene name or a set of chromosomal coordinates. For example, a gene can have its sequence and chromosomal location obtained by reference to its name, or have its sequence and chromosomal location determined by reference to its chromosomal coordinates. The present application uses the methylation status of these specific DNA regions as a series of analytical indicators, which can provide significant improvement in sensitivity and/or specificity, and can simplify the screening process. For example, "sensitivity" may refer to the proportion of positive results correctly identified, i.e., the percentage of individuals correctly identified as having the disease under discussion, and "specificity" may refer to the proportion of negative results correctly identified, i.e., the percentage of individuals correctly identified as not having the disease under discussion.

In another aspect, the present application provides a method for determining the methylation status of a DNA region, which may comprise: determining the presence and/or the content of modification status of a DNA region derived from human chr1:213123862-213125211, or a complementary region thereof, or a fragment thereabove in a sample to be tested.

The DNA region of the present application may comprise all forms of these molecules and fragments or variants thereof. For example, a variant may comprise at least 80%, at least 85%, at least 90%, 95%, 98%, or 99% sequence identity relative to the DNA region described in the present application, and a variant may comprise one or more deletions, additions, substitutions, inverted sequences, etc. For example, the modification status of the variants described in the present application can achieve the same assessment results. The DNA region of the present application may comprise any other mutation, polymorphic variation or allelic variation in all forms.

For example, the method of the present application may comprise: providing a nucleic acid capable of binding to a DNA region selected from SEQ ID NO: 127, or a complementary region thereof, or a transformed region thereabove, or a fragment thereabove.

In another aspect, the present application provides a method for identifying the presence of a disease, assessing the development or development risk of the disease, and/or assessing the progress of the disease, which may comprise determining the presence and/or the content of modification status of a DNA region selectable from the following group, or a complementary region thereof, or a fragment thereabove in a sample to be tested: derived from human chr1:213124569-213124670 and derived from human chr1:213124036-213124162.

In another aspect, the present application provides a method for determining methylation status of a DNA region, which may comprise determining the presence and/or the content of modification status of a DNA region selectable from the following group, or a complementary region thereof, or a fragment thereabove in a sample to be tested: derived from human chr1:213124569-213124670 and derived from human chr1:213124036-213124162.

For example, the method of the present application may comprise providing a nucleic acid capable of binding to a DNA region which may comprise a region selected from the following group, or a complementary region thereof, or a transformed region thereabove, or a fragment thereabove: derived from human chr1:213124569-213124670 and derived from human chr1:213124036-213124162.

For example, one or more of the above regions may serve as amplification regions and/or detection regions.

For example, the method of the present application may comprise: providing a nucleic acid selectable from the group consisting of SEQ ID NO: 128 and SEQ ID NO: 131, or a complementary nucleic acid thereof, or a fragment thereabove. For example, the nucleic acid may be used to detect a target region. For example, the nucleic acid may be used as a probe.

For example, the method of the present application may comprise: providing a nucleic acid as set forth in SEQ ID NO: 128, or a complementary nucleic acid thereof, or a fragment thereabove. For example, the nucleic acid as a probe may be used to detect the DNA region derived from human chr1:213123862-213125211. For example, the nucleic acid as a probe may be used to detect the DNA region derived from human chr1:213124569-213124670.

For example, the method of the present application may comprise: providing a nucleic acid as set forth in SEQ ID NO: 131, or a complementary nucleic acid thereof, or a fragment thereabove. For example, the nucleic acid as a probe may be used to detect the DNA region derived from human chr1:213123862-213125211. For example, the nucleic acid as a probe may be used to detect the DNA region derived from human chr1:213124036-213124162.

For example, the method of the present application may comprise providing a nucleic acid set selectable from the following group, or a complementary nucleic acid set thereof, or a fragment thereabove: SEQ ID NOs: 129 and 130, and SEQ ID NOs: 132 and 133. For example, the nucleic acid set may be used to amplify a target region. For example, the nucleic acid set may serve as a primer set.

For example, the method of the present application may comprise: providing a nucleic acid set as set forth in SEQ ID NOs: 129 and 130, or a complementary nucleic acid set thereof, or a fragment thereabove. For example, the nucleic acid as a primer set may be used to amplify the DNA region derived from human chr1:213123862-213125211. For example, the nucleic acid set as a primer set may be used to amplify the DNA region derived from human chr1:213124569-213124670.

For example, the method of the present application may comprise: providing a nucleic acid set as set forth in SEQ ID NOs: 132 and 133, or a complementary nucleic acid set thereof, or a fragment thereabove. For example, the nucleic acid as a primer set may be used to amplify the DNA region derived from human chr1:213123862-213125211. For example, the nucleic acid set as a primer set may be used to amplify the DNA region derived from human chr1:213124036-213124162.

For example, the disease may comprise tumors. For example, the disease may comprise solid tumors. For example, the disease may comprise any tumor such as liver tumors.

For example, a "primer" may be a natural or synthetic oligonucleotide capable of serving as a starting point for nucleic acid synthesis after forming a duplex with a polynucleotide template and extending along the template from its 3' end to form an extended duplex. The sequence of the nucleotide added during extension is determined by the sequence of the template polynucleotide. Primers can generally be extended by a polymerase such as a nucleic acid polymerase.

For example, "complementary" and "substantially complementary" in the present application may include hybridization or base pairing or formation of a duplex between nucleotides or nucleic acids, for example between the two strands of a double-stranded DNA molecule, or between oligonucleotide primers and primer binding sites on a single-stranded nucleic acid. Complementary nucleotides may typically be A and T (or A and U) or C and G. For two single-stranded RNA or DNA molecules, when the nucleotides of one strand are paired with at least about 80% (usually at least about 90% to about 95%, or even about 98% to about 100%) of those of the other strand when they are optimally aligned and compared and have appropriate nucleotide insertions or deletions, they can be considered to be substantially complementary. In one aspect, two complementary nucleotide sequences are capable of hybridizing with less than 25% mismatch, more preferably less than 15% mismatch, and less than 5% mismatch or without mismatch between reverse nucleotides. For example, two molecules can hybridize under highly stringent conditions.

For example, the modification status in the present application may refer to the presence, absence and/or content of modification status at a specific nucleotide or multiple nucleotides within a DNA region. For example, the modification status in the present application may refer to the modification status of each base or each specific base (e.g., cytosine) in a specific DNA sequence. For example, the modification status in the present application may refer to the modification status of base pair combinations and/or base combinations in a specific DNA sequence. For example, the modification status in the present application may refer to information about the density of region modifications in a specific DNA sequence (including the DNA region where the gene is located or specific region fragments thereof), but may not provide precise location information on where modifications occur in the sequence.

For example, the modification status of the present application may be a methylation status or a state similar to methylation. For example, a state of being methylated or being highly methylated can be associated with transcriptional silencing of a specific region. For example, a state of being methylated or being highly methylated may be associated with being able to be transformed by a methylation-specific conversion reagent (such as a deamination reagent and/or a methylation-sensitive restriction enzyme). For example, transformation may refer to being converted into other substances and/or being cleaved or digested.

For example, "methylation state" or "methylation status" may refer to the presence or absence of 5-methylcytosine ("5-mC" or "5-mCyt") at one or more CpG dinucleotides within a DNA sequence. The methylation status at one or more specific CpG methylation sites (each having two CpG dinucleotide sequences) within the DNA sequence include "unmethylation", "complete methylation", and "semi-methylation". The term "hemimethylation" may refer to the methylation status of double-stranded DNA in which only one strand of the double-stranded DNA is methylated. The term "hypermethylation" may refer to the average methylation status corresponding to an increase in 5-mCyt present at one or more CpG dinucleotides in the DNA sequence of the test DNA sample relative to the content of 5-mCyt found at the corresponding CpG dinucleotides in the normal control DNA sample. The term "hypomethylation" may refer to the average methylation status corresponding to a decrease in 5-mCyt present at one or more CpG dinucleotides in the DNA sequence of the test DNA sample relative to the content of 5-mCyt found at the corresponding CpG dinucleotides in the normal control DNA sample.

For example, the method may further comprise obtaining the nucleic acid in the sample to be tested. For example, the nucleic acid may include a cell-free nucleic acid. For example, the sample to be tested may comprise tissues, cells and/or body fluids. For example, the sample to be tested may comprise plasma. For example, the detection method of the present application can be performed on any suitable biological sample. For example, the sample to be tested can be any sample of biological materials, such as it may be derived from an animal, but is not limited to cellular materials, biological fluids (such as blood), discharge, tissue biopsy specimens, surgical specimens, or fluids that have been introduced into the body of an animal and subsequently removed. For example, the sample to be tested in the present application may include a sample that has been processed in any form after the sample is isolated.

For example, the method may further comprise transforming the DNA region or a fragment thereof. For example, through the transformation step of the present application, the bases with the modification and the bases without the modification can form different substances after transformation. For example, the base with the modification status is substantially unchanged after transformation, and the base without the modification status is changed to other bases (for example, the other bases may include uracil) different from the base after transformation or is cleaved after transformation. For example, the base may include cytosine. For example, the modification may include methylation modification. For example, the conversion may comprise conversion by a deamination reagent and/or a methylation-sensitive restriction enzyme. For example, the deamination reagent may include bisulfite or analogues thereof. For example, it is sodium bisulfite or potassium bisulfite.

For example, the method may optionally further comprise amplifying the DNA region or a fragment thereof in the sample to be tested before determining the presence and/or the content of modification status of the DNA region or a fragment thereof. For example, the amplification may include PCR amplification. For example, the amplification in the present application may include any known amplification system. For example, the amplification step in the present application may be optional. For example, "amplification" may refer to the process of producing multiple copies of a desired sequence. "Multiple copies" may refer to at least two copies. "Copy" may not imply perfect sequence complementarity or identity to the template sequence. For example, copies may include nucleotide analogs such as deoxyinosine, intentional sequence changes (such as those introduced by primers containing sequences that are hybridizable but not complementary to the template), and/or may occur during amplification Sequence error.

For example, the method for determining the presence and/or the content of modification status may comprise determining the presence and/or the content of a substance formed by a base with the modification status after the transformation. For example, the method for determining the presence and/or the content of modification status may comprise determining the presence and/or the content of a DNA region with the modification status or a fragment thereof. For example, the presence and/or the content of a DNA region with the modification status or a fragment thereof can be directly detected. For example, it can be detected in the following manner: a DNA region with the modification status or a fragment thereof may have different characteristics from a DNA region without the modification status or a fragment thereof during a reaction (e.g., an amplification reaction). For example, in a fluorescence PCR method, a DNA region with the modification status or a fragment thereof can be specifically amplified and emit fluorescence; and a DNA region without the modification status or a fragment thereof can be substantially not amplified, and substantially do not emit fluorescence. For example, alternative methods of determining the presence and/or the content of species formed upon conversion of bases with the modification status may be included within the scope of the present application.

For example, the presence and/or the content of the DNA region with the modification status or fragment thereof is determined by the fluorescence Ct value detected by the fluorescence PCR method. For example, the presence of a liver tumor, or the development or risk of development of a liver tumor is determined by the presence of modification status of the DNA region or a fragment thereof and/or a higher content of modification status of the DNA region or a fragment thereof relative to the reference level. For example, when the fluorescence Ct value of the sample to be tested is lower than the reference fluorescence Ct value, the presence of modification status of the DNA region or a fragment thereof can be determined and/or it can be determined that the content of modification status of the DNA region or a fragment thereof is higher than the content of modification status in the reference sample. For example, the reference fluorescence Ct value can be determined by detecting the reference sample. For example, when the fluorescence Ct value of the sample to be tested is higher than or substantially equivalent to the reference fluorescence Ct value, the presence of modification status of the DNA region or a fragment thereof may not be ruled out; when the fluorescence Ct value of the sample to be tested is higher than or substantially equivalent to the reference fluorescence Ct value, it can be confirmed that the content of modification status of the DNA region or a fragment thereof is lower than or substantially equal to the content of modification status in the reference sample.

For example, the present application can represent the presence and/or the content of modification status of a specific DNA region or a fragment thereof through a cycle threshold (i.e., Ct value), which, for example, includes the methylation level of a sample to be tested and a reference level. For example, the Ct value may refer to the number of cycles at which fluorescence of the PCR product can be detected above the background signal. For example, there can be a negative correlation between the Ct value and the starting content of the target marker in the sample, that is, the lower the Ct value, the greater the content of modification status of the DNA region or a fragment thereof in the sample to be tested.

For example, when the Ct value of the sample to be tested is the same as or lower than its corresponding reference Ct value, it may be confirmed as the presence of a particular disease, diagnosed as the development or risk of development of a particular disease, or assessed as certain progress of a particular disease. For example, when the Ct value of the sample to be tested is lower than its corresponding reference Ct value by at least 1 cycle, at least 2 cycles, at least 5 cycles, at least 10 cycles, at least 20 cycles, or at least 50 cycles, it may be confirmed as the presence of a particular disease, diagnosed as the development or risk of development of a particular disease, or assessed as certain progress of a particular disease.

For example, when the Ct value of a cell sample, a tissue sample or a sample derived from a subject is the same as or higher than its corresponding reference Ct value, it may be confirmed as the absence of a particular disease, not diagnosed as the development or risk of development of a particular disease, or not assessed as certain progress of a particular disease. For example, when the Ct value of a cell sample, a tissue sample or a sample derived from a subject is higher than its corresponding reference Ct value by at least 1 cycle, at least 2 cycles, at least 5 cycles, at least 10 cycles, at least 20 cycles, or at least 50 cycles, it may be confirmed as the absence of a particular disease, not diagnosed as the development or risk of development of a particular disease, or not assessed as certain progress of a particular disease. For example, when the Ct value of a cell sample, a tissue sample or a sample derived from a subject is the same as its corresponding reference Ct value, it may be confirmed as the presence or absence of a particular disease, diagnosed as developing or not developing a particular disease, having or not having risk of development of a particular disease, or assessed as having or not having certain progress of a particular disease, and at the same time, suggestions for further testing can be given.

For example, the reference level or control level in the present application may refer to a normal level or a healthy level. For example, the normal level may be the modification status level of a DNA region of a sample derived from cells, tissues or individuals free of the disease. For example, when used for the assessment of a tumor, the normal level may be the modification status level of a DNA region of a sample derived from cells, tissues or individuals free of the tumor. For example, when used for the assessment of a liver tumor, the normal level may be the modification status level of a DNA region of a sample derived from cells, tissues or individuals free of the liver tumor.

For example, the reference level in the present application may refer to a threshold level at which the presence or absence of a particular disease is confirmed in a subject or sample. For example, the reference level in the present application may refer to a threshold level at which a subject is diagnosed as developing or at risk of developing a particular disease. For example, the reference level in the present application may refer to a threshold level at which a subject is assessed as having certain progress of a particular disease. For example, when the modification status of a DNA region in a cell sample, a tissue sample or a sample derived from a subject is higher than or substantially equal to the corresponding reference level (for example, the reference level here may refer to the modification status of a DNA region of a patient without a particular disease), it may be confirmed as the presence of a particular disease, diagnosed as developing or at risk of developing a particular disease, or assessed as certain progress of a particular disease. For example, A and B are "substantially equal" in the present application may mean that the difference between A and B is 1% or less, 0.5% or less, 0.1% or less, 0.01% or less, 0.001% or less, or 0.0001% or less. For example, when the modification status of a DNA region in a cell sample, a tissue sample, or a sample derived from a subject is higher than the corresponding reference level by at least 1%, at least 5%, at least 10%, at least 20%, at least 50%, at least 1 times, at least 2 times, at least 5 times, at least 10 times, or at least 20 times, it may be confirmed as the presence of a particular disease, diagnosed as the development or risk of development of a particular disease, or assessed as certain progress of a particular disease. For example, in at least one, at least two, or at least three times of detection among many times of detection, when the modification status of a DNA region in a cell sample, a tissue sample, or a sample derived from a subject is higher than the corresponding reference level by at least 1%, at least 5%, at least 10%, at least 20%, at least 50%, at least 1 times, at least 2 times, at least 5 times, at least 10 times, or at least 20 times, it may be confirmed as the presence of a particular disease, diagnosed as the development or risk of development of a particular disease, or assessed as a certain progress of a particular disease.

For example, when the modification status of a DNA region in a cell sample, a tissue sample or a sample derived from a subject is lower than or substantially equal to the corresponding reference level (for example, the reference level here may refer to the modification status of a DNA region of a patient with a particular disease), it may be confirmed as the absence of a particular disease, not diagnosed as the development or risk of development of a particular disease, or not assessed as certain progress of a particular disease. For example, when the modification status of a DNA region in a cell sample, a tissue sample, or a sample derived from a subject is lower than the corresponding reference level by at least 1%, at least 5%, at least 10%, at least 20%, at least 50%, and at least 100%, it may be confirmed as the absence of a particular disease, not diagnosed as the development or risk of development of a particular disease, or not assessed as certain progress of a particular disease.

Reference levels can be selected by those skilled in the art based on the desired sensitivity and specificity. For example, the reference levels in various situations in the present application may be readily identifiable by those skilled in the art. For example, appropriate reference levels and/or appropriate means of obtaining the reference levels can be identified based on a limited number of attempts. For example, the reference levels may be derived from one or more reference samples, where the reference levels are obtained from experiments performed in parallel with experiments testing the sample of interest. Alternatively, reference levels may be obtained in a database that includes a collection of data, standards or levels from one or more reference samples or disease reference samples. In some embodiments, a set of data, standards or levels can be standardized or normalized so that it can be compared with data from one or more samples and thereby used to reduce errors arising from different detection conditions.

For example, the reference levels may be derived from a database, which may be a reference database that includes, for example, modification status levels of target markers from one or more reference samples and/or other laboratories and clinical data. For example, a reference database can be established by aggregating reference level data from reference samples obtained from healthy individuals and/or individuals not suffering from the corresponding disease (i.e., individuals known not to have the disease). For example, a reference database can be established by aggregating reference level data from reference samples obtained from individuals with the corresponding disease under treatment. For example, a reference database can be built by aggregating data from reference samples obtained from individuals at different stages of the disease. For example, different stages may be evidenced by different modification status levels of the marker of interest of the present application. Those skilled in the art can also determine whether an individual suffers from the corresponding disease or is at risk of suffering from the corresponding disease based on various factors, such as age, gender, medical history, family history, symptoms.

For example, the method of the present application may comprise the following steps: obtaining the nucleic acid in the sample to be tested; transforming the DNA region or a fragment thereof; and determining the presence and/or the content of the substance formed by the base with the modification status after the transformation.

For example, the method of the present application may comprise the following steps: obtaining the nucleic acid in the sample to be tested; transforming the DNA region or a fragment thereof; amplifying the DNA region or a fragment thereof in the sample to be detected; and determining the presence and/or the content of the substance formed by the base with the modification status after the transformation.

For example, the method of the present application may comprise the following steps: obtaining the nucleic acid in the sample to be tested; treating the DNA obtained from the sample to be tested with a reagent capable of differentiating unmethylated sites and methylated sites in the DNA, thereby obtaining treated DNA; optionally amplifying the DNA region or a fragment thereof in the sample to be tested; quantitatively, semi-quantitatively or qualitatively analyzing the presence and/or the content of methylation status of the treated DNA in the sample to be tested; comparing the methylation level of the treated DNA in the sample to be tested with the corresponding reference level. When the methylation status of the DNA region in the sample to be tested is higher than or substantially equal to the corresponding reference level, it may be confirmed as presence of a particular disease, diagnosed as the development or risk of development of a particular disease, or assessed as certain progress of a particular disease.

In another aspect, the present application provides a nucleic acid, which may comprise a sequence capable of binding to a DNA region in which a target gene is located, or a complementary region thereabove, or a transformed region thereabove, or a fragment thereabove of the present application. For example, the nucleic acid can be any probe of the present application. In another aspect, the present application provides a method for preparing a nucleic acid, which may comprise designing a nucleic acid capable of binding to a DNA region in which a target gene is located, or a complementary region thereof, or a transformed region thereabove, or a fragment thereabove of the present application, based on the modification status of the DNA region, or a complementary region thereof, or a transformed region thereabove, or a fragment thereabove. For example, the method of preparing nucleic acids can be any suitable method known in the art.

In another aspect, the present application provides a nucleic acid set, which may comprise a sequence capable of binding to a DNA region in which a target gene is located, or a complementary region thereof, or a transformed region thereabove, or a fragment thereabove of the present application. For example, the nucleic acid set can be any primer set of the present application. In another aspect, the present application provides a method for preparing a nucleic acid set, which may comprise designing a nucleic acid set capable of amplifying a DNA region in which a target gene is located, or a complementary region thereof, or a transformed region thereabove, or a fragment thereabove of the present application, based on the modification status of the DNA region, or a complementary region thereof, or a transformed region thereabove, or a fragment thereabove. For example, the method of preparing the nucleic acids in the nucleic acid set can be any suitable method known in the art. For example, the methylation status of a target polynucleotide can be assessed using a single probe or primer configured to hybridize with the target polynucleotide. For example, the methylation status of a target polynucleotide can be assessed using multiple probes or primers configured to hybridize with the target polynucleotide.

In another aspect, the present application provides a nucleic acid, which comprises a sequence capable of binding to a DNA region in which a target gene is located, or a complementary region thereof, or a transformed region thereabove, or a fragment thereabove in the method of the present application; and in another aspect, the present application provides a nucleic acid, which may comprise a sequence capable of binding to the target DNA region, or a complementary region thereof, or a transformed region thereabove, or a fragment thereabove in the method of the present application.

In another aspect, the present application provides a method for preparing a nucleic acid, which may comprise designing a nucleic acid capable of binding to a DNA region in which a target gene is located, or a complementary region thereof, or a transformed region thereabove, or a fragment thereabove in the method of the present application, based on the modification status of the DNA region in which a target gene is located, or a complementary region thereof, or a transformed region thereabove, or a fragment thereabove; and in another aspect, the present application provides a nucleic acid, the method may comprise designing a nucleic acid capable of binding to the target DNA region, or a complementary region thereof, or a transformed region thereabove, or a fragment thereabove in the method of the present application, based on the modification status of the target DNA region, or a complementary region thereof, or a transformed region thereabove, or a fragment thereabove.

In another aspect, the present application provides a nucleic acid set, which comprises a sequence capable of binding to a DNA region in which a target gene is located, or a complementary region thereof, or a transformed region thereabove, or a fragment thereabove in the method of the present application; and in another aspect, the present application provides a nucleic acid, and the nucleic acid set may comprise a sequence capable of binding to the target DNA region, or a complementary region thereof, or a transformed region thereabove, or a fragment thereabove in the method of the present application. For example, the method of preparing nucleic acids can be any suitable method known in the art.

In another aspect, the present application provides a method for preparing a nucleic acid set, which may comprise designing a nucleic acid set capable of binding to a DNA region in which a target gene is located, or a complementary region thereof, or a transformed region thereabove, or a fragment thereabove in the method of the present application, based on the modification status of the DNA region in which a target gene is located, or a complementary region thereof, or a transformed region thereabove, or a fragment thereabove; and in another aspect, the present application provides a nucleic acid, the method may comprise designing a nucleic acid set capable of binding to the target DNA region, or a complementary region thereof, or a transformed region thereabove, or a fragment thereabove in the method of the present application, based on the modification status of the target DNA region, or a complementary region thereof, or a transformed region thereabove, or a fragment thereabove. For example, the methylation status of a target polynucleotide can be assessed using a single probe or primer configured to hybridize with the target polynucleotide. For example, the methylation status of a target polynucleotide can be assessed using multiple probes or primers configured to hybridize with the target polynucleotide.

In another aspect, the present application provides a kit, which may comprise the nucleic acid of the present application and/or the nucleic acid set of the present application. For example, the kit of the present application may optionally comprise reference samples for corresponding uses or provide reference levels for corresponding uses.

### Diagnostic methods, and preparation uses

For example, the methods of the present application can be used for diagnosis, prognosis, stratification, risk assessment, or treatment monitoring of cancer or neoplasia in a subject. For example, a "subject" may mean an organism, or a part or component of the organism, to which the method, nucleic acid, nucleic acid set, kit, device, and system provided by the present application may be administered or applied. For example, the subject may be a mammal or a cell, tissue, organ or part of the mammal. As used herein, "mammal" means any kind of mammal, preferably a person (including a human, a human subject, or a human patient). Subjects and mammals include, but are not limited to, farm animals, sports animals, pets, primates, horses, dogs, cats, and rodents such as mice and rats.

In another aspect, the method of the present application may be used to assess cancer or neoplasia in any suitable subject. For example, the method of the present application may be used to assess cancer or neoplasia in mammals. The mammal may be a non-human mammal, such as a pet, farm animal, companion animal or laboratory animal. Preferably, the mammal is a human. For example, a subject may be a person who needs to be screened for risk of cancer or neoplasia, a person who is in a high-risk population, a person who has been diagnosed with cancer or neoplasia but needs further stratification or classification, a person who has been diagnosed with cancer or neoplasia and are receiving active treatment, or a person who has cancer or neoplasia and are in remission.

In another aspect, the present application provides the use of the nucleic acid of the present application, the nucleic acid set of the present application and/or the kit of the present application in the preparation of a substance for determining the modification status of a DNA region or a fragment thereof.

In another aspect, the present application provides a method for determining the modification status of the DNA region or a fragment thereof, which may comprise providing the nucleic acid of the present application, the nucleic acid set of the present application and/or the kit of the present application.

In another aspect, the present application provides the nucleic acid of the present application, the nucleic acid set of the present application and/or the kit of the present application, which may be used for determining the modification status of the DNA region or a fragment thereof.

In another aspect, the present application provides the use of the nucleic acid of the present application, the nucleic acid set of the present application and/or the kit of the present application in the preparation of a disease detection product. For example, in the use of the present application, the disease may comprise tumors. For example, it is a solid tumor. For example, in the use of the present application, the disease may comprise a liver tumor.

In another aspect, the present application provides a detection method for disease , which may include providing the nucleic acid of the present application, the nucleic acid set of the present application and/or the kit of the present application. For example, in the use of the present application, the disease may comprise tumors. For example, it is a solid tumor. For example, in the use of the present application, the disease may comprise a liver tumor.

In another aspect, the present application provides the nucleic acid of the present application, the nucleic acid set of the present application and/or the kit of the present application for use in disease detection. For example, in the use of the present application, the disease may comprise tumors. For example, it is a solid tumor. For example, in the use of the present application, the disease may comprise a liver tumor.

In another aspect, the present application provides the use of the nucleic acid of the present application, the nucleic acid set of the present application and/or the kit of the present application in the preparation of a substance for identifying the presence of a disease, assessing the development or risk of development of a disease and/or assessing the progress of a disease. For example, in the use of the present application, the disease may comprise tumors. For example, it is a solid tumor. For example, in the use of the present application, the disease may comprise a liver tumor.

In another aspect, the present application provides a method for identifying the presence of a disease, assessing the development or development risk of the disease, and/or assessing the progress of the disease, which may comprise providing the nucleic acid of the present application, the nucleic acid set of the present application and/or the kit of the present application. For example, in the use of the present application, the disease may comprise tumors. For example, it is a solid tumor. For example, in the use of the present application, the disease may comprise a liver tumor.

In another aspect, the present application provides the nucleic acid of the present application, the nucleic acid set of the present application and/or the kit of the present application, which may be used for identifying the presence of a disease, assessing the development or risk of development of a disease and/or assessing the progress of a disease. For example, in the use of the present application, the disease may comprise tumors. For example, it is a solid tumor. For example, in the use of the present application, the disease may comprise a liver tumor.

In another aspect, the present application provides the use of a nucleic acid, a nucleic acid set and/or a kit for determining the modification status of a DNA region, in the preparation of a substance for identifying the presence of a liver tumor, assessing the development or risk of development of a liver tumor and/or assessing the progress of a liver tumor, wherein the DNA region for determination includes a DNA region in which a target gene is located, or a fragment thereof in the method of the present application.

In another aspect, the present application provides a method for identifying the presence of a liver tumor, assessing the development or risk of development of a liver tumor, and/or assessing the progress of a liver tumor, which may comprise providing a nucleic acid, a nucleic acid set and/or a kit for determining the modification status of a DNA region. The DNA region used for determination may comprise a DNA region in which a target gene is located, or a fragment thereof in the method of the present application.

In another aspect, the present application provides a nucleic acid, a nucleic acid set and/or a kit for determining the modification status of a DNA region, which may be used for identifying the presence of a liver tumor, assessing the development or risk of development of a liver tumor, and/or assessing the progress of a liver tumor. The DNA region used for determination may comprise a DNA region in which a target gene is located, or a fragment thereof in the method of the present application.

In another aspect, the present application provides the use of a nucleic acid, a nucleic acid set and/or a kit for determining the modification status of a DNA region, in the preparation of a substance for identifying the presence of a disease, assessing the development or risk of development of a disease and/or assessing the progress of a disease, wherein the DNA region for determination may comprise the target DNA region in the method of the present application, a complementary region thereof, or a fragment thereabove.

In another aspect, the present application provides a method for identifying the presence of a disease, assessing the development or risk of development of a disease, and/or assessing the progress of a disease, which may comprise providing a nucleic acid, a nucleic acid set and/or a kit for determining the modification status of a DNA region. The DNA region used for determination may comprise the target DNA region, a complementary region thereof, or a fragment thereabove in the method of the present application.

In another aspect, the present application provides a nucleic acid, a nucleic acid set and/or a kit for determining the modification status of a DNA region, which may be used for identifying the presence of a disease, assessing the development or risk of development of a disease, and/or assessing the progress of a disease. The DNA region used for determination may comprise the target DNA region, a complementary region thereof, or a fragment thereabove in the method of the present application.

For example, in the use of the present application, the disease may comprise tumors. For example, it is a solid tumor. For example, in the use of the present application, the disease may comprise a liver tumor. For example, in the use of the present application, the modification may include methylation modification.

In another aspect, the present application provides the use of nucleic acids of a DNA region in which a target gene is located, or a transformed region thereabove, or a fragment thereabove, or a combination of the nucleic acids in the method of the present application, in the preparation of a substance for identifying the presence of a liver tumor, assessing the development or risk of development of a liver tumor, and/or assessing the progress of a liver tumor.

In another aspect, the present application provides a method for identifying the presence of a liver tumor, assessing the development or risk of development of a liver tumor, and/or assessing the progress of a liver tumor, which may comprise providing nucleic acids of a DNA region in which a target gene is located, or a transformed region thereabove, or a fragment thereabove, or a combination of the nucleic acids in the method of the present application.

In another aspect, the present application provides nucleic acids of a DNA region in which a target gene is located, or a transformed region thereabove, or a fragment thereabove, or a combination of the nucleic acids in the method of the present application, which may be used for identifying the presence of a liver tumor, assessing the development or risk of development of a liver tumor, and/or assessing the progress of a liver tumor.

In another aspect, the present application provides the use of nucleic acids of the target DNA region, or a complementary region thereof, or a transformed region thereabove, or a fragment thereabove, or a combination of the nucleic acids in the method of the present application, in the preparation of a substance for identifying the presence of a disease, assessing the development or risk of development of a disease, and/or assessing the progress of a disease. For example, in the use of the present application, the disease may comprise tumors. For example, in the use of the present application, the disease may comprise a liver tumor.

In another aspect, the present application provides a method for identifying the presence of a disease, assessing the development or risk of development of a disease, and/or assessing the progress of a disease, which may comprise providing nucleic acids of the target DNA region, or a complementary region thereof, or a transformed region thereabove, or a fragment thereabove, or a combination of the nucleic acids in the method of the present application. For example, in the use of the present application, the disease may comprise tumors. For example, in the use of the present application, the disease may comprise a liver tumor.

In another aspect, the present application provides nucleic acids of the target DNA region, or a complementary region thereof, or a transformed region thereabove, or a fragment thereabove, or a combination of the nucleic acids in the method of the present application, which may be used for identifying the presence of a disease, assessing the development or risk of development of a disease, and/or assessing the progress of a disease. For example, in the use of the present application, the disease may comprise tumors. For example, in the use of the present application, the disease may comprise a liver tumor.

In another aspect, the present application provides a device which may comprises the storage medium of the present application. In another aspect, the present application provides a non-volatile computer-readable storage medium on which a computer program is stored, and the program is executed by a processor to implement any one or more methods of the present application. For example, the method may include a method for classifying samples with different possibilities of being cancerous by using methylation indicators. Exemplary classification algorithms may be linear discriminant analysis, logistic regression, naive Bayes classification, categorization of perception, binary classification, k-nearest neighbor, lifting scheme, decision-making tree, random forest, neural network, learning vector quantization, or support vector machines, or a combination thereof. For example, the non-volatile computer-readable storage medium may include floppy disks, flexible disks, hard disks, solid state storage (SSS) (such as solid state drives (SSD)), solid state cards (SSC), solid state modules (SSM)), enterprise flash drives, magnetic tapes, or any other non-transitory magnetic media, etc. Non-volatile computer-readable storage media may also include punched card, paper tape, optical mark card (or any other physical media having a hole pattern or other optically identifiable markings), compact disk read-only memory (CD-ROM), compact disc rewritable (CD-RW), digital versatile disc (DVD), blu-ray disc (BD) and/or any other non-transitory optical media.

For example, the device of the present application may further include a processor coupled to the storage medium, and the processor is configured to execute based on a program stored in the storage medium to implement the method of the present application. For example, the device may implement various mechanisms to ensure that the method of the present application when executed on a database system produce correct results. In the present application, the device may use magnetic disks as permanent data storage. In the present application, the device can provide database storage and processing services for multiple database clients. The device may store database data across multiple shared storage devices and/or may utilize one or more execution platforms with multiple execution nodes. The device can be organized so that storage and computing resources can be expanded effectively infinitely.

The present invention uses fluorescence PCR detection technology to detect the methylation status of markers related to the buffy coat (most of which are white blood cells), para-cancerous tissue and liver cancer tissue. The potential of the marker for detection of liver tumors in blood is preliminarily verified. An ideal liver cancer methylation detection marker should have the following characteristics: 1. low DNA methylation level in the buffy coat; and 2. high DNA methylation level in the liver cancer tissue.

Detection for methylation marker can be performed using methylation-specific PCR (MSP). The basic principle of MSP is that during detection, only methylated sequence templates produce amplification signals, and unmethylated sequence templates do not produce amplification signals. This method can be realized by designing methylation-specific sequences using primers or probes, or can also be realized by designing methylation-specific primers and probe pairs at the same time. The main steps include: 1. based on the marker sequence, designing detection primers and probes in the detection segment suitable for MSP (which can be a CpG-rich region); 2. extraction of nucleic acids from white blood cell and tissue samples; 3. treating the nucleic acid with bisulfite to transform unmethylated cytosine into uracil, with the sequence of methylated cytosine unchanged; and 4. fluorescence PCR detection.

Markers that meet the above characteristics, tested by using white blood cell and tissue samples, prove their potential to detect liver cancer by blood. These markers are then verified in plasma samples, the detection samples including control group, liver cancer group, and populations interfered with hepatitis B and liver cirrhosis. The reference levels of these markers and the performance of marker combinations are analyzed. Since the plasma free DNA of a single sample is limited, optionally, when performing fluorescence PCR detection, the target can be pre-amplified so that with the minimum amount of DNA, as many methylation sites as possible can be detected. Its main steps include: 1. nucleic acid extraction from plasma samples; 2. treating the nucleic acid with bisulfite to transform unmethylated cytosine into uracil, with the sequence of methylated cytosine unchanged; 3. pre-amplification and dilution of the target; and 4. fluorescence PCR detection.

During analysis of target combinations, any combination can be further made based on the above target combinations, or machine learning mathematical models can be introduced into the data algorithm, such as linear regression, support vector regression, ridge regression, random forest, etc., to obtain a combination of liver cancer methylation detection markers with high detection accuracy.

The contents provided by the present application are: 1. many liver cancer methylation markers with excellent performance are found; 2. the combination of detection markers for liver cancer methylation with high detection accuracy is developed; 3. the detection performance is greatly improved compared with existing fluorescence PCR detection methods; and 4. compared with the second-generation sequencing detection methods, it has many advantages such as easy development, easy clinical promotion, and low cost. The application methods of the markers and combinations of markers provided by the present application are not limited to specific detection methods.

### Embodiments

1. A method for identifying the presence of a liver tumor, assessing the development or development risk of the liver tumor, and/or assessing the progress of the liver tumor, comprising determining the presence and/or the content of modification status of a DNA region in which BCAT1, IKZF1, VAV3, IRF4, B4GALNT1, GRASP, BEST4, BEND4, GPAM and VASH2 genes are located, a DNA region derived from human chr1:145384249:145413094, and/or a DNA region derived from human chr7:26415938:26416740, or a fragment thereof in a sample to be tested.
2. A method for assessing a methylation status of a liver tumor-related DNA region, comprising determining the presence and/or the content of modification status of a DNA region in which BCAT1, IKZF1, VAV3, IRF4, B4GALNT1, GRASP, BEST4, BEND4, GPAM and VASH2 genes are located, a DNA region derived from human chr1:145384249:145413094, and/or a DNA region derived from human chr7:26415938:26416740, or a fragment thereof in a sample to be tested.
3. The method of any one of embodiments 1-2, wherein the DNA region is derived from human chr12:24964295-25102393, derived from human chr7:50343720:50472799, derived from human chr1:108113782-108507766, derived from human chr6:391739-411447, derived from human chr12: 58017193-58027138, derived from human chr12: 52400724-52409673, derived from human chr1: 45249257-45253377, derived from human chr4: 42112955-42154895, derived from human chr1:145384249:145413094, derived from human chr7:26415938:26416740, derived from human chr10: 113909624-113975135, and/or derived from human chr1: 213123862-213165379.
4. The method of any one of embodiments 1-3, further comprising obtaining a nucleic acid in the sample to be tested.
5. The method of embodiment 4, wherein the nucleic acid comprises a cell-free free nucleic acid.
6. The method of any one of embodiments 1-5, wherein the sample to be tested comprises a tissue, a cell and/or a body fluid.
7. The method of any one of embodiments 1-6, wherein the sample to be tested comprises a plasma.
8. The method of any one of embodiments 1-7, further comprising transforming the DNA region or a fragment thereof.
9. The method of embodiment 8, wherein the base with the modification status and the base without the modification status form different substances after the transformation.
10. The method of any one of embodiments 8-9, wherein the base with the modification status is substantially unchanged after the transformation, and the base without the modification status is changed to another base different from the base after the transformation, or is cleaved after the transformation.
11. The method of any one of embodiments 9-10, wherein the base comprises cytosine.
12. The method of any one of embodiments 1-11, wherein the modification status comprises a methylation modification.
13. The method of any one of embodiments 10-12, wherein the other base comprises uracil.
14. The method of any one of embodiments 8-13, wherein the transformation comprises transformation by a deamination reagent and/or a methylation sensitive restriction enzyme.
15. The method of embodiment 14, wherein the deamination reagent comprises a bisulfite or an analog thereof.
16. The method of any one of embodiments 1-15, wherein the method for determining the presence and/or the content of modification status comprises identifying the presence and/or the content of a substance formed by a base with the modification status after the transformation.
17. The method of any one of embodiments 1-16, wherein the method for determining the presence and/or the content of modification status comprises determining the presence and/or the content of a DNA region with the modification status or a fragment thereof.
18. The method of any one of embodiments 1-17, wherein the presence and/or the content of a DNA region with the modification status or a fragment thereof is determined by the fluorescence Ct value detected by the fluorescence PCR method.
19. The method of any one of embodiments 1-18, wherein the presence of a liver tumor or the development or development risk of the liver tumor is determined by identifying the presence of a modification status of the DNA region or a fragment thereof, and/or the content of modification status of which the DNA region or a fragment thereof is higher relative to a reference level.
20. The method of any one of embodiments 1-19, further comprising amplifying the DNA region or a fragment thereof in the sample to be tested before determining the presence and/or the content of a modification of the DNA region or a fragment thereof.
21. The method of embodiment 20, wherein the amplification comprises PCR amplification.
22. A method for identifying the presence of a disease, assessing the development or development risk of the disease and/or assessing the progress of the disease, comprising determining the presence and/or the content of modification status of a DNA region selected from the following group, or a complementary region thereof, or a fragment thereabove in a sample to be tested: derived from human chr12:25101630-25102393, derived from human chr:12:25078661-25079410 and derived from human chr12:25054781-25056311, or derived from human chr7:50343720-50344547, derived from human chr7:50450118-50450531 and derived from human chr7:50467368-50469092, or derived from human chr1:108507215-108507766, or derived from human chr6:391739-394056 and derived from human chr6:401233-401801, or derived from human chr12:58020498-58022962 and derived from human chr12:58025539-58027138, or derived from human chr12:52400724-52401698 and derived from human chr12:52406880-52409127, or derived from human chr1:45251728-45252477 and derived from human chr1:45249853-45250527, or derived from human chr4:42152705-42154895, or derived from human chr1:145389746-145401075, derived from human chr1:145384910-145385929 and derived from human chr1:145406714-145408013, or derived from human chr7:26416257-26416363 and derived from human chr7:26416026-26416126, or derived from human chr10:113942657-113943906, or derived from human chr1:213123862-213125211.
23. A method for determining a methylation status of a DNA region, comprising determining the presence and/or the content of modification status of a DNA region selected from the following group, or a complementary region thereof, or a fragment thereabove in a sample to be tested: derived from human chr12:25101630-25102393, derived from human chr:12:25078661-25079410 and derived from human chr12:25054781-25056311, or derived from human chr7:50343720-50344547, derived from human chr7:50450118-50450531 and derived from human chr7:50467368-50469092, or derived from human chr1:108507215-108507766, or derived from human chr6:391739-394056 and derived from human chr6:401233-401801, or derived from human chr12:58020498-58022962 and derived from human chr12:58025539-58027138, or derived from human chr1:45251728-45252477 and derived from human chr1:45249853-45250527, or derived from human chr4:42152705-42154895, or derived from human chr1:145389746-145401075, derived from human chr1:145384910-145385929 and derived from human chr1:145406714-145408013, or derived from human chr7:26416257-26416363 and derived from human chr7:26416026-26416126, or derived from human chr10:113942657-113943906, or derived from human chr1:213123862-213125211.
24. The method of any one of embodiments 22-23, comprising providing a nucleic acid capable of binding to a DNA region comprising a region selected from the following group, or a complementary region thereof, or a transformed region thereabove, or a fragment thereabove: SEQ ID NO: 32, SEQ ID NO: 39 and SEQ ID NO: 43, or SEQ ID NO: 5, SEQ ID NO: 50 and SEQ ID NO: 54, or SEQ ID NO: 58, or SEQ ID NO: 21 and SEQ ID NO: 71, or SEQ ID NO: 13 and SEQ ID NO: 78, or SEQ ID NO: 17 and SEQ ID NO: 85, or SEQ ID NO: 9 and SEQ ID NO: 92, or SEQ ID NO: 25, or SEQ ID NO: 99, SEQ ID NO: 106 and SEQ ID NO: 110, or SEQ ID NO: 120 or SEQ ID NO: 127.
25. The method of any one of embodiments 22-24, comprising providing a nucleic acid capable of binding to a DNA region comprising a region selected from the following group, or a complementary region thereof, or a transformed region thereabove, or a fragment thereabove: derived from human chr12:25102016-25102110, derived from human chr12:25101992-25102093, derived from human chr12:25079051-25079133 and derived from human chr12:25056027-25056134, or derived from human chr7:50343793-50343896, derived from human chr7:50343867-50343961, derived from human chr7:50450311-50450411 and derived from human chr7:50467865-50467980, or derived from human chr1:108507591-108507674 and derived from human chr1:108507624-108507725, or derived from human chr6:392282-392377, derived from human chr6:392036-392145, derived from human chr6:392405-392500 and derived from human chr6:401641-401752, or derived from human chr12:58021586-58021670, derived from human chr12:58021907-58021987 and derived from human chr12:58026383-58026475, or derived from human chr12:52401083-52401169, derived from human chr12:52400965-52401055 and derived from human chr12:52408471-52408566, or derived from human chr1:45252095-45252176, derived from human chr1:45252187-45252275 and derived from human chr1:45249894-45249981, or derived from human chr4:42153816-42153921 and derived from human chr4:42153513-42153601, or derived from human chr1:145399249:145399476, derived from human chr1:145396880-145396983, derived from human chr1:145385298-145385376 and derived from human chr1:145407431-145407518, or derived from human chr10:113943540:113943739 and derived from human chr10:113943511-113943582, or derived from human chr1:213124569-213124670 and derived from human chr1:213124036-213124162.
26. The method of any one of embodiments 22-25, comprising providing a nucleic acid selected from the following group, or a complementary nucleic acid thereof, or a fragment thereabove: SEQ ID NO: 33, SEQ ID NO: 36, SEQ ID NO: 40 and SEQ ID NO: 44, or SEQ ID NO: 6, SEQ ID NO: 47, SEQ ID NO: 51 and SEQ ID NO: 55, or SEQ ID NO: 59 and SEQ ID NO: 62, or SEQ ID NO: 22, SEQ ID NO: 65, SEQ ID NO: 68 and SEQ ID NO: 72, or SEQ ID NO: 14, SEQ ID NO: 75 and SEQ ID NO: 79, or SEQ ID NO: 18, SEQ ID NO: 82 and SEQ ID NO: 86, or SEQ ID NO: 10, SEQ ID NO: 89 and SEQ ID NO: 93, or SEQ ID NO: 26 and SEQ ID NO: 96, or SEQ ID NO: 100, SEQ ID NO: 103, SEQ ID NO: 107 and SEQ ID NO: 111, or SEQ ID NO: 114 and SEQ ID NO: 117, or SEQ ID NO: 121 and SEQ ID NO: 124, or SEQ ID NO: 128 and SEQ ID NO: 131.
27. The method of any one of embodiments 22-26, comprising providing a nucleic acid set selected from the following group, or a complementary nucleic acid set thereof, or a fragment thereabove: SEQ ID NOs: 34 and 35, SEQ ID NOs: 37 and 38, SEQ ID NOs: 41 and 42, and SEQ ID NOs: 45 and 46, or SEQ ID NOs: 7 and 8, SEQ ID NOs: 48 and 49, SEQ ID NOs: 52 and 53, and SEQ ID NOs: 56 and 57, or SEQ ID NOs: 60 and 61, and SEQ ID NOs: 63 and 64, or SEQ ID NOs: 23 and 24, SEQ ID NOs: 66 and 67, SEQ ID NOs: 69 and 70, and SEQ ID NOs: 73 and 74, or SEQ ID NOs: 15 and 16, SEQ ID NOs: 76 and 77, and SEQ ID NOs: 80 and 81, or SEQ ID NOs: 19 and 20, SEQ ID NOs: 83 and 84, and SEQ ID NOs: 87 and 88, or SEQ ID NOs: 11 and 12, SEQ ID NOs: 90 and 91, and SEQ ID NOs: 94 and 95, or SEQ ID NOs: 27 and 28, and SEQ ID NOs: 97 and 98, or SEQ ID NOs: 101 and 102, SEQ ID NOs: 104 and 105, SEQ ID NOs: 108 and 109, and SEQ ID NOs: 112 and 113, or SEQ ID NOs: 115 and 116, and SEQ ID NOs: 118 and 119, or SEQ ID NOs: 122 and 123, and SEQ ID NOs: 125 and 126, or SEQ ID NOs: 129 and 130, and SEQ ID NOs: 132 and 133.
28. The method of any one of embodiments 22-27, wherein the disease comprises a tumor.
29. The method of any one of embodiments 22-28, further comprising obtaining a nucleic acid in the sample to be tested.
30. The method of embodiment 29, wherein the nucleic acid comprises a cell-free free nucleic acid.
31. The method of any one of embodiments 22-30, wherein the sample to be tested comprises a tissue, a cell and/or a body fluid.
32. The method of any one of embodiments 22-31, wherein the sample to be tested comprises a plasma.
33. The method of any one of embodiments 22-32, further comprising transforming the DNA region or a fragment thereof.
34. The method of embodiment 33, wherein the base with the modification status and the base without the modification status form different substances after the transformation.
35. The method of any one of embodiments 33-34, wherein the base with the modification status is substantially unchanged after the transformation, and the base without the modification status is changed to another base different from the base after the transformation, or is cleaved after the transformation.
36. The method of any one of embodiments 34-35, wherein the base comprises cytosine.
37. The method of any one of embodiments 22-36, wherein the modification status comprises a methylation modification.
38. The method of any one of embodiments 35-37, wherein the other base comprises uracil.
39. The method of any one of embodiments 33-38, wherein the transformation comprises transformation by a deamination reagent and/or a methylation sensitive restriction enzyme.
40. The method of embodiment 39, wherein the deamination reagent comprises a bisulfite or an analog thereof.
41. The method of any one of embodiments 22-40, wherein the method for determining the presence and/or the content of modification status comprises identifying the presence and/or the content of a substance formed by a base with the modification status after the transformation.
42. The method of any one of embodiments 22-41, wherein the method for determining the presence and/or the content of modification status comprises determining the presence and/or the content of a DNA region with the modification status or a fragment thereof.
43. The method of any one of embodiments 22-42, wherein the presence and/or the content of a DNA region with the modification status or a fragment thereof is determined by the fluorescence Ct value detected by the fluorescence PCR method.
44. The method of any one of embodiments 22-43, wherein the presence of a liver tumor or the development or development risk of the liver tumor is determined by identifying the presence of a modification status of the DNA region or a fragment thereof, and/or the content of modification status of which the DNA region or a fragment thereof is higher relative to a reference level.
45. The method of any one of embodiments 22-44, further comprising amplifying the DNA region or a fragment thereof in the sample to be tested before determining the presence and/or the content of a modification of the DNA region or a fragment thereof.
46. The method of embodiment 45, wherein the amplification comprises PCR amplification.
47. A nucleic acid, comprising a sequence capable of binding to a DNA region in which BCAT1, IKZF1, VAV3, IRF4, B4GALNT1, GRASP, BEST4, BEND4, GPAM and VASH2 genes are located, a DNA region derived from human chr1:145384249:145413094 and/or a DNA region derived from human chr7:26415938:26416740, or a complementary region thereof, or a transformed region thereabove, or a fragment thereabove.
48. A method for preparing a nucleic acid, comprising, based on modification status of a DNA region in which BCAT1, IKZF1, VAV3, IRF4, B4GALNT1, GRASP, BEST4, BEND4, GPAM and VASH2 genes are located, a DNA region derived from human chr1:145384249:145413094, and/or a DNA region derived from human chr7:26415938:26416740, or a complementary region thereof, or a transformed region thereabove, or a fragment thereabove, designing a nucleic acid capable of binding to the DNA region, or a complementary region thereof, or a transformed region thereabove, or a fragment thereabove.
49. A nucleic acid set, comprising a sequence capable of binding to a DNA region in which BCAT1, IKZF1, VAV3, IRF4, B4GALNT1, GRASP, BEST4, BEND4, GPAM and VASH2 genes are located, a DNA region derived from human chr1:145384249:145413094 and/or a DNA region derived from human chr7:26415938:26416740, or a complementary region thereof, or a transformed region thereabove, or a fragment thereabove.
50. A method for preparing a nucleic acid set, comprising, based on modification status of a DNA region in which BCAT1, IKZF1, VAV3, IRF4, B4GALNT1, GRASP, BEST4, BEND4, GPAM and VASH2 genes are located, a DNA region derived from human chr1:145384249:145413094, and/or a DNA region derived from human chr7:26415938:26416740, or a complementary region thereof, or a transformed region thereabove, or a fragment thereabove, designing a nucleic acid set capable of amplifying the DNA region, or a complementary region thereof, or a transformed region thereabove, or a fragment thereabove.
51. A kit, comprising the nucleic acid of embodiment 47 and/or the nucleic acid set of embodiment 49.
52. Use of the nucleic acid of embodiment 47, the nucleic acid set of embodiment 49, and/or the kit of embodiment 51 in the preparation of a disease detection product.
53. Use of the nucleic acid of embodiment 47, the nucleic acid set of embodiment 49 and/or the kit of embodiment 51 in the preparation of a substance for identifying the presence of a disease, assessing the development or development risk of the disease and/or assessing the progress of the disease.
54. Use of the nucleic acid of embodiment 47, the nucleic acid set of embodiment 49 and/or the kit of embodiment 51 in the preparation of a substance for determining a modification status of the DNA region or a fragment thereof.
55. Use of a nucleic acid, a nucleic acid set and/or a kit for determining modification status of a DNA region in the preparation of a substance for identifying the presence of a liver tumor, assessing the development or development risk of the liver tumor and/or assessing the progress of the liver tumor, wherein the DNA region for the determination comprises a DNA region in which BCAT1, IKZF1, VAV3, IRF4, B4GALNT1, GRASP, BEST4, BEND4, GPAM and VASH2 genes are located, a DNA region derived from human chrl:145384249:145413094, and/or a DNA region derived from human chr7:26415938:26416740.
56. Use of a nucleic acid, a nucleic acid set and/or a kit for determining modification status of a DNA region in the preparation of a substance for identifying the presence of a disease, assessing the development or development risk of the disease and/or assessing the progress of the disease, wherein the DNA region comprises a DNA region selected from the following group, or a complementary region thereof, or a fragment thereabove: derived from human chr12:25101630-25102393, derived from human chr:12:25078661-25079410 and derived from human chr12:25054781-25056311, or derived from human chr7:50343720-50344547, derived from human chr7:50450118-50450531 and derived from human chr7:50467368-50469092, or derived from human chr1:108507215-108507766, or derived from human chr6:391739-394056 and derived from human chr6:401233-401801, or derived from human chr12:58020498-58022962 and derived from human chr12:58025539-58027138, or derived from human chr12:52400724-52401698 and derived from human chr12:52406880-52409127, or derived from human chr1:45251728-45252477 and derived from human chr1:45249853-45250527, or derived from human chr4:42152705-42154895, or derived from human chr1:145389746-145401075, derived from human chr1:145384910-145385929 and derived from human chr1:145406714-145408013, or derived from human chr7:26416257-26416363 and derived from human chr7:26416026-26416126, or derived from human chr10:113942657-113943906, or derived from human chr1:213123862-213125211.
57. Use of a nucleic acid of a DNA region in which BCAT1, IKZF1, VAV3, IRF4, B4GALNT1, GRASP, BEST4, BEND4, GPAM and VASH2 genes are located, a DNA region derived from human chr1:145384249:145413094 and/or a DNA region derived from human chr7:26415938:26416740, or a transformed region thereabove, or a fragment thereabove, and a combination of the above nucleic acids in the preparation of a substance for identifying the presence of a liver tumor, assessing the development or development risk of the liver tumor and/or assessing the progress of the liver tumor.
58. Use of a nucleic acid of a DNA region selected from the following group, or a complementary region thereof, or a transformed region thereabove, or a fragment thereabove, and a combination of the above nucleic acids, in the preparation of a substance for identifying the presence of a disease, assessing the development or development risk of the disease and/or assessing the progress of the disease: derived from human chr12:25101630-25102393, derived from human chr:12:25078661-25079410 and derived from human chr12:25054781-25056311, or derived from human chr7:50343720-50344547, derived from human chr7:50450118-50450531 and derived from human chr7:50467368-50469092, or derived from human chr1:108507215-108507766, or derived from human chr6:391739-394056 and derived from human chr6:401233-401801, or derived from human chr12:58020498-58022962 and derived from human chr12:58025539-58027138, or derived from human chr12:52400724-52401698 and derived from human chr12:52406880-52409127, or derived from human chrl :45251728-45252477 and derived from human chr1:45249853-45250527, or derived from human chr4:42152705-42154895, or derived from human chr1:145389746-145401075, derived from human chr1:145384910-145385929 and derived from human chr1:145406714-145408013, or derived from human chr7:26416257-26416363 and derived from human chr7:26416026-26416126, or derived from human chr10:113942657-113943906, or derived from human chr1:213123862-213125211.
59. A storage medium recording a program capable of executing the method of any one of embodiments 1-46.
60. A device comprising the storage medium of embodiment 59.
61. The device of embodiment 59, further comprising a processor coupled to the storage medium, wherein the processor is configured to execute based on a program stored in the storage medium to implement the method of any one of embodiments 1-46.

Without wishing to be bound by any theory, the following examples are only for illustrating the methods and uses of the present application, and are not used to limit the scope of the invention of the present application.

### EXAMPLES

### Example 1

### Comparison of methylation abundance of DNA samples from liver cancer, paracancerous tissue and buffy coat

DNA samples were obtained from the buffy coats of healthy population without abnormality of liver and the cancerous tissues of patients with liver cancer (among them, 10 samples from buffy coats, and 10 samples from cancerous tissues). The buffy coat DNA was selected as the reference sample because most of the plasma free DNA was derived from the DNA released after the rupture of the buffy coat, and its background could be a basic background signal of the detection site of the plasma free DNA. According to the instructions, the buffy coat DNA was extracted with Qiagen QIAamp DNA Mini Kit, and the tissue DNA was extracted with Qiagen QIAamp DNA FFPE Tissue Kit.

A 20 ng sample of the DNA obtained in the above steps was treated with a bisulfite reagent (D5031, ZYMO RESEARCH) to obtain the transformed DNA.

The detection fluorescence Ct value of the marker was obtained by fluorescence PCR detection. In the fluorescence PCR reaction system, the final concentration of each primer was 500 nM, and the final concentration of each detection probe was 200 nM. The PCR reaction system comprises: 10µL of pre-amplified diluted product, 2.5µL of primer and probe premixed solution containing detection sites; and 12.5µL of PCR reagent (Luna^{®}Universal Probe qPCR Master Mix (NEB)). The PCR reaction conditions were as follows: 95°C for 5 minutes; 95°C for 30 seconds and 56°C for 60 seconds (fluorescence was collected), and 50 cycles were performed. Different fluorescence was detected in the corresponding fluorescence channels using ABI 7500 Real-Time PCR System. Ct values of the samples obtained from the buffy coat, paracancerous tissue and cancerous tissue were calculated and compared, and the target Ct value at which no amplification signal was detected was set as 50. Here, the primer sequence of each methylation marker was shown in Table 1-1, and the probe sequence was shown in Table 1-2.

**Table 1-1 Primer Sequences**

| SEQ ID NO. | Name | Sequence |
|---|---|---|
| 3 | SEPT9 (1) forward primer | AAATAATCCCATCCAACTA |
| 4 | SEPT9 (1) reverse primer | GATTCGTTGTTTATTAGTTATTATGT |
| 7 | IKZF1 forward primer | GTTTTTTTGGTTCGGAGTTG |
| 8 | IKZF1 reverse primer | CAAAACGAAACACGAAAAAAATA |
| 11 | BEST4 forward primer | TGTGGGtCgGAtttttAGAG |
| 12 | BEST4 reverse primer | TaCTTAAacGCTTCCCCAC |
| 15 | B4GALNT1 forward primer | AGtAGtTGtCgATAAGTGGT |
| 16 | B4GALNT1 reverse primer | GCCTaaAaaCAACCTCCCT |
| 19 | GRASP forward primer | GttttTtTtCgAtTtttTAtAGGG |
| 20 | GRASP reverse primer | aaCcgaaaAAaAAaaTaaaaaACTC |
| 23 | IRF4 forward primer | AAAAAAAAAAAAACTCCACATTT |
| 24 | IRF4 reverse primer | TAGTTGNGGAGTTTGGG |
| 27 | BEND4 forward primer | AGTttTtAAGTGGtttTGGGAT |
| 28 | BEND4 reverse primer | CCAaacgCAaAaCTCCTAC |
| 30 | SEPT9 (1a) forward primer | GTAGTTGGATGGGATTATTT |
| 31 | SEPT9 (1a) reverse primer | CACCCGCAAAATCCTCT |
| 33 | ACTB forward primer | GTGATGGAGGAGGTTTAGTAAGTT |
| 34 | ACTB reverse primer | CCAATAAAACCTACTCCTCCCTTAA |

**Table 1-2 Detection Probe Sequences**

| SEQ ID NO. | Name | Sequence |
|---|---|---|
| 2 | SEPT9 (1) probe | TTAACCGCGAAATCCGAC |
| 6 | IKZF1 probe | CGCCCCGTCGCCGAAT |
| 10 | BEST4 probe | CGGCGTATTTGCGTTTATTACGT |
| 14 | B4GALNT1 probe | AGATTTCGCGTATCGCGTTTT |
| 18 | GRASP probe | TGTTTTTTTTTCGGCGTTCGCG |
| 22 | IRF4 probe | ATCGTACGTAAGGTTCGGAGCGA |
| 26 | BEND4 probe | TAGGACGGCGACGACGA |
| 29 | SEPT9 (1a) probe | TTGTTGCGGTCGCGGACG |
| 32 | ACTB probe | ACCACCACCCAACACACAATAACAAACACA |

**Table 1-3 Summary of detection results for sample**

| Site | Average Ct of cancerous tissues | Average Ct of buffy coats | Ratio of detected cancerous tissues | Ratio of detected buffy coats | p value |
|---|---|---|---|---|---|
| IKZF1 | 29.983 | 50.000 | 80% | 0% | 1.19E-05 |
| SEPT9 (1) | 26.395 | 45.120 | 90% | 20% | 9.55E-09 |
| SEPT9 (1a) | 23.636 | 50.000 | 90% | 0% | 2.37E-11 |
| IRF4 | 24.328 | 48.800 | 90% | 0% | 2.20E-11 |
| BEND4 | 21.333 | 50.000 | 90% | 0% | 1.64E-11 |
| GRASP | 25.649 | 50.000 | 80% | 0% | 6.00E-08 |
| B4GALNT1 | 19.258 | 50.000 | 100% | 0% | 1.73E-27 |
| BEST4 | 24.498 | 50.000 | 80% | 0% | 1.29E-08 |

The above results showed that the average Ct value for cancerous tissue detection was small, indicating a stronger methylation signal. The detection rate of methylation signal in cancerous tissue could be much higher than that in buffy coat, also indicating a strong methylation signal. Target methylation signal could not be detected in most samples of buffy coat. These targets might all have the potential for detection of liver cancer in blood. It was proved that the selected target markers were feasible and specific for tumor tissue.

### Comparison of methylation signals in plasma samples from patients with liver cancer and population without abnormality of liver

266 healthy control plasma samples without abnormality of liver, 372 preoperative plasma samples from patients with liver cancer (among which stage I accounted for 86%), 37 plasma samples from patients with hepatitis B and 39 plasma samples from patients with liver cirrhosis were selected for detection.

The extracellular free DNA in the above plasma samples was extracted using commercial Qiagen QIAamp Circulating Nucleic Acid Kit. The extracted extracellular free DNA was subjected to a bisulfite transformation treatment using a commercial bisulfite transformation reagent MethylCodeTM Bisulfite Conversion Kit to obtain a transformed DNA.

Optionally, the transformed DNA described above was used for pre-amplification, and PCR amplification was performed using a primer pool containing methylation marker-specific primers and internal reference primers as shown in Table 1 (ACTB, the forward primers and reverse primers can be as shown in Table 1, respectively, and the probes can be shown as in Table 2), and using the transformed DNA as a template, with a final concentration of 100 nM for each primer. The PCR reaction system was 10µL of transformed DNA, 2.5µL of premixed solution containing the above primers; and 12.5µL of PCR reagent (Luna^{®}Universal Probe qPCR Master Mix (NEB)). The PCR reaction conditions were as follows: 95°C for 5 minutes; 95°C for 30 seconds and 56°C for 60 seconds, and 15 cycles were performed.

The pre-amplified product was diluted 10X, and then used for fluorescence PCR detection. Primers of methylation markers as shown in Table 1 and detection probe sequences as shown in Table 2 were used, and the internal reference gene ACTB was detected at the same time as a control. The final concentration of primer was 500 nM, and the final concentration of probe was 200 nM. The PCR reaction system comprises: 10µL of pre-amplified diluted product, 2.5µL of primer and probe premixed solution containing detection sites; and 12.5µL of PCR reagent (Luna^{®}Universal Probe qPCR Master Mix (NEB)).

The fluorescence PCR reaction system was the same as the above example. The PCR reaction conditions were as follows: 95°C for 5 minutes; 95°C for 15 seconds and 56°C for 40 seconds (fluorescence was collected), and 50 cycles were performed. For the modified fluorescence of different gene probes, the corresponding detection fluorescence channels were selected. The target Ct value at which no amplification signal was detected was set as 50. The results showed that each target of the present application could have the ability to detect liver cancer in blood.

In the case of about 90% specificity, the detection sensitivity statistics of the detection sites were shown in Table 5:

**Table 1-4 Detection Sensitivity of Detection Sites**

| Site | Specificity | Sensitivity |
|---|---|---|
| BEST4 | 94.9% | 48.8% |
| GRASP | 91.8% | 40.2% |
| B4GALNT1 | 92.9% | 40.2% |
| IRF4 | 90.3% | 40.0% |
| SEPT9 (1a) | 91.8% | 40.0% |
| SEPT9 (1) | 92.3% | 40.0% |
| IKZF1 | 98.3% | 35.1% |
| BEND4 | 99.1% | 17.8% |

The results in the table above showed the comparison of the DNA methylation signals in the control plasma and the liver cancer plasma at the detection sites. It was proved that the selected target markers had relatively high sensitivity for blood samples of the patients with liver cancer.

When the targets were analyzed in combination, the data can be analyzed by setting a positive interpretation threshold for a single target, and when the targets were combined, any target was positive, that was, the sample was comprehensively interpreted as positive. In this way, it was verified that:
By combining SEPT9 (1) and IKZF1, the sensitivity of detection for liver cancer was 64.5%, and the specificity for healthy controls was as high as 95.9%, while the positive rate of hepatitis B patients was 2.7%, and the positive rate of patients with liver cirrhosis was 20.5%; by combining SEPT9 (1), IKZF1, BEST4 and B4GALNT1, the sensitivity of detection for liver cancer was 73.1%, and the specificity for healthy controls was 91.0%, while the positive rate of patients with hepatitis B was 9.1%, and the positive rate of patients with liver cirrhosis was 20.5%; by combining SEPT9 (1), SEPT9 (1a), IKZF1, BEST4, GRASP and B4GALNT1, the sensitivity of detection for liver cancer was 75.8%, and the specificity for healthy controls was 90.2%, while the positive rate of patients with hepatitis B was 16.2%, and the positive rate of patients with liver cirrhosis was 20.5%; and by combining IRF4, IKZF1, BEST4, B4GALNT1, BEND4 and SEPT9 (1), the sensitivity of detection for liver cancer was 76.9%, and the specificity for healthy controls was 91.0%, while the positive rate of patients with hepatitis B was 9.1%, and the positive rate of patients with liver cirrhosis was 20.5%; and all of the above target combinations had proved that the performance of differentiating healthy control from liver cancer was superior, and the specificity was good in patients with hepatitis B and liver cirrhosis.

### Example 2

### Comparison of methylation abundance of DNA samples from liver cancer, paracancerous tissue and leukocyte

DNA samples were obtained from the leukocytes of healthy population without abnormality of liver and the paracancerous tissues and cancerous tissues of patients with liver cancer (among them, about 10 samples from leukocytes, about 24 samples from paracancerous tissue and about 24 samples from cancerous tissues), respectively. The leukocytic DNA was selected as the reference sample because most of the plasma free DNA was derived from the DNA released after the rupture of the leukocytes, and its background could be a basic background signal of the detection site of the plasma free DNA. According to the instructions, the leukocytic DNA was extracted with Qiagen QIAamp DNA Mini Kit, and the tissue DNA was extracted with Qiagen QIAamp DNA FFPE Tissue Kit. A 20 ng sample of the DNA obtained in the above steps was treated with a bisulfite reagent (D5031, ZYMO RESEARCH) to obtain the transformed DNA.

Optionally, PCR amplification was performed using a primer pool containing a target methylation specific primer and an internal reference (ACTB) primer pair, and using the transformed DNA as a template, with a final concentration of 100 nM for each primer. The PCR reaction system comprises: 10µL of transformed DNA (a pre-mixed solution comprising 2.5µL of the above primers) and 12.5µL of PCR reagent (Luna^{®}Universal Probe qPCR Master Mix (NEB)). The PCR reaction conditions were: 95°C for 5 minutes; 95°C for 30 seconds and 56°C for 60 seconds, and 10 cycles were performed. For example, the position of a lowercase letter in the tabular sequence of the present application could correspond to the site of a natural cytosine on the region of the genome to which the sequence was bound. For example, the bases indicated by the lowercase letters of the sequences in the tables of the present application could be used to pair with the transformed bases; for example, a natural cytosine could be transformed into a uracil after bisulfite treatment, a lowercase base a could be paired with the transformed uracil, and a lowercase base t could be further paired with the adenine with which the uracil was paired. Here, the primer sequences were shown in Table 2-1.

**Table 2-1 Target specific primers of the present application**

| SEQ ID NO. | Name | Sequence |
|---|---|---|
| 34 | BCAT1 forward primer 1 | TACGTGGCGGGTTGG |
| 35 | BCAT1 reverse primer 1 | AAAAAAACAACCTTAATATCTTC |
| 37 | BCAT1 forward primer 1a | GTTTTTTTGTTGATGTAATTCGTTAGGTC |
| 38 | BCAT1 reverse primer 1a | CAATACCCGAAACGACGACG |
| 41 | BCAT1 forward primer 2 | tAGGGtAGAGGCGtTttttAtAT |
| 42 | BCAT1 reverse primer 2 | CCGaCTaCCATCCCGTCTAa |
| 45 | BCAT1 forward primer 3 | tTGGGACGAGACGGTTGGAG |
| 46 | BCAT1 reverse primer 3 | AaaaCGCTaaaTACCACGACCTa |
| 7 | IKZF 1 forward primer 1 | GTTTTTTTGGTTCGGAGTTG |
| 8 | IKZF 1 reverse primer 1 | CAAAACGAAACACGAAAAAAATA |
| 48 | IKZF 1 forward primer 1a | GACGACGTATTTTTTTCGTGTTTC |
| 49 | IKZF1 reverse primer 1a | GCGCACCTCTCGACCG |
| 52 | IKZF 1 forward primer 2 | GtTGtATTtCGGGGAGAAGtt |
| 53 | IKZF 1 reverse primer 2 | ACCTACCGaAaTaCGTCCTC |
| 56 | IKZF1 forward primer 3 | AtAGCGtCGTGGAGAAttTGt |
| 57 | IKZF1 reverse primer 3 | CTCGTTaTTaCTCTCGaTaTCCG |
| 60 | VAV3 forward primer 1 | CGGAGTCGAGTTTAG |
| 61 | VAV3 reverse primer 1 | ACCGCCGACCCTTT |
| 63 | VAV3 forward primer 1a | CGCGGGATTCGTTGTAGC |
| 64 | VAV3 reverse primer 1a | AACAAAAACCGCGACTAACGA |
| 23 | IRF4 forward primer 1 | AAAAAAAAAAAAACTCCACATTT |
| 24 | IRF4 reverse primer 1 | TAGTTGNGGAGTTTGGG |
| 66 | IRF4 forward primer 1a | TGGGTGTTTTGGACGGTTTC |
| 67 | IRF4 reverse primer 1a | CGCCTACCCTCCGCG |
| 69 | IRF4 forward primer 1b | AtAAGTGGCGtAGACGCGGG |
| 70 | IRF4 reverse primer 1b | CCTCCGCTCTCCCGaaCCTa |
| 73 | IRF4 forward primer 2 | CGACGGGtTtTATGCGAAAAG |
| 74 | IRF4 reverse primer 2 | aAaCTTaCAaaTCTaaTCTCTCTCC |
| 135 | ACTB forward primer | GTGATGGAGGAGGTTTAGTAAGTT |
| 136 | ACTB reverse primer | CCAATAAAACCTACTCCTCCCTTAA |

The obtained pre-amplification product was diluted 10X, and the detection fluorescence Ct value of the marker was obtained by multiplex fluorescence PCR detection. In the fluorescence PCR reaction system, the final concentration of each primer was 500 nM, and the final concentration of each detection probe was 200 nM. The PCR reaction system comprises: 10µL of pre-amplified diluted product, 2.5µL of primer and probe premixed solution containing detection sites; and 12.5µL of PCR reagent (Luna^{®}Universal Probe qPCR Master Mix (NEB)). The PCR reaction conditions were as follows: 95°C for 5 minutes; 95°C for 30 seconds and 56°C for 60 seconds (fluorescence was collected), and 50 cycles were performed. Different fluorescence was detected in the corresponding fluorescence channels using ABI 7500 Real-Time PCR System. Ct values of the samples obtained from the leukocyte, paracancerous tissue and cancerous tissue were calculated and compared, and the target Ct value at which no amplification signal was detected was set as 50. Here, the primer sequence was shown in Table 2-1, and the probe sequence was shown in Table 2-2.

**Table 2-2 Target detection probe sequences of the present application**

| SEQ ID NO. | Name | Sequence |
|---|---|---|
| 33 | BCAT1 probe 1 | TCGGTTTTTTCGCGGCG |
| 36 | BCAT1 probe 1a | TTCGTCGCGAGAGGGTCGGTT |
| 40 | BCAT1 probe 2 | TtAGACGATGGGCGGtCG |
| 44 | BCAT1 probe 3 | TGCGTCGTAtttTGCGttTG |
| 6 | IKZF1 probe 1 | CGCCCCGTCGCCGAAT |
| 47 | IKZF1 probe 1a | TTTGTATCGGAGTAGCGATTCGGGAGG |
| 51 | IKZF1 probe 2 | TACGttTGtCGtCGGAGGG |
| 55 | IKZF1 probe 3 | TGtttTCGGAGCGCGAGGC |
| 59 | VAV3 probe 1 | TTTCGATTTCGCGCGGGG |
| 62 | VAV3 probe 1a | CGCGGCGTTCGCGATTCGTT |
| 22 | IRF4 probe 1 | ATCGTACGTAAGGTTCGGAGCGA |
| 65 | IRF4 probe 1a | TCGTTTAGTTTGTGGCGATTTCGTCG |
| 68 | IRF4 probe 1b | TACGGGGGATTtCGCGCGtA |
| 72 | IRF4 probe 2 | TGGCGtTGTGtAACGAtCGGt |
| 134 | ACTB probe | ACCACCACCCAACACACAATAACAAACACA |

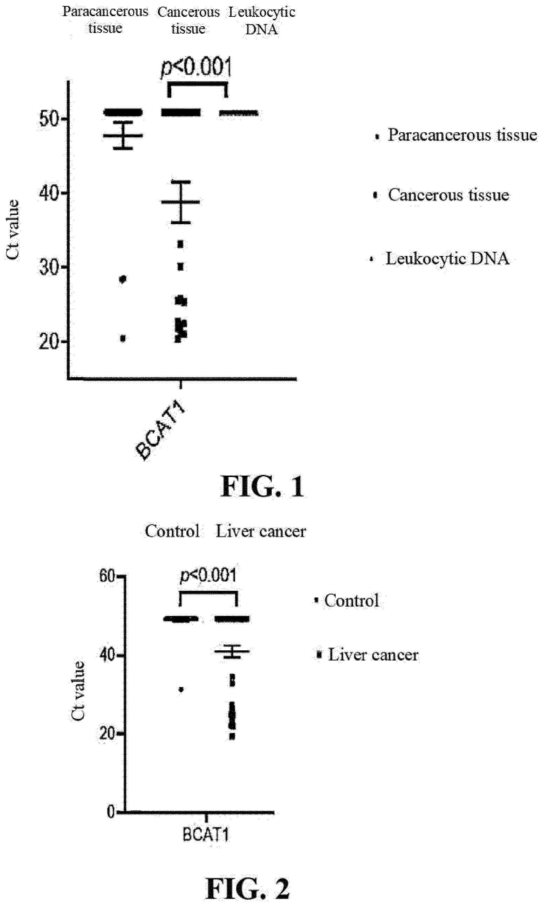

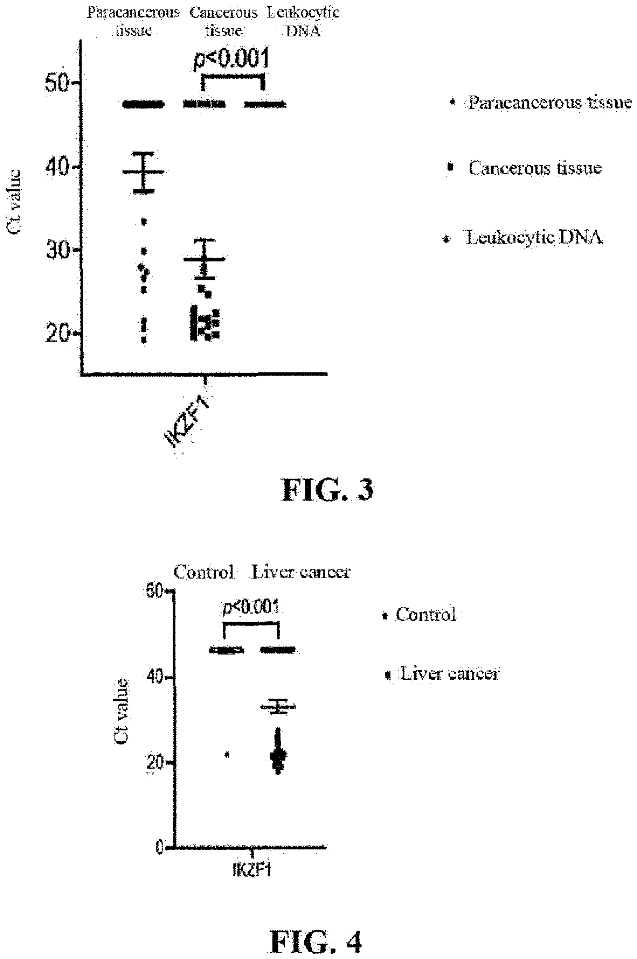

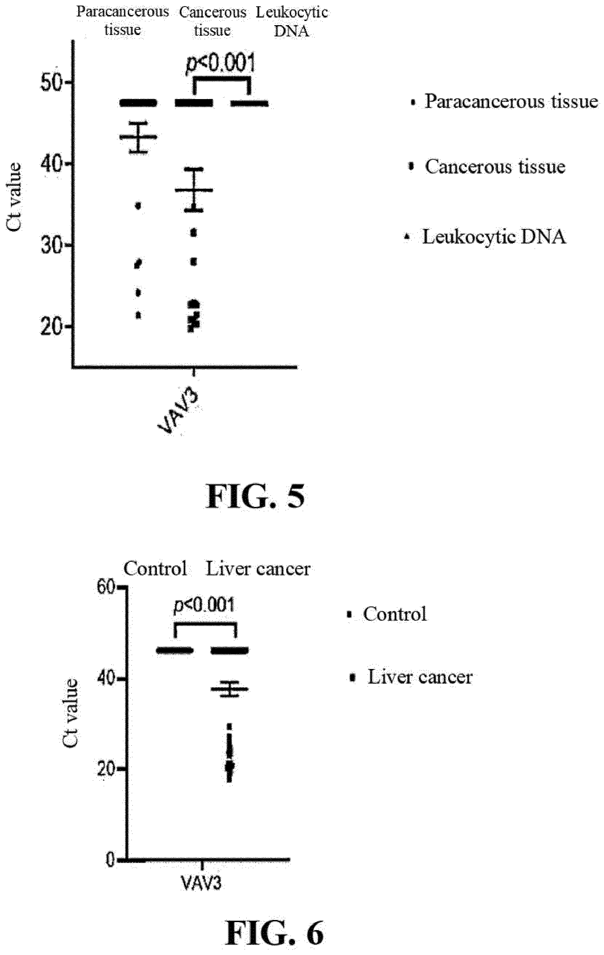

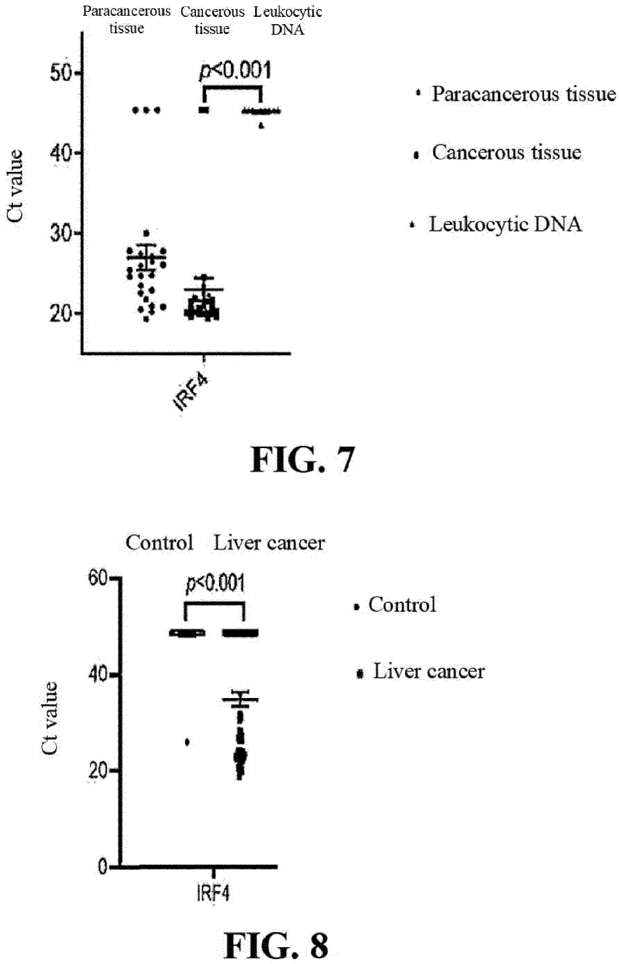

The results were shown in Figs 1, 3, 5 and 7. It could be seen that the background signals of methylation at these detection sites in leukocytic DNA were very low, the signal of tissue was stronger than that of leukocytic DNA, and the signal of cancerous tissue was stronger than that of paracancerous tissue. It was proved that the selected target markers were feasible and specific for tumor tissue.

### Comparison of methylation signals in plasma samples from patients with liver cancer and population without abnormality of liver

The plasma samples from about 65 individuals with liver cancer and the plasma samples from about 76 control individuals were tested using fluorescence PCR assay:
The extracellular free DNA in the above plasma samples was extracted using commercial Qiagen QIAamp Circulating Nucleic Acid Kit. The extracted extracellular free DNA was subjected to a bisulfite transformation treatment using a commercial bisulfite transformation reagent MethylCodeTM Bisulfite Conversion Kit to obtain a transformed DNA.

Optionally, the transformed DNA was used for pre-amplification, and PCR amplification was performed using a primer pool containing a target methylation specific primer and an internal reference (ACTB) primer pair shown in Table 2-1, and using the transformed DNA as a template, with a final concentration of 100 nM for each primer. The PCR reaction system was 10µL of transformed DNA, 2.5µL of premixed solution containing the above primers; and 12.5µL of PCR reagent (Luna^{®}Universal Probe qPCR Master Mix (NEB)). The PCR reaction conditions were as follows: 95°C for 5 minutes; 95°C for 30 seconds and 56°C for 60 seconds, and 15 cycles were performed.

The pre-amplified product was diluted 10X, and then used for fluorescence PCR detection. Primers as shown in Table 2-1 and detection probe sequences as shown in Table 2-2 were used, and the internal reference gene ACTB was detected at the same time as a control. The final concentration of primer was 500 nM, and the final concentration of probe was 200 nM. The PCR reaction system comprises: 10 µL of pre-amplified diluted product, 2.5 µL of primer and probe premixed solution containing detection sites; and 12.5 µL of PCR reagent (Luna^{®}Universal Probe qPCR Master Mix (NEB)).

The fluorescence PCR reaction system was the same as this example. The PCR reaction conditions were as follows: 95°C for 5 minutes; 95°C for 15 seconds and 56°C for 40 seconds (fluorescence was collected), and 50 cycles were performed. For the modified fluorescence of different gene probes, the corresponding detection fluorescence channels were selected. The target Ct value at which no amplification signal was detected was set as 50.

The results were shown in Figs 2, 4, 6 and 8, showing the comparison of DNA methylation signals in control plasma and liver cancer plasma at the detection site. It was proved that the selected target markers had high sensitivity to tumor tissues.

In the case of greater than 90% specificity, the detection sensitivity statistics of the detection sites were shown in the table below.

**Table 2-3 Detection sensitivity of detection sites**

| Site | Sensitivity |
|---|---|
| BCAT1 | 34% |
| IKZF1 | 55% |
| VAV3 | 35% |
| IRF4 | 51% |

Considering a group of 5 markers (Septin9, IKZF1, IRF4, VAV3 and BCAT1) comprehensively, when the specificity of control group was 90.8%, the detection sensitivity of liver cancer could achieve 90.8%, and the specific information was shown in the table below:

**Table 2-4 Detection accuracy for detecting multiple sites**

| | Number of patients | Detection results | | Accuracy |
|---|---|---|---|---|
| | | Positive | Negative | |
| Liver cancer | 65 | 59 | 6 | 90.8% |
| Control | 76 | 7 | 69 | 90.8% |

### Comparison of methylation abundance of DNA samples from liver cancer, paracancerous tissue and buffy coat

DNA samples were obtained from the buffy coats of healthy population without abnormality of liver and the cancerous tissues of patients with liver cancer (among them, about 10 samples from buffy coats, and about 10 samples from cancerous tissues). The buffy coat DNA was selected as the reference sample because most of the plasma free DNA was derived from the DNA released after the rupture of the buffy coat, and its background could be a basic background signal of the detection site of the plasma free DNA. According to the instructions, the buffy coat DNA was extracted with Qiagen QIAamp DNA Mini Kit, and the tissue DNA was extracted with Qiagen QIAamp DNA FFPE Tissue Kit. A 20 ng sample of the DNA obtained in the above steps was treated with a bisulfite reagent (D5031, ZYMO RESEARCH) to obtain the transformed DNA.

Optionally, PCR amplification was performed using a primer pool containing a target methylation specific primer and an internal reference (ACTB) primer pair, and using the transformed DNA as a template, with a final concentration of 100 nM for each primer. The PCR reaction system comprises: 10µL of transformed DNA (a pre-mixed solution comprising 2.5µL of the above primers) and 12.5µL of PCR reagent (Luna^{®}Universal Probe qPCR Master Mix (NEB)). The PCR reaction conditions were: 95°C for 5 minutes; 95°C for 30 seconds and 56°C for 60 seconds, and 10 cycles were performed. For example, the position of a lowercase letter in the tabular sequence of the present application could correspond to the site of a natural cytosine on the region of the genome to which the sequence was bound. For example, the bases indicated by the lowercase letters of the sequences in the tables of the present application could be used to pair with the transformed bases; for example, a natural cytosine could be transformed into a uracil after bisulfite treatment, a lowercase base a could be paired with the transformed uracil, and a lowercase base t could be further paired with the adenine with which the uracil was paired. Here, the primer sequences were shown in Table 2-5.

The obtained pre-amplification product was diluted 10X, and the detection fluorescence Ct value of the marker was obtained by multiplex fluorescence PCR detection. In the fluorescence PCR reaction system, the final concentration of each primer was 500 nM, and the final concentration of each detection probe was 200 nM. The PCR reaction system comprises: 10µL of pre-amplified diluted product, 2.5µL of primer and probe premixed solution containing detection sites; and 12.5µL of PCR reagent (Luna^{®}Universal Probe qPCR Master Mix (NEB)). The PCR reaction conditions were as follows: 95°C for 5 minutes; 95°C for 30 seconds and 56°C for 60 seconds (fluorescence was collected), and 50 cycles were performed. Different fluorescence was detected in the corresponding fluorescence channels using ABI 7500 Real-Time PCR System. Ct values of the samples obtained from the buffy coat, paracancerous tissue and cancerous tissue were calculated and compared, and the target Ct value at which no amplification signal was detected was set as 50. Here, the primer sequence was shown in Table 2-5, and the probe sequence was shown in Table 2-6. Gene spacer 2 indicated human chr1:145384249:145413094 region, and gene spacer 1 indicated human chr7:26415938:26416740 region.

**Table 2-5 Primer Sequences**

| SEQ ID NO. | Name | Sequence |
|---|---|---|
| 15 | B4GALNT1 forward primer 1 | AGtAGtTGtCgATAAGTGGT |
| 16 | B4GALNT1 reverse primer 1 | GCCTaaAaaCAACCTCCCT |
| 76 | B4GALNT1 forward primer 1a | GGTttAGCGGCGTtCGtTttAG |
| 77 | B4GALNT1 reverse primer 1a | aaaTaaACGACGACTTCGTCTTC |
| 80 | B4GALNT1 forward primer 2 | GAATtATGttCGTGtTGGCGGG |
| 81 | B4GALNT1 reverse primer 2 | GGAtAGAGTGGAtCGGGAAGCG |
| 19 | GRASP forward primer 1 | GttttTtTtCgAtTtttTAtAGGG |
| 20 | GRASP reverse primer 1 | aaCcgaaaAAaAAaaTaaaaaACTC |
| 83 | GRASP forward primer 1a | GtTGTACGCGGCGtTGGAGG |
| 84 | GRASP reverse primer 1a | ACGCACCTTTCGaCGaaaCA |
| 87 | GRASP forward primer 2 | TGCGtAGGttTGGTGGTGAAG |
| 88 | GRASP reverse primer 2 | CAaCGAaCCCGaaAaCAaaCCC |
| 11 | BEST4 forward primer 1 | TGTGGGtCgGAtttttAGAG |
| 12 | BEST4 reverse primer 1 | TaCTTAAacGCTTCCCCAC |
| 90 | BEST4 forward primer 1a | GAtCGAGCGtAGtAttAGtACGG |
| 91 | BEST4 reverse primer 1a | TaCACGaCGTaaACCAaCGaa |
| 94 | BEST4 forward primer 2 | GGttTCGTtttCGGAtTtCGt |
| 95 | BEST4 reverse primer 2 | TCTaCTaCGCTTCCGaaCGaA |
| 27 | BEND4 forward primer 1 | AGTttTtAAGTGGtttTGGGAT |
| 28 | BEND4 reverse primer 1 | CCAaacgCAaAaCTCCTAC |
| 97 | BEND4 forward primer 1a | TtTCGAAGTTTtCGGGTGCG |
| 98 | BEND4 reverse primer 1a | CGaaaaaCCGCCGACACTTA |
| 101 | Gene spacer 2 forward primer 1 | TTAtTtTTTtttTGAtCgGGAAT |
| 102 | Gene spacer 2 reverse primer 1 | ATAaCTaCAACcGaaAAaaaTTA |
| 104 | Gene spacer 2 forward primer 1a | tTGtCGTGAtTCGGATTCGAA |
| 105 | Gene spacer 2 reverse primer 1a | CAaCTaTAaCGCGCCGCCTa |
| 108 | Gene spacer 2 forward primer 2 | ACGCGGTGAGGAtAttACGG |
| 109 | Gene spacer 2 reverse primer 2 | GttCGGCGGAAATGAtTGTGAG |
| 112 | Gene spacer 2 forward primer 3 | GAtAAGAGAtCGGGCGCGGT |
| 113 | Gene spacer 2 reverse primer 3 | CTCGATCTCCTaACCTCGTaATCC |
| 115 | Gene spacer 1 forward primer 1 | GtttCgttAGGtTGGAGAG |
| 116 | Gene spacer 1 reverse primer 1 | CTaCAaAACAAAaCCTaaAaTCC |
| 118 | Gene spacer 1 forward primer 1a | CGAtCGtAAAGAGAtAGCGATTT |
| 119 | Gene spacer 1 reverse primer 1a | AATCGTCACCGTAaCGCGaa |
| 122 | GPAM forward primer 1 | gGttTGcgAGttTtAGGG |
| 123 | GPAM reverse primer 1 | CAaacgCTCACACACTATC |
| 125 | GPAM forward primer 1a | GtTGtAGTGGCGtAtttTGATGAC |
| 126 | GPAM reverse primer 1a | CGaCGCTaaCTAaCCGaCGa |
| 129 | VASH2 forward primer 1 | TtAGCgTGtAGGGGGGA |
| 130 | VASH2 reverse primer 1 | cGaAaACACAaCTaACACCA |
| 132 | VASH2 forward primer 1a | ttCGtCGttCGGAGAGGtTt |
| 133 | VASH2 reverse primer 1a | TTCCAaCGCCTATCACCGaaA |
| 135 | ACTB forward primer | GTGATGGAGGAGGTTTAGTAAGTT |
| 136 | ACTB reverse primer | CCAATAAAACCTACTCCTCCCTTAA |

**Table 2-6 Detection probe sequences**

| SEQ ID NO. | Name | Sequence |
|---|---|---|
| 14 | B4GALNT1 probe 1 | AGATTTCGCGTATCGCGTTTT |
| 75 | B4GALNT1 probe 1a | ttAGtCGCGTtCGCGtCGT |
| 79 | B4GALNT1 probe 2 | CGATGAGTGGttTCGGtAAGCG |
| 18 | GRASP probe 1 | TGTTTTTTTTTCGGCGTTCGCG |
| 82 | GRASP probe 1a | TAtCGCGCGtTCGtCGTGTt |
| 86 | GRASP probe 2 | ACGAtACGtTGGAGTCGGTGC |
| 10 | BEST4 probe 1 | CGGCGTATTTGCGTTTATTACGT |
| 89 | BEST4 probe 1a | tAGGTTCGCGTAGCGGATGA |
| 93 | BEST4 probe 2 | TCGATGCGGGtTGCGGttT |
| 26 | BEND4 probe 1 | TAGGACGGCGACGACGA |
| 96 | BEND4 probe 1a | TGCGtttCGAtATttCGAtACG |
| 100 | Gene spacer 2 probe 1 | CGGTGAGAGCGTCGAATTTTA |
| 103 | Gene spacer 2 probe 1a | CGAGGTTGtTGCGGttAtAACG |
| 107 | Gene spacer 2 probe 2 | ttACGCGCGCGTtCGtTAG |
| 111 | Gene spacer 2 probe 3 | TTGGGAGGtCGAGGCGGGC |
| 114 | Gene spacer 1 probe 1 | TTGAGGGGCGGTCGC |
| 117 | Gene spacer 1 probe 1a | CGGGttTGCGGtTttCGGtC |
| 121 | GPAM probe 1 | CGTGAGCGGATTCGAGGGT |
| 124 | GPAM probe 1a | AtCGGCGCGTGGtATTGAC |
| 128 | VASH2 probe 1 | TTCGGTTTCGTTGTGCGT |
| 131 | VASH2 probe 1a | TGtCGCGCGtttTTttTGCG |
| 134 | ACTB probe | ACCACCACCCAACACACAATAACAAACACA |

**Table 2-7 Summary of detection results for sample**

| | Average Ct of cancerous tissues | Average Ct of buffy coats | Ratio of detected cancerous tissues | Ratio of detected buffy coats | p value |
|---|---|---|---|---|---|
| B4GALNT1 | 19.258 | 50.000 | 100% | 0% | 1.73E-27 |
| GRASP | 25.649 | 50.000 | 80% | 0% | 6.00E-08 |
| BEST4 | 24.498 | 50.000 | 80% | 0% | 1.29E-08 |
| BEND4 | 21.333 | 50.000 | 90% | 0% | 1.64E-11 |
| Gene spacer 2 | 28.479 | 50.000 | 100% | 0% | 1.28E-05 |
| Gene spacer 1 | 33.845 | 50.000 | 50% | 0% | 2.49E-03 |
| GPAM | 23.310 | 50.000 | 100% | 0% | 9.83E-06 |
| VASH2 | 32.603 | 50.000 | 60% | 0% | 8.14E-04 |

The results showed that the detection rate of methylation signal in cancerous tissue could be much higher than that in buffy coat, also indicating a strong methylation signal. Target methylation signal could not be detected in most samples of buffy coat. These targets might all have the potential for detection of liver cancer in blood. It was proved that the selected target markers were feasible and specific for tumor tissue.

### Comparison of methylation signals in plasma samples from patients with liver cancer and population without abnormality of liver

About 170 healthy control plasma samples without abnormality of liver, about 321 preoperative plasma samples from patients with liver cancer (among which stage I accounted for 86%), about 36 plasma samples from patients with hepatitis B and 20 plasma samples from patients with liver cirrhosis were selected for detection: the extracellular free DNA in the above plasma samples was extracted using a commercial Qiagen QIAamp Circulating Nucleic Acid Kit. The extracted extracellular free DNA was subjected to a bisulfite transformation treatment using a commercial bisulfite transformation reagent MethylCodeTM Bisulfite Conversion Kit to obtain a transformed DNA.

Optionally, the transformed DNA was used for pre-amplification, and PCR amplification was performed using a primer pool containing a target methylation specific primer and an internal reference (ACTB) primer pair shown in Table 2-5, and using the transformed DNA as a template, with a final concentration of 100 nM for each primer. The PCR reaction system was 10µL of transformed DNA, 2.5µL of premixed solution containing the above primers; and 12.5µL of PCR reagent (Luna^{®}Universal Probe qPCR Master Mix (NEB)). The PCR reaction conditions were as follows: 95°C for 5 minutes; 95°C for 30 seconds and 56°C for 60 seconds, and 15 cycles were performed.

The pre-amplified product was diluted 10X, and then used for fluorescence PCR detection. Primers as shown in Table 2-5 and detection probe sequences as shown in Table 2-6 were used, and the internal reference gene ACTB was detected at the same time as a control. The final concentration of primer was 500 nM, and the final concentration of probe was 200 nM. The PCR reaction system comprises: 10µL of pre-amplified diluted product, 2.5µL of primer and probe premixed solution containing detection sites; and 12.5µL of PCR reagent (Luna^{®}Universal Probe qPCR Master Mix (NEB)).

The fluorescence PCR reaction system was the same as this example. The PCR reaction conditions were as follows: 95°C for 5 minutes; 95°C for 15 seconds and 56°C for 40 seconds (fluorescence was collected), and 50 cycles were performed. For the modified fluorescence of different gene probes, the corresponding detection fluorescence channels were selected. The target Ct value at which no amplification signal was detected was set as 50.

**Table 2-8 Summary of detection results for sample**

| | AUC | p value |
|---|---|---|
| B4GALNT1 | 0.743 | 7.12E-19 |
| GRASP | 0.748 | 1.67E-19 |
| BEST4 | 0.787 | 1.05E-25 |
| BEND4 | 0.586 | 1.64E-03 |
| Gene spacer 2 | 0.777 | 4.97E-24 |
| Gene spacer 1 | 0.597 | 4.14E-04 |
| GPAM | 0.755 | 1.24E-20 |
| VASH2 | 0.592 | 8.00E-04 |

The results in the above tables showed the AUC statistics of the markers between the liver cancer group and the healthy control group, and all of the targets in the present application could be used for detection of liver cancer in blood.

In the case of greater than 90% specificity, the detection sensitivity statistics of the detection sites were shown in the table below.

**Table 2-9 Detection sensitivity of detection sites**

| Site | Sensitivity | Specificity |
|---|---|---|
| B4GALNT1 | 52.02% | 93.69% |
| GRASP | 53.89% | 93.2% |
| BEST4 | 57.01% | 89.81% |
| BEND4 | 17.76% | 99.51% |
| Gene spacer 2 | 54.21% | 86.41% |
| Gene spacer 1 | 19.94% | 99.03% |
| GPAM | 56.7% | 89.81% |
| VASH2 | 18.38% | 100% |

The results showed the comparison of the DNA methylation signals in the control plasma and the liver cancer plasma at the detection sites. It was proved that the selected target markers had relatively high sensitivity for blood samples of the patients with liver cancer.

### Example 3

### The sample detection ability of each sub-region of methylation markers in the present application

Referring to the above examples, the detection of liver cancer samples was performed for each sub-region of methylation markers in the present application. The detection results were shown in the table below:

**Table 3 Summary of detection results for sample**

| Inspection area number | Average Ct of cancerous tissues | Average Ct of leukocytes | Level classification of target detection effects | Fold difference in signal of cancerous tissues relative to leukocytes | p value |
|---|---|---|---|---|---|
| BCAT1_1 | 38.27 | 50.00 | A | 3401.61 | 9.30E-03 |
| BCAT1_1a | 25.87 | 50.00 | A | 18402132.85 | 5.00E-14 |
| BCAT1_2 | 13.85 | 15.88 | C | 4.09 | 6.06E-01 |
| BCAT1_3 | 26.11 | 30.08 | B | 15.66 | 6.80E-03 |
| IKZF1_1 | 29.98 | 50.00 | A | 1061004.98 | 1.19E-05 |
| IKZF1_1a | 35.03 | 48.98 | A | 15798.59 | 4.02E-03 |
| IKZF1_2 | 25.00 | 22.03 | C | 0.13 | 5.81E-06 |
| IKZF1_3 | 24.59 | 21.65 | C | 0.13 | 1.82E-05 |
| IRF4_1 | 24.33 | 48.80 | A | 23270518.17 | 2.20E-11 |
| IRF4_1a | 29.15 | 40.16 | A | 2063.11 | 2.84E-03 |
| IRF4_1b | 25.08 | 46.23 | A | 2315942.53 | 4.01E-07 |
| IRF4_2 | 24.51 | 21.70 | C | 0.14 | 1.22E-04 |
| VAV3_1 | 38.51 | 50.00 | A | 2876.30 | 1.05E-02 |
| VAV3_1a | 41.58 | 50.00 | A | 342.47 | 7.77E-03 |
| SEPT9_1 | 26.40 | 45.12 | A | 433297.98 | 9.55E-09 |
| SEPT9_1a | 23.64 | 50.00 | A | 86368386.37 | 2.37E-11 |
| SEPT9_2 | 27.15 | 35.90 | A | 429.73 | 1.94E-02 |
| B4GALNT1_1 | 19.26 | 50.00 | A | 1795825469.51 | 1.73E-27 |
| B4GALNT1_1a | 28.54 | 35.74 | A | 147.34 | 8.62E-05 |
| B4GALNT1_2 | 26.59 | 28.59 | C | 4.00 | 3.34E-01 |
| GRASP_1 | 25.65 | 50.00 | A | 21398406.06 | 6.00E-08 |
| GRASP_1a | 28.12 | 49.14 | A | 2124444.06 | 6.60E-07 |
| GRASP_2 | 22.03 | 20.83 | C | 0.44 | 1.04E-04 |
| BEST4_1 | 24.50 | 50.00 | A | 47518962.44 | 1.29E-08 |
| BEST4_1a | 32.66 | 46.37 | A | 13400.51 | 8.85E-03 |
| BEST4_1b | 30.72 | 50.00 | A | 636914.61 | 1.59E-05 |
| BEST4_2 | 40.55 | 50.00 | A | 700.31 | 3.58E-02 |
| BEND4_1 | 21.33 | 50.00 | A | 426213190.02 | 1.64E-11 |
| BEND4_1a | 27.33 | 45.22 | A | 243047.73 | 6.96E-05 |
| Gene spacer 2_1 | 28.48 | 50.00 | A | 3009307.29 | 1.28E-05 |
| Gene spacer 2_1a | 36.29 | 48.47 | A | 4644.05 | 3.23E-03 |
| Gene spacer 2_2 | 50.00 | 45.92 | C | 0.06 | 3.31E-01 |
| Gene spacer 2_3 | 28.56 | 26.99 | C | 0.34 | 3.84E-01 |
| Gene spacer 1_1 | 33.85 | 50.00 | A | 72969.20 | 2.49E-03 |
| Gene spacer 1_1a | 28.96 | 48.37 | A | 695664.30 | 1.73E-05 |
| GPAM_1 | 23.31 | 50.00 | A | 108265624.23 | 9.83E-06 |
| GPAM_1a | 20.97 | 48.73 | A | 227532373.94 | 6.04E-09 |
| VASH2_1 | 32.60 | 50.00 | A | 172591.27 | 8.14E-04 |
| VASH2_1a | 25.47 | 42.71 | A | 155264.18 | 6.50E-04 |
| DAB2IP_1 | 23.10 | 50.00 | A | 124969431.27 | 1.72E-09 |
| DAB2IP_1a | 25.66 | 42.14 | A | 91499.65 | 9.84E-10 |
| DAB2IP_2 | 24.04 | 26.92 | C | 7.35 | 3.02E-03 |
| CHFR_1 | 18.96 | 50.00 | A | 2213987606.22 | 2.76E-27 |
| CHFR_1a | 25.36 | 35.93 | A | 1517.90 | 6.29E-03 |
| CHFR_2 | 47.81 | 50.00 | C | 4.55 | 3.31E-01 |
| CHFR_3 | 36.64 | 37.53 | C | 1.85 | 8.32E-01 |
| CHFR_4 | 38.36 | 35.34 | C | 0.12 | 4.76E-01 |

Here, the number after the underline in each detection region number was the sub-region number corresponding to the detection region, and if the same number after the underline had different letters, it indicated different specific detection regions in the same sub-region. The primer pairs and probes used in each detection region were shown in Tables 2-1, 2-5 as well as 2-2 and 2-6, and the number after underline in each detection region corresponded to the number at the end of the primer pair and probe name in the corresponding table used.

The Ct values of methylation signals of cancerous tissues compared with those of leukocytes reflected the exponential difference of target detection effects. The criteria for grading the detection effects of specific regions of each target were as follows: A indicated that the methylation signal of cancerous tissue was 100 times greater compared with that of leukocyte, which was the preferred site for liver cancer detection; B indicated that the methylation signal of cancerous tissue was 10 times to 100 times compared with that of leukocyte, which might be the sites for liver cancer detection; and C indicated that the methylation signal of cancerous tissue was 10 times less compared with that of leukocyte, and it was highly probable that it could not be used for detection of liver cancer.

### Example 4

DNA samples were obtained from the buffy coats of healthy population without abnormality of liver and the cancerous tissues of patients with liver cancer (among them, 10 samples from buffy coats, and 10 samples from cancerous tissues). The buffy coat DNA was selected as the reference sample because most of the plasma free DNA was derived from the DNA released after the rupture of the buffy coat, and its background could be a basic background signal of the detection site of the plasma free DNA. According to the instructions, the buffy coat DNA was extracted with Qiagen QIAamp DNA Mini Kit, and the tissue DNA was extracted with Qiagen QIAamp DNA FFPE Tissue Kit.

A 20 ng sample of the DNA obtained in the above steps was treated with a bisulfite reagent (D5031, ZYMO RESEARCH) to obtain the transformed DNA.

The detection fluorescence Ct value of the marker was obtained by fluorescence PCR detection. In the fluorescence PCR reaction system, the final concentration of each primer was 500 nM, and the final concentration of each detection probe was 200 nM. The PCR reaction system comprises: 10µL of pre-amplified diluted product, 2.5µL of primer and probe premixed solution containing detection sites; and 12.5µL of PCR reagent (Luna^{®}Universal Probe qPCR Master Mix (NEB)). Here, the primer sequence of each methylation marker was shown in Table 4-1, and the probe sequence was shown in Table 4-2. The PCR reaction conditions were as follows: 95°C for 5 minutes; 95°C for 30 seconds and 56°C for 60 seconds (fluorescence was collected), and 50 cycles were performed. Different fluorescence was detected in the corresponding fluorescence channels using ABI 7500 Real-Time PCR System. Ct values of the samples obtained from the buffy coat, paracancerous tissue and cancerous tissue were calculated and compared, and the target Ct value at which no amplification signal was detected was set as 50.

**Table 4-1 Primer Sequences**

| SEQ ID NO. | Name | Sequence |
|---|---|---|
| 138 | SDC2 forward primer | GTAGAAATTAATAAGTGAGAGGGC |
| 139 | SDC2 reverse primer | AACGACTCAAACTCGAAAACTCG |

**Table 4-2 Detection Probe Sequences**

| SEQ ID NO. | Name | Sequence |
|---|---|---|
| 137 | SDC2 probe | TTCGGGGCGTAGTTGCGGGCGG |

**Table 4-3 Summary of detection results for sample**

| Site | Average Ct of cancerous tissues | Average Ct of buffy coats | Ratio of detected cancerous tissues | Ratio of detected buffy coats | p value |
|---|---|---|---|---|---|
| SDC2 | 37.76 | 32.83 | 60% | 90% | 0.258 |

The results in the above table showed that the average Ct value detected in liver cancer tissue was greater compared with that in buffy coat, indicating that the methylation signal of SDC2 in liver cancer tissue sample was weaker than that in buffy coat. For example, not all methylation targets known in the art could be used for detection of blood samples for liver cancer.

The foregoing detailed description is provided by way of explanation and example, and is not intended to limit the scope of the appended claims. Various modifications to the embodiments described herein will be apparent to those of ordinary skill in the art and remain within the scope of the appended claims and their equivalents.

## Claims

1. A method for identifying the presence of a liver tumor, assessing the development or development risk of the liver tumor, and/or assessing the progress of the liver tumor, comprising determining the presence of and/or the content of modification status of a DNA region in which a target gene is located or a fragment thereof in a sample to be tested, wherein the target gene comprises SEPT9 and IKZF1.

2. A method for assessing the methylation status of a liver tumor-related DNA region, comprising determining the presence and/or the content of modification status of a DNA region in which a target gene is located or a fragment thereof in a sample to be tested, wherein the target gene comprises SEPT9 and IKZF1.

3. The method of any one of claims 1-2, wherein a DNA region of the SEPT9 is derived from human chr17:75276651-75496678.

4. The method of any one of claims 1-3, wherein a DNA region of the IKZF1 is derived from human chr7:50343720-50472799.

5. The method of any one of claims 1-4, wherein the target gene further comprises a gene selected from the group consisting of BEST4, B4GALNT1, GRASP, IRF4 and BEND4.

6. The method of any one of claims 1-5, wherein the target gene comprises at least 2 genes.

7. The method of any one of claims 1-6, wherein the target gene comprises 2 to 7 genes.

8. The method of any one of claims 1-7, wherein the target gene comprises SEPT9, IKZF1, BEST4 and B4GALNT1.

9. The method of any one of claims 1-8, wherein the target gene comprises SEPT9, IKZF1, BEST4, GRASP and B4GALNT1.

10. The method of any one of claims 1-9, wherein the target gene comprises SEPT9, IKZF1, BEST4, IRF4, B4GALNT1 and BEND4.

11. The method of any one of claims 5-10, wherein a DNA region of the BEST4 is derived from human chr1:45249257-45253377.

12. The method of any one of claims 5-11, wherein a DNA region of the B4GALNT1 is derived from human chr12:58017193-58027138.

13. The method of any one of claims 5-12, wherein a DNA region of the GRASP is derived from human chr12:52400724-52409673.

14. The method of any one of claims 5-13, wherein a DNA region of the IRF4 is derived from human chr6:391739-411447.

15. The method of any one of claims 5-14, wherein a DNA region of the BEND4 is derived from human chr4:42112955-42154895.

16. A method for identifying the presence of a disease, assessing the development or development risk of the disease and/or assessing the progress of the disease, comprising determining the presence and/or the content of modification status of a target DNA region, or a complementary region thereof, or a fragment thereabove in a sample to be tested, wherein the target DNA region comprises regions defined from human chr17:75368651-75370720 and from human chr7:50343720-50344547.

17. A method for determining a methylation status of a DNA region, comprising determining the presence and/or the content of modification status of a target DNA region, or a complementary region thereof, or a fragment thereabove in a sample to be tested, wherein the target DNA region comprises regions defined from human chr17:75368651-75370720 and from human chr7:50343720-50344547.

18. The method of any one of claims 1-17, comprising providing a nucleic acid capable of binding to a DNA region comprising SEQ ID NO: 1, or a complementary region thereof, or a transformed region thereabove, or a fragment thereabove.

19. The method of any one of claims 16-18, wherein the target region comprises a region defined from human chr17:75369558-75369622.

20. The method of any one of claims 1-19, comprising providing a nucleic acid as set forth in SEQ ID NO: 2, or a complementary nucleic acid thereof, or a fragment thereabove.

21. The method of any one of claims 1-20, comprising providing a nucleic acid set as set forth in SEQ ID NOs: 3 and 4, or a complementary nucleic acid set thereof, or a fragment thereabove.

22. The method of any one of claims 1-21, comprising providing a nucleic acid capable of binding to a DNA region comprising SEQ ID NO: 5, or a complementary region thereof, or a transformed region thereabove, or a fragment thereabove.

23. The method of any one of claims 16-22, wherein the target region comprises a region defined from human chr7:50343793-50343896.

24. The method of any one of claims 1-23, comprising providing a nucleic acid as set forth in SEQ ID NO: 6, or a complementary nucleic acid thereof, or a fragment thereabove.

25. The method of any one of claims 1-24, comprising providing a nucleic acid set as set forth in SEQ ID NOs: 7 and 8, or a complementary nucleic acid set thereof, or a fragment thereabove.

26. The method of any one of claims 16-25, wherein the target DNA region further comprises a region selected from the group consisting of the regions defined from human chr1:45251728-45252477, from human chr12:58020498-58022962, from human chr12:52400724-52401698, from human chr6:391739-394056, and from human chr4:42152705-42154895.

27. The method of any one of claims 16-26, wherein the target DNA region comprises at least 2 regions.

28. The method of any one of claims 16-27, wherein the target DNA region comprises 2 to 8 regions.

29. The method of any one of claims 16-28, wherein the target DNA region comprises a region defined from the group consisting of human chr17:75368651-75370720, human chr7:50343720-50344547, human chr1:45251728-45252477, and human chr12:58020498-58022962.

30. The method of any one of claims 16-29, wherein the target DNA region comprises a region defined from the group consisting of human chr17:75368651-75370720, human chr7:50343720-50344547, human chr1:45251728-45252477, human chr12:52400724-52401698, and human chr12:58020498-58022962.

31. The method of any one of claims 16-30, wherein the target DNA region comprises a region defined from the group consisting of human chr6:391739-394056, human chr7:50343720-50344547, human chr1:45251728-45252477, human chr12:58020498-58022962, human chr4:42152705-42154895, and human chr17:75368651-75370720.

32. The method of any one of claims 1-31, comprising providing a nucleic acid capable of binding to a DNA region comprising SEQ ID NO: 9, or a complementary region thereof, or a transformed region thereabove, or a fragment thereabove.

33. The method of any one of claims 16-32, wherein the target region comprises a region defined from human chrl :45252095-45252176.

34. The method of any one of claims 1-33, comprising providing a nucleic acid as set forth in SEQ ID NO: 10, or a complementary nucleic acid thereof, or a fragment thereabove.

35. The method of any one of claims 1-34, comprising providing a nucleic acid set as set forth in SEQ ID NOs: 11 and 12, or a complementary nucleic acid set thereof, or a fragment thereabove.

36. The method of any one of claims 1-35, comprising providing a nucleic acid capable of binding to a DNA region comprising SEQ ID NO: 13, or a complementary region thereof, or a transformed region thereabove, or a fragment thereabove.

37. The method of any one of claims 16-36, wherein the target region comprises a region defined from human chr12:58021586-58021670.

38. The method of any one of claims 1-37, comprising providing a nucleic acid as set forth in SEQ ID NO: 14, or a complementary nucleic acid thereof, or a fragment thereabove.

39. The method of any one of claims 1-38, comprising providing a nucleic acid set as set forth in SEQ ID NOs: 15 and 16, or a complementary nucleic acid set thereof, or a fragment thereabove.

40. The method of any one of claims 1-39, comprising providing a nucleic acid capable of binding to a DNA region comprising SEQ ID NO: 17, or a complementary region thereof, or a transformed region thereabove, or a fragment thereabove.

41. The method of any one of claims 16-40, wherein the target region comprises a region defined from human chr12:52401083-52401169.

42. The method of any one of claims 1-41, comprising providing a nucleic acid as set forth in SEQ ID NO: 18, or a complementary nucleic acid thereof, or a fragment thereabove.

43. The method of any one of claims 1-42, comprising providing a nucleic acid set as set forth in SEQ ID NOs: 19 and 20, or a complementary nucleic acid set thereof, or a fragment thereabove.

44. The method of any one of claims 1-43, comprising providing a nucleic acid capable of binding to a DNA region comprising SEQ ID NO: 21, or a complementary region thereof, or a transformed region thereabove, or a fragment thereabove.

45. The method of any one of claims 16-44, wherein the target region comprises a region defined from human chr6:392282-392377.

46. The method of any one of claims 1-45, comprising providing a nucleic acid as set forth in SEQ ID NO: 22, or a complementary nucleic acid thereof, or a fragment thereabove.

47. The method of any one of claims 1-46, comprising providing a nucleic acid set as set forth in SEQ ID NOs: 23 and 24, or a complementary nucleic acid set thereof, or a fragment thereabove.

48. The method of any one of claims 1-47, comprising providing a nucleic acid capable of binding to a DNA region comprising SEQ ID NO: 25, or a complementary region thereof, or a transformed region thereabove, or a fragment thereabove.

49. The method of any one of claims 16-48, wherein the target region comprises a region defined from human chr4:42153816-42153921.

50. The method of any one of claims 1-49, comprising providing a nucleic acid as set forth in SEQ ID NO: 26, or a complementary nucleic acid thereof, or a fragment thereabove.

51. The method of any one of claims 1-50, comprising providing a nucleic acid set as set forth in SEQ ID NOs: 27 and 28, or a complementary nucleic acid set thereof, or a fragment thereabove.

52. The method of any one of claims 1-51, comprising providing a nucleic acid capable of binding to a DNA region comprising SEQ ID NO: 1, or a complementary region thereof, or a transformed region thereabove, or a fragment thereabove.

53. The method of any one of claims 16-52, wherein the target region comprises a region defined from human chr17:75369603-75369693.

54. The method of any one of claims 1-53, comprising providing a nucleic acid as set forth in SEQ ID NO: 29, or a complementary nucleic acid thereof, or a fragment thereabove.

55. The method of any one of claims 1-54, comprising providing a nucleic acid set as set forth in SEQ ID NOs: 30 and 31, or a complementary nucleic acid set thereof, or a fragment thereabove.

56. The method of any one of claims 16-55, wherein the disease comprises a tumor.

57. The method of any one of claims 16-56, wherein the disease comprises a liver tumor.

58. The method of any one of claims 1-57, the method further comprising obtaining a nucleic acid in the sample to be tested.

59. The method of claim 58, wherein the nucleic acid comprises a cell-free free nucleic acid.

60. The method of any one of claims 1-59, wherein the sample to be tested comprises a tissue, a cell and/or a body fluid.

61. The method of any one of claims 1-60, wherein the sample to be tested comprises a plasma.

62. The method of any one of claims 1-61, further comprising transforming the DNA region or a fragment thereof.

63. The method of claim 62, wherein the base with the modification status and the base without the modification status form different substances after the transformation.

64. The method of any one of claims 1-63, wherein the base with the modification status is substantially unchanged after the transformation, and the base without the modification status is changed to other bases different from the base after the transformation, or is cleaved after the transformation.

65. The method of any one of claims 63-64, wherein the base comprises cytosine.

66. The method of any one of claims 1-65, wherein the modification status comprises methylation modification.

67. The method of any one of claims 64-66, wherein the other bases comprise uracil.

68. The method of any one of claims 62-67, wherein the transformation comprises transformation by a deamination reagent and/or a methylation sensitive restriction enzyme.

69. The method of claim 68, wherein the deamination reagent comprises a bisulfite or an analog thereof.

70. The method of any one of claims 1-69, wherein the method for determining the presence and/or the content of modification status comprises identifying the presence and/or the content of a substance formed by a base with the modification status after the transformation.

71. The method of any one of claims 1-70, wherein the method for determining the presence and/or the content of modification status comprises determining the presence and/or the content of a DNA region with the modification status or a fragment thereof.

72. The method of any one of claims 1-71, wherein the presence and/or the content of a DNA region with the modification status or a fragment thereof is determined by the fluorescence Ct value detected by the fluorescence PCR method.

73. The method of any one of claims 1-72, wherein the presence of a liver tumor or the development or development risk of the liver tumor is determined by identifying the presence of a modification status of the DNA region or a fragment thereof, and/or the content of modification status of which the DNA region or a fragment thereof is higher relative to a reference level.

74. The method of any one of claims 1-73, further comprising amplifying the DNA region or a fragment thereof in the sample to be tested before determining the presence of and/or the content of modification of the DNA region or a fragment thereof.

75. The method of claim 74, wherein the amplification comprises PCR amplification.

76. A nucleic acid comprising a sequence capable of binding to a DNA region in which a target gene is located, or a complementary region thereof, or a transformed region thereabove, or a fragment thereabove in the method of any one of claims 1-15 and 58-75; or comprising a sequence capable of binding to a target DNA region, or a complementary region thereof, or a transformed region thereabove, or a fragment thereabove in the method of any one of claims 16-75.

77. A method for preparing a nucleic acid, comprising, based on modification status of a DNA region in which a target gene is located, or a complementary region thereof, or a transformed region thereabove in the method of any one of claims 1-15 and 58-75, or a fragment thereabove, designing a nucleic acid capable of binding to the DNA region in which a target gene is located, or a complementary region thereof, or a transformed region thereabove, or a fragment thereabove; or, based on a modification status of a target DNA region, or a complementary region thereof, or a transformed region thereabove, or a fragment thereabove in the method of any one of claims 16-75, designing a nucleic acid capable of binding to the target DNA region, or a complementary region thereof, or a transformed region thereabove, or a fragment thereabove.

78. A nucleic acid set, comprising a sequence capable of binding to a DNA region in which a target gene is located in the method of any one of claims 1-15 and 58-75, or a complementary region thereof, or a transformed region thereabove, or a fragment thereabove; or comprising a sequence capable of binding to a target DNA region in the method of any one of claims 16-75, or a complementary region thereof, or a transformed region thereabove, or a fragment thereabove.

79. A method for preparing nucleic acid set, comprising, based on modification status of a DNA region in which a target gene is located, or a complementary region thereof, or a transformed region thereabove, or a fragment thereabove in the method of any one of claims 1-15 and 58-75, designing a nucleic acid set capable of binding to the DNA region in which a target gene is located, or a complementary region thereof, or a transformed region thereabove, or a fragment thereabove; or, based on a modification status of a target DNA region, or a complementary region thereof, or a transformed region thereabove, or a fragment thereabove in the method of any one of claims 16-75, designing a nucleic acid set capable of binding to the target DNA region, or a complementary region thereof, or a transformed region thereabove, or a fragment thereabove.

80. A kit comprising the nucleic acid of claim 76 and/or the nucleic acid set of claim 78.

81. Use of the nucleic acid of claim 76, the nucleic acid set of claim 78 and/or the kit of claim 80 in the preparation of a substance for determining modification status of a DNA region or a fragment thereof.

82. Use of the nucleic acid of claim 76, the nucleic acid set of claim 78 and/or the kit of claim 80 in the preparation of a disease detection product.

83. Use of the nucleic acid of claim 76, the nucleic acid set of claim 78 and/or the kit of claim 80 in the preparation of a substance for identifying the presence of a disease, assessing the development or development risk of the disease and/or assessing the progress of the disease.

84. The use of any one of claims 82-83, wherein the disease comprises a tumor.

85. The use of any one of claims 82-84, wherein the disease comprises a liver tumor.

86. Use of a nucleic acid, a nucleic acid set and/or a kit for determining modification status of a DNA region in the preparation of a substance for identifying the presence of a liver tumor, assessing the development or development risk of the liver tumor and/or assessing the progress of the liver tumor, wherein the DNA region for determination comprises a DNA region in which a target gene is located, or a fragment thereof in the method of any one of claims 1-15 and 58-75.

87. Use of a nucleic acid, a nucleic acid set and/or a kit for determining modification status of a DNA region in the preparation of a substance for identifying the presence of a disease, assessing the development or development risk of the disease and/or assessing the progress of the disease, wherein the DNA region for determination comprises a target DNA region, or a complementary region thereof, or a fragment thereabove in the method of any one of claims 16-75.

88. The use of claim 87, wherein the disease comprises a tumor.

89. The use of any one of claims 87-88, wherein the disease comprises a liver tumor.

90. The use of any one of claims 81 and 86-89, wherein the modification status comprises a methylation modification.

91. Use of a nucleic acid of a DNA region in which a target gene is located, or a transformed region thereof or a fragment thereabove, or a combination thereof in the method of any one of claims 1-15 and 58-75, in the preparation of a substance for identifying the presence of a liver tumor, assessing the development or development risk of the liver tumor, and/or assessing the progress of the liver tumor.

92. Use of a nucleic acid of a target DNA region, or a complementary region thereof or a transformed region thereabove or a fragment thereabove, or a combination thereof in the method of any one of claims 16-75, in the preparation of a substance for identifying the presence of a disease, assessing the development or development risk of the disease, and/or assessing the progress of the disease.

93. The use of claim 92, wherein the disease comprises a tumor.

94. The use of any one of claims 92-93, wherein the disease comprises a liver tumor.

95. A storage medium recording a program capable of executing the method of any one of claims 1-75.

96. A device comprising the storage medium of claim 95.

97. The device of claim 96, further comprising a processor coupled to the storage medium, wherein the processor is configured to execute based on a program stored in the storage medium to implement the method of any one of claims 1-75.
